# EUROPEAN PATENT SPECIFICATION

(11) **EP 4 192 486 B1**
(45) Date of publication and mention of the grant of the patent: **22.07.2026**
(21) Application number: 21852249.8
(22) Date of filing: 09.08.2021
(51) Int. Cl.: A61K 38/00, A61K 39/00, A61K 39/395, A61K 45/00, A61P 35/00, C07K 14/47

(54) **THERAPEUTIC TARGETING OF PHOSPHATE DYSREGULATION IN CANCER VIA THE XPR1:KIDINS220 PROTEIN COMPLEX**
THERAPEUTISCHES TARGETING DER PHOSPHATDYSREGULATION BEI KREBS ÜBER DEN XPR1:KIDS220-PROTEINKOMPLEX
CIBLAGE THÉRAPEUTIQUE D'UNE DÉRÉGULATION DU PHOSPHATE DANS LE CANCER PAR L'INTERMÉDIAIRE DU COMPLEXE PROTÉIQUE XPR1 : KIDINS220

(30) Priority: 07.08.2020 US 202063062890 P
(43) Date of publication of application: 14.06.2023
(73) Proprietor: The Broad Institute, Inc., Cambridge, MA 02142 (US); Dana-Farber Cancer Institute, Inc., Boston, Massachusetts 02215 (US)
(72) Inventor: GOLUB, Todd, Cambridge, Massachusetts 02142 (US); BONDESON, Daniel, Cambridge, Massachusetts 02142 (US); PAOLELLA, Brenton, Cambridge, Massachusetts 02142 (US); VAZQUEZ, Francisca, Cambridge, Massachusetts 02142 (US)
(74) Representative: Impact Intellectual Property LLP
(86) International application number: PCT/US2021/045227
(87) International publication number: WO 2022/032228

(56) References cited:
- WO-A1-2017/060304
- WO-A1-2020/153467
- WO-A1-2021/030627
- WO-A2-02/20786
- US-A1- 2006 166 941
- US-A1- 2011 129 483
- US-B2- 10 925 948
- YANG W ET AL: "MiR-4638-5p inhibits castration resistance of prostate cancer through repressing Kidins220 expression and PI3K/AKT pathway activity", EUROPEAN UROLOGY SUPPLEMENTS, ELSEVIER BV, NL, vol. 17, no. 10, 4 October 2018 (2018-10-04), XP085498738, ISSN: 1569-9056, DOI: 10.1016/S1569-9056(18)33035-5
- CHEN DAR-REN ET AL: "SLC34A2 as a Novel Marker for Diagnosis and Targeted Therapy of Breast Cancer", ANTICANCER RESEARCH, 1 October 2010 (2010-10-01), Attiki, pages 4135 - 4140, XP093144139, Retrieved from the Internet <URL:https://ar.iiarjournals.org/content/anticanres/30/10/4135.full.pdf> [retrieved on 20240321]
- LI XINGYAO, GU CHUNFANG, HOSTACHY SARAH, SAHU SOUMYADIP, WITTWER CHRISTOPHER, JESSEN HENNING J., FIEDLER DOROTHEA, WANG HUANCHEN, : "Control of XPR1-dependent cellular phosphate efflux by InsP 8 is an exemplar for functionally-exclusive inositol pyrophosphate signaling", PROCEEDINGS OF THE NATIONAL ACADEMY OF SCIENCES, NATIONAL ACADEMY OF SCIENCES, vol. 117, no. 7, 18 February 2020 (2020-02-18), pages 3568 - 3574, XP055906357, ISSN: 0027-8424, DOI: 10.1073/pnas.1908830117
- HIBBS ET AL.: "Differential Gene Expression in Ovarian Carcinoma", AMERICAN JOURNAL OF PATHOLOGY, vol. 165, no. 2, 1 August 2004 (2004-08-01), pages 397 - 414, XP002581801
- BONDESON DANIEL P, PAOLELLA BRENTON R, ASFAW ADHANA, ROTHBERG MICHAEL, SKIPPER THOMAS, LANGAN CARLY, GONZALEZ ALFREDO, SURFACE LAU: "Phosphate dysregulation via the XPR1:KIDINS220 protein complex is a therapeutic vulnerability in ovarian cancer", BIORXIV, 8 April 2021 (2021-04-08), XP055906362, Retrieved from the Internet <URL:https://www.biorxiv.org/content/10.1101/2020.10.16.339374v2.full.pdf> [retrieved on 20220329], DOI: 10.1101/2020.10.16.339374

## Description

### CROSS-REFERENCE TO RELATED APPLICATIONS

This application claims the benefit of U.S. Provisional Application No. 63/062,890, filed August 7, 2020.

### REFERENCE TO AN ELECTRONIC SEQUENCE LISTING

The contents of the electronic sequence listing ("BROD-5160WP_ST25.txt"; Size is 18,185 bytes and it was created on August 6, 2021.

### TECHNICAL FIELD

The subject matter disclosed herein is generally directed to inhibition of XPR1:KIDINS220-mediated phosphate export in the treatment of cancer and determining cancer dependency on phosphate export by detecting the expression of SLC34A2.

### BACKGROUND

Ovarian and uterine cancers are among the deadliest cancers that affect women, and while platinum-based chemotherapies and recently approved PARP inhibitors show efficacy for some patients ²⁻⁴, outcomes in these cancers have not improved greatly in the past twenty years ^{5,6}. Clearly, new therapeutic options are needed.

While organismal phosphate homeostasis is well understood, the cellular coordination of phosphate import, storage, and efflux is poorly understood. Phosphate uptake is highly regulated and involves members of the SLC34 and SLC20 gene families ⁷⁻⁹. In yeast and plants, specialized vacuoles and metabolites coordinate phosphate storage, but these pathways are not elucidated in human biology ^{10,11}. Phosphate efflux is achieved via the only annotated human exporter - the Xenotropic and Polytropic Receptor 1 (XPR1) ¹². XPR1 is involved in phosphate homeostasis of various tissues ¹³⁻¹⁵, although the mechanisms of its regulation and function are poorly understood ^{16,17}.

### SUMMARY

The invention is set out in the appended set of claims.

In one aspect, the present invention provides for one or more therapeutic agents for use in a method of treating uterine or ovarian cancer in a subject in need thereof wherein at least one therapeutic agent in the one or more therapeutic agents is capable of inhibiting XPR1:KIDINS220-mediated phosphate export, the method comprising administering to the subject one or more therapeutic agents , wherein the one or more therapeutic agents comprises a Receptor Binding Domain (RBD) fusion protein comprising the amino acid sequence SEQ ID NO: 2, wherein the uterine or ovarian cancer is characterized by higher expression of SLC34A2 in tumor tissue as compared to expression in normal tissue. The cancer is selected from the group consisting of ovarian cancer and uterine cancer. In certain embodiments described but not specifically claimed herein, the cancer is selected from the group consisting of breast cancer, bile duct cancer, liver and lung cancer. The cancer is characterized by higher expression of SLC34A2 in tumor tissue as compared to expression in normal tissue. In certain embodiments, the one or more therapeutic agents disrupt XPR1/KIDINS220 interaction. In certain embodiments, the one or more therapeutic agents comprise a receptor binding domain (RBD) protein derived from an enveloped virus glycoprotein and capable of interacting with the XPR1 membrane receptor. In certain embodiments, the RBD protein is a fusion protein, wherein the fusion protein comprises a domain capable of dimerization and/or stabilization of the protein. In certain embodiments, the RBD protein is fused to an Fc domain, glutathione S-transferase (GST), and/or serum albumin. In certain embodiments, the RBD protein is derived from xenotropic or polytropic murine leukemia retrovirus (X- and P-MLV) Env. In certain embodiments, described but not specifically claimed herein, the RBD fusion protein comprises the amino acid sequence: MLVMEGSAFSKPLKDKINPWGPLIVMGILVRAGASVQRDSPHQIFNVTWRVTNLMTGQTANA TSLLGTMTDTFPKLYFDLCDLVGDYWDDPEPDIGDGCRTPGGRRRTRLYDFYVCPGHTVPIG CGGPGEGYCGKWGCETTGQAYWKPSSSWDLISLKRGNTPKDQGPCYDSSVSSGVQGATPGGR CNPLVLEFTDAGRKASWDAPKVWGLRLYRSTGADPVTRFSLTRQVLNVGPRVPIGSVDVPRD CGCKPCICTVPEVSSVFIFPPKPKDVLTITLTPKVTCVVVDISKDDPEVQFSWFVDDVEVHT AQTQPREEQFNSTFRSVSELPIMHQDWLNGKEFKCRVNSAAFPAPIEKTISKTKGRPKAPQV YTIPPPKEQMAKDKVSLTCMITDFFPEDITVEWQWNGQPAENYKNTQPIMDTDGSYFVYSKL NVQKSNWEAGNTFTCSVLHEGLHNHHTEKSLSHSPGK **(SEQ ID NO:** 1). In certain embodiments, described but not specifically claimed herein, the one or more therapeutic agents comprise a vector encoding for the RBD protein. In certain embodiments, the one or more therapeutic agents comprise an antibody specific for XPR1, an antibody specific for KIDINS220, or an antibody specific to the XPR1/KIDINS220 protein complex. In certain embodiments, the antibody targets a Walker A/B motif of KIDINS220. In certain embodiments, the one or more therapeutic agents comprise a degrader molecule. In certain embodiments, the degrader molecule is a LYTAC molecule, whereby a cell surface protein is targeted. In certain embodiments, the one or more therapeutic agents comprise a genetic modifying agent capable of inhibiting the expression of XPR1 or KIDINS220. In certain embodiments, the genetic modifying agent comprises a CRISPR-Cas system, a RNAi, a zinc finger nuclease, a TALE system, or a meganuclease. In certain embodiments, the CRISPR-Cas system is a CRISPR-Cas base editing system, a prime editor system, or a CAST system. In certain embodiments, the method further comprises administering to the subject one or more agents capable of inhibiting the expression or activity of FGF23, capable of inhibiting the suppression of SLC34A2, or capable of modulating one or more genes up or down-regulated in response to XPR1 inhibition. In certain embodiments, one or more therapeutic agents capable of inhibiting XPR1:KIDINS220-mediated phosphate export are co-administered within a standard of care treatment regimen. In certain embodiments, the standard of care treatment regimen comprises surgery and chemotherapy. In certain embodiments, the standard of care treatment regimen comprises administration of an immunotherapy, checkpoint blockade therapy or a PARP inhibitor.

In another aspect, described but not specifically claimed herein, the present invention provides for a method of treating cancer in a subject in need thereof comprising: detecting tumors sensitive to phosphate dysregulation by detecting increased expression of SLC34A2 relative to a control, wherein if the subject has a tumor sensitive to phosphate dysregulation, including administration of one or more therapeutic agents capable of inhibiting XPR1:KIDINS220-mediated phosphate export according to any embodiment herein; if the subject does not have a tumor sensitive to phosphate dysregulation, administering a standard of care treatment that does not include administration of one or more therapeutic agents capable of inhibiting XPR1:KIDINS220-mediated phosphate export. In certain embodiments, described but not specifically claimed herein, the cancer is selected from the group consisting of ovarian cancer, uterine cancer, breast cancer, bile duct cancer, liver and lung cancer. In certain embodiments, described but not specifically claimed herein, the standard of care treatment comprises one or more of surgery, chemotherapy, immunotherapy, checkpoint blockade therapy or administration of a PARP inhibitor. In certain embodiments, described but not specifically claimed herein, the method further comprises monitoring the efficacy of the treatment comprising detecting in a tumor sample obtained from the subject the expression of one or more genes selected from the group consisting of SLC34A2, SLC20A1 and FGF23, wherein the treatment is effective if SLC34A2 and/or SLC20A1 are decreased, and/or FGF23 is increased. In certain embodiments, described but not specifically claimed herein, the method further comprises monitoring the efficacy of the treatment comprising detecting increased morphological changes associated with phosphate dysregulation in tumor cells obtained from the subject, wherein the treatment is effective if increased morphological changes associated with phosphate dysregulation are detected. In certain embodiments, described but not specifically claimed herein, the morphological changes associated with phosphate dysregulation comprise vacuole-like structures in tumor cells.

In another aspect, the present invention provides for an *in vitro* method of determining whether a uterine or ovarian cancer has is sensitive to phosphate dysregulation comprising detecting the expression of SLC34A2 in a tumor sample obtained from the subject, wherein if SLC34A2 expression is higher in the tumor sample as compared to expression in normal tissue the tumor is sensitive. In certain embodiments, the method further comprises detecting PAX8. In certain embodiments, the method further comprises detecting any gene that co-varies with SLC34A2. The cancer is selected from the group consisting of ovarian cancer and uterine cancer. In certain embodiments, described but not specifically claimed herein, the cancer is selected from the group consisting of breast cancer, bile duct cancer, liver and lung cancer.

In another aspect, the present invention provides for an *in vitro* method of determining whether a subject suffering from cancer has a tumor sensitive to phosphate dysregulation comprising detecting the amplification in XPR1 copy number in a tumor sample obtained from the subject, wherein if XPR1 copy number amplification is detected in the tumor sample the tumor is sensitive. In certain embodiments, copy number is detected by inference from a target sequencing panel at the XPR1 locus on chromosome 1. The cancer is selected from the group consisting of ovarian cancer and uterine cancer. In certain embodiments, described but not specifically claimed herein, the cancer is selected from the group consisting of breast cancer, bile duct cancer, liver and lung cancer.

In certain embodiments, detecting comprises one or more of immunohistochemistry (IHC), in situ RNA-seq, quantitative PCR, RNA-seq, CITE-seq, western blot, Fluorescence In Situ Hybridization (FISH), RNA-FISH, mass spectrometry, or FACS.

In another aspect, the present invention provides for a method for identifying an agent capable of inhibiting XPR1:KIDINS220-mediated phosphate export, comprising: applying a candidate agent to a cancer cell or cell population; and detecting inhibition of phosphate efflux in the cell or cell population by the candidate agent, thereby identifying the agent.

In another aspect, the present invention provides for a method for identifying a cancer sensitive to inhibition of XPR1:KIDINS220-mediated phosphate export, comprising: applying an inhibitor of XPR1:KIDINS220-mediated phosphate export to a cancer cell or cell population; and detecting the phosphate concentration in the cell or cell population, wherein the cancer is sensitive if the phosphate concentration is increased as compared to a control cell or population not treated with the inhibitor. In certain embodiments, the inhibitor of XPR1:KIDINS220-mediated phosphate export is one or more therapeutic agents according to any embodiment herein. In certain embodiments, the cancer cell or population is obtained or derived from a subject in need thereof.

These and other aspects, objects, features, and advantages of the example embodiments will become apparent to those having ordinary skill in the art upon consideration of the following detailed description of example embodiments.

### BRIEF DESCRIPTION OF THE DRAWINGS

An understanding of the features and advantages of the present invention will be obtained by reference to the following detailed description that sets forth illustrative embodiments, in which the principles of the invention may be utilized, and the accompanying drawings of which:

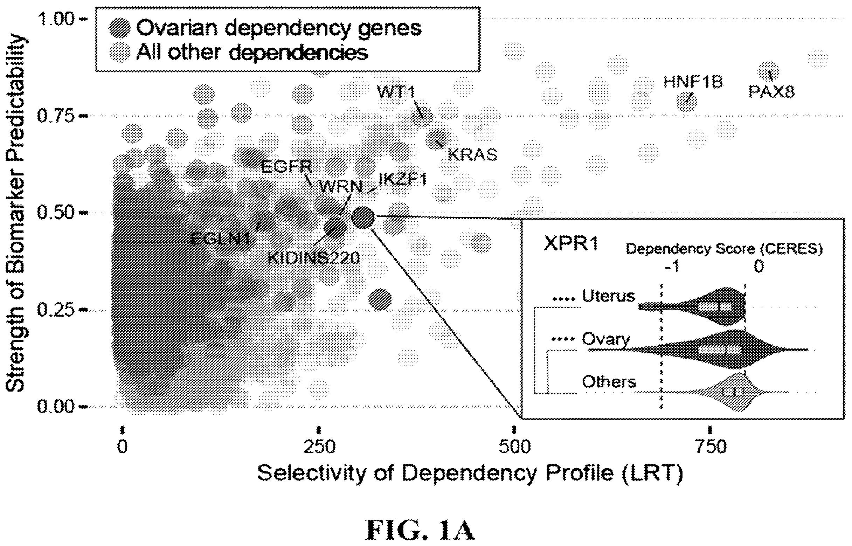

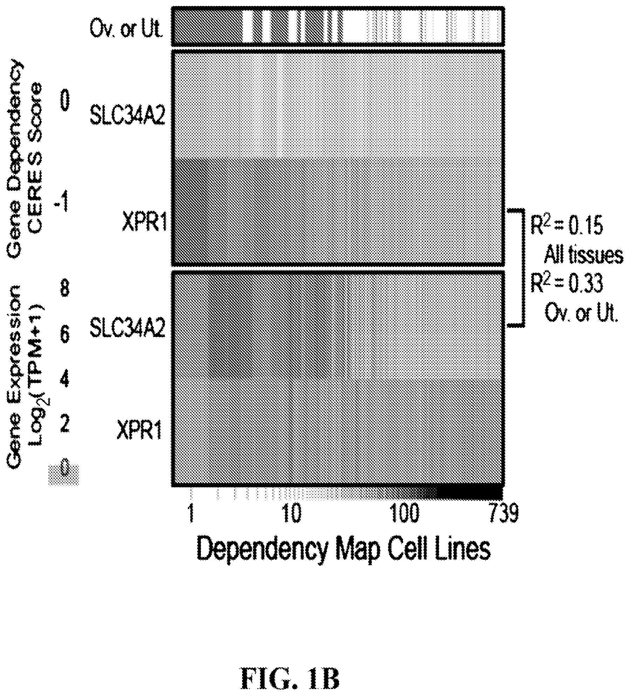

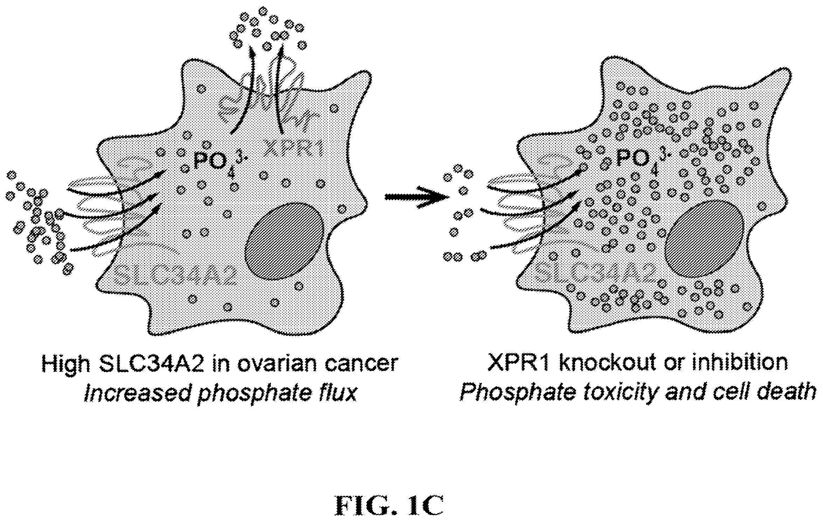

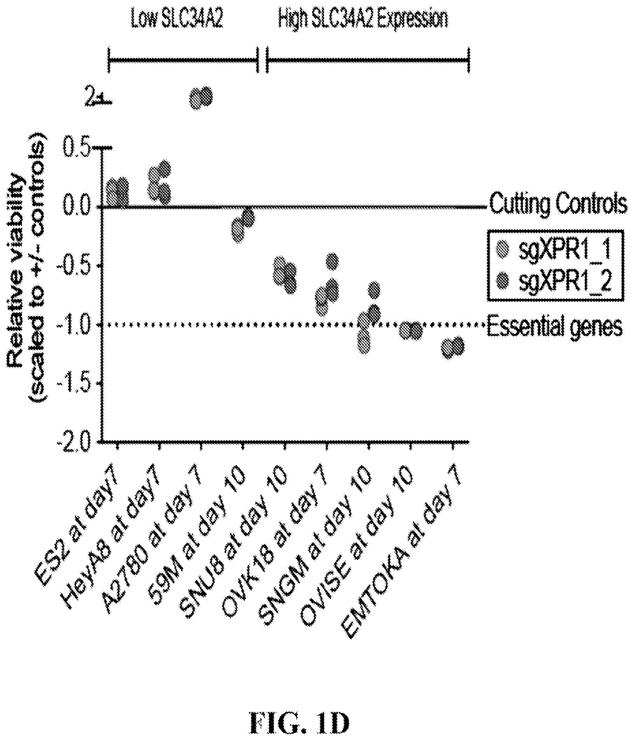

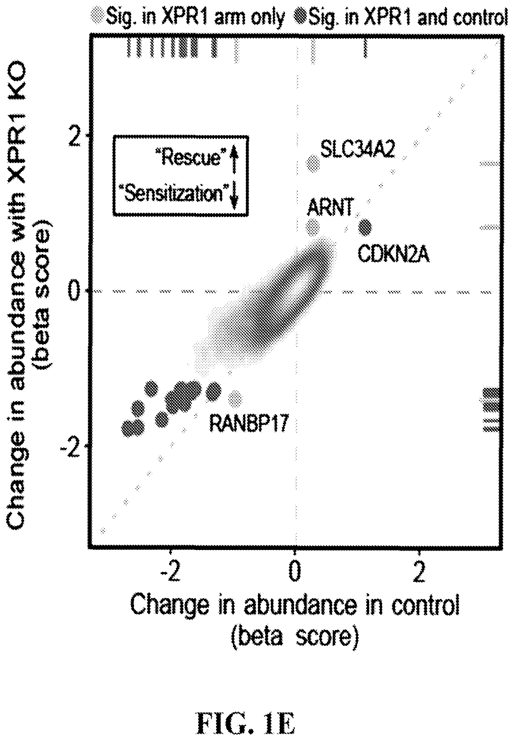

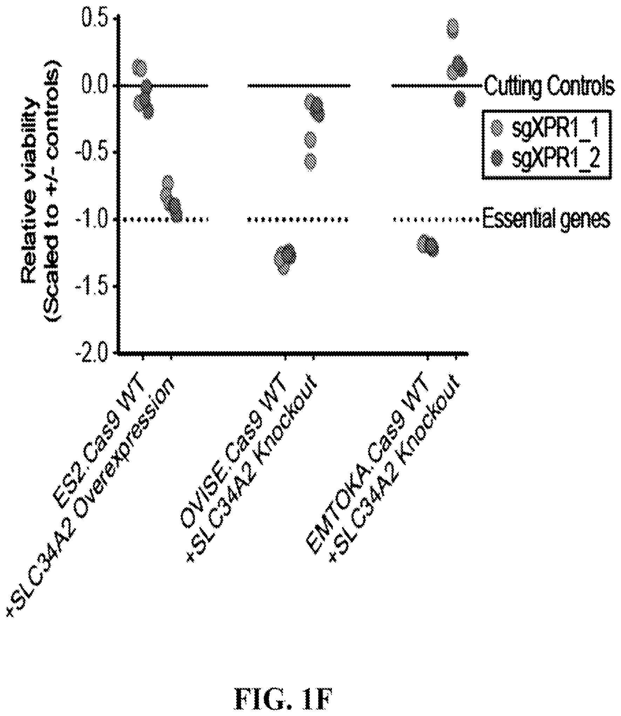

**FIG. 1** - **XPR1 is a dependency in ovarian cancer in the context of SLC34A2 overexpression. a.** Across 733 cancer cell lines (DepMap Avana 20Q1), the predictability and selectivity of cell line killing across all ~17,000 genes tested. Teal dots represent dependencies in ovarian cancer cell lines. The inset shows the dependency profile for XPRR1. **b.** XPR1 and SLC34A2 expression and dependency profiles across all cell lines, approximately ranked by decreasing dependency on XPR1. **c.** Because of their relative directionalities of phosphate transport, Applicants hypothesize that XPR1 inactivation is toxic because of intracellular phosphate accumulation in SLC34A2-high ovarian cancer. **d.** Viability effects after inactivation of XPR1, scaled compared to negative and positive control genes. **e.** Genome-scale CRISPR/Cas9 screen combined with iInactivation of XPR1 is combined - pairwise - with a genome-scale loss of the function screen to find potential 'modifier' genes for the dependency. Beta scores (determined by MaGeCK MLE) represent the change in representation for each gene from the initial library to the final timepoint. Teal points are genes significant only when combined with XPR1 inactivation, while mauve points are were significantly changed in the XPR1 and in the control arms (such as the tumor suppressor CDKN2A). f. The SLC34A2 status of normally XPR1-resistant (ES2) or XPR1-sensitive (EMTOKA and OVISE) cell lines was modified by overexpression or knockout, and the XPR1 dependency was evaluated as in panel **d.**

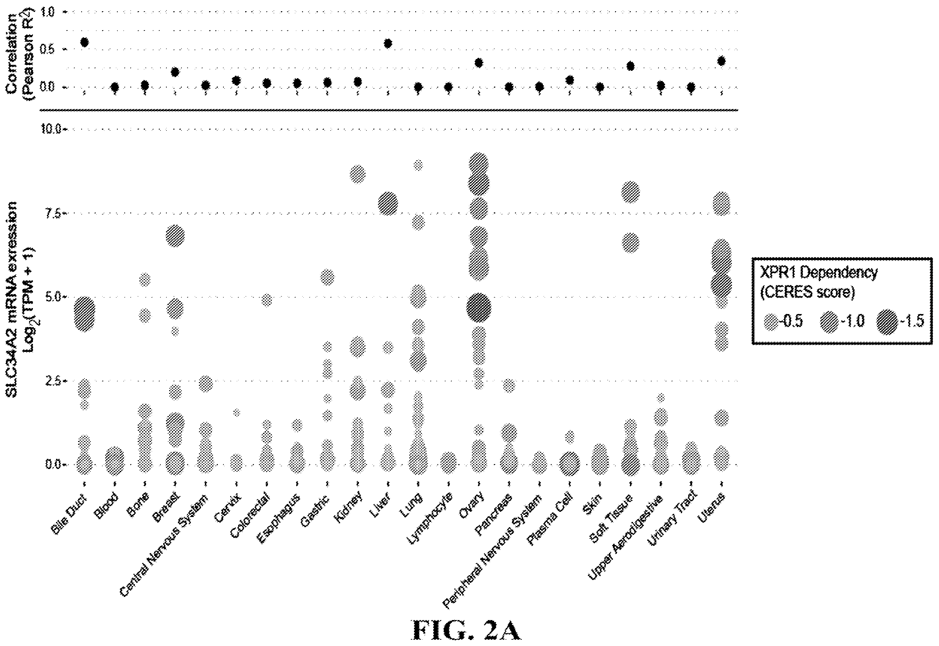

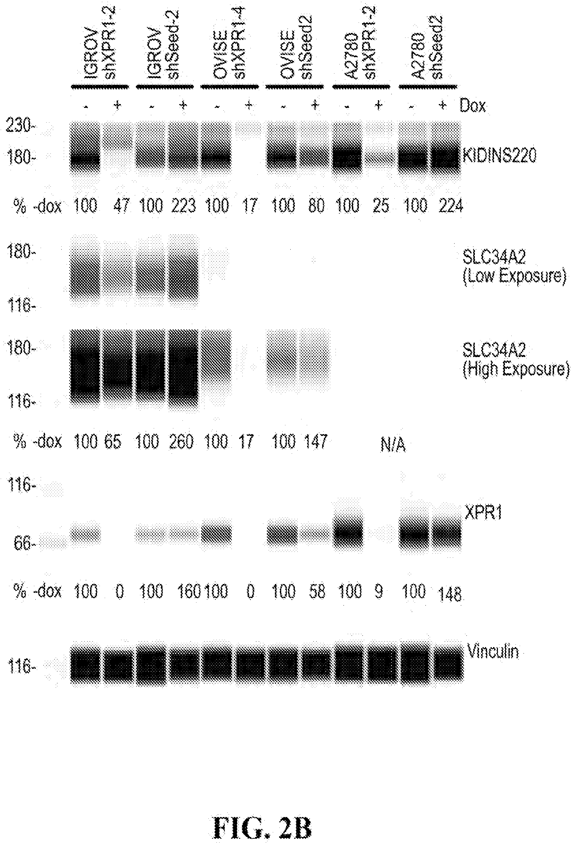

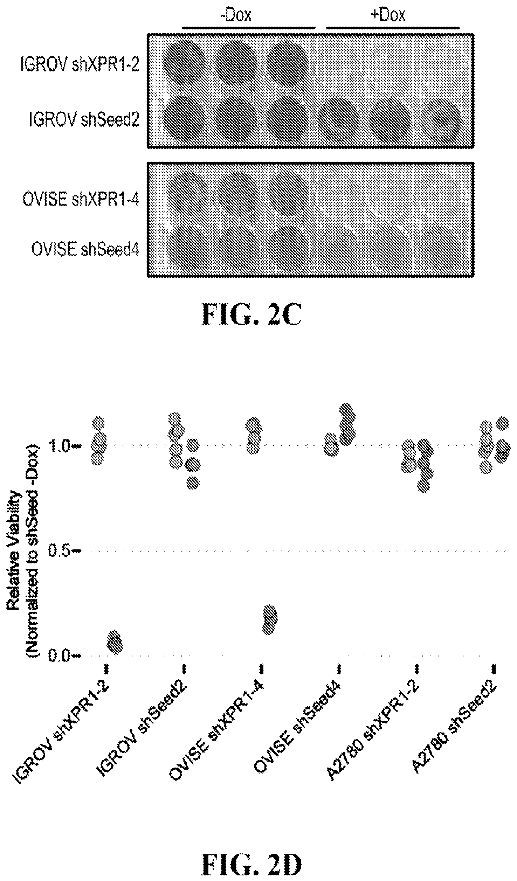

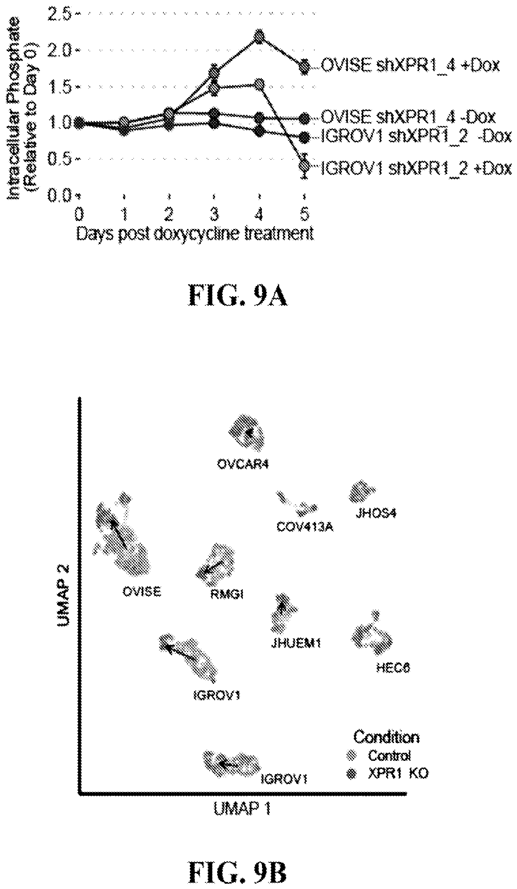

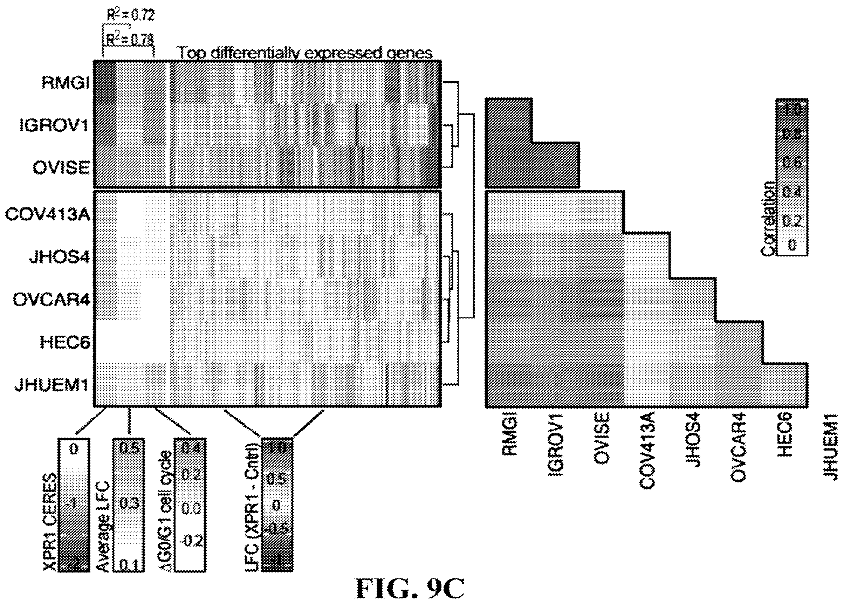

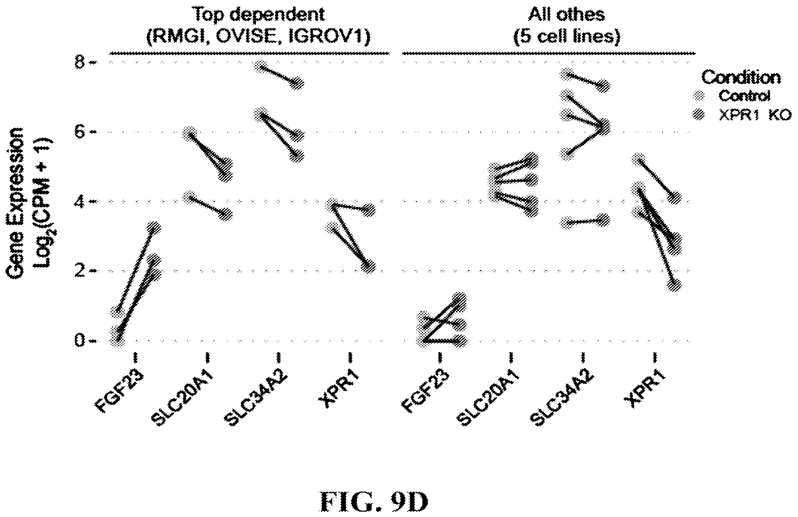

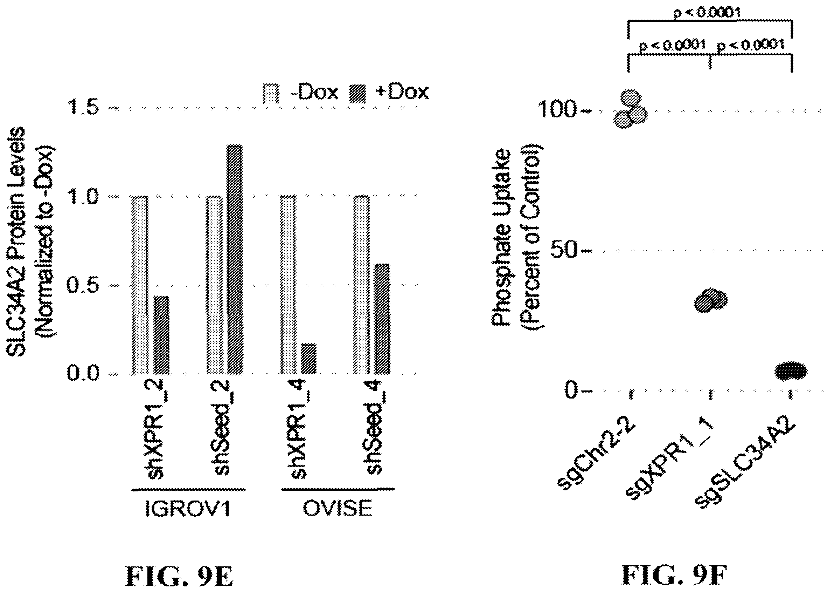

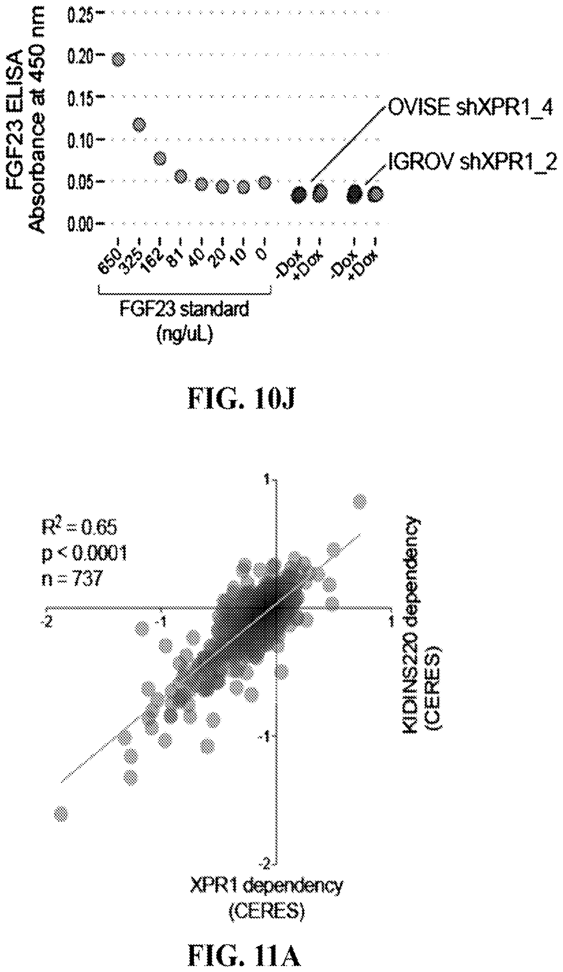

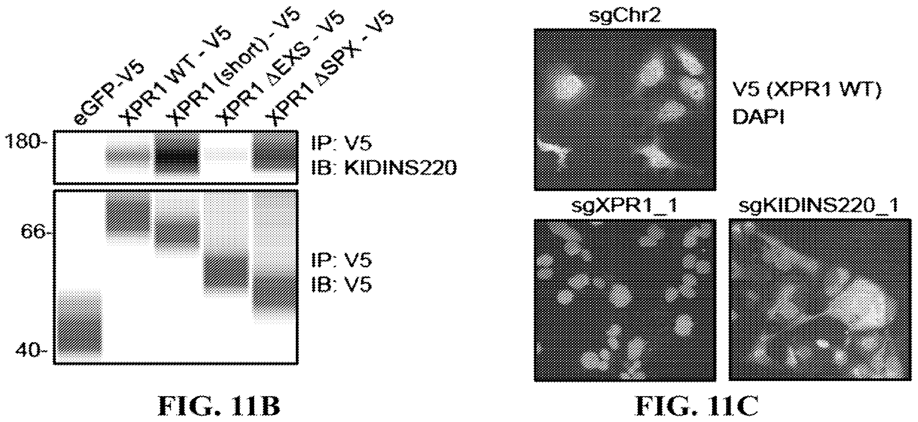

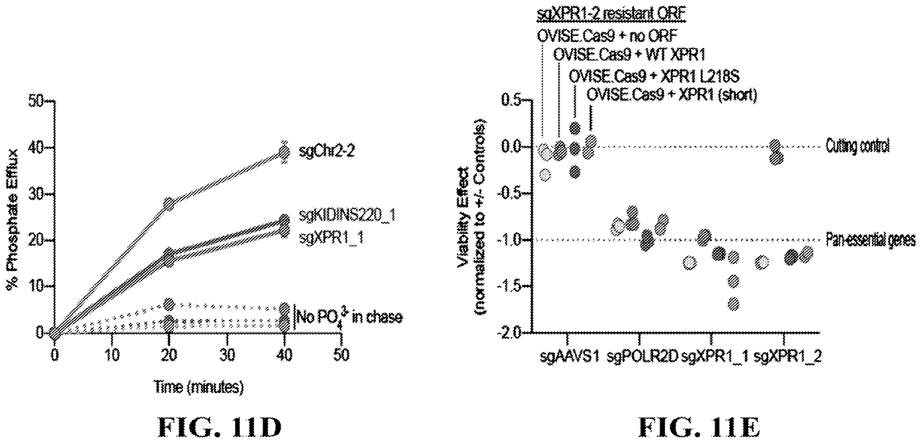

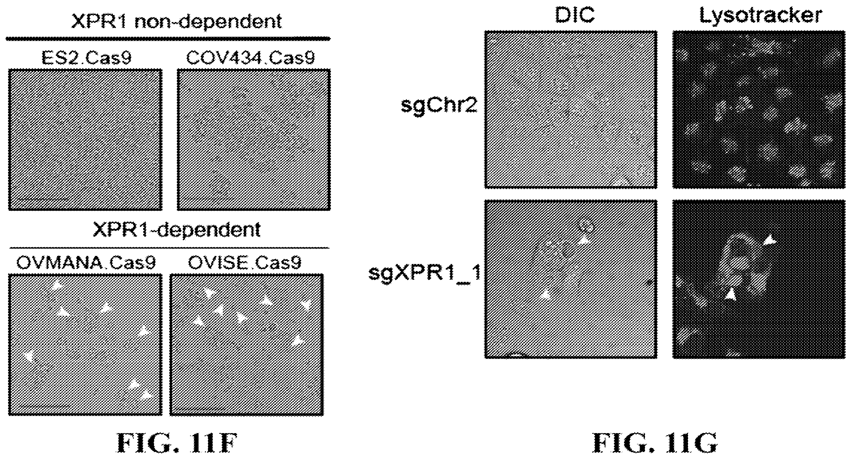

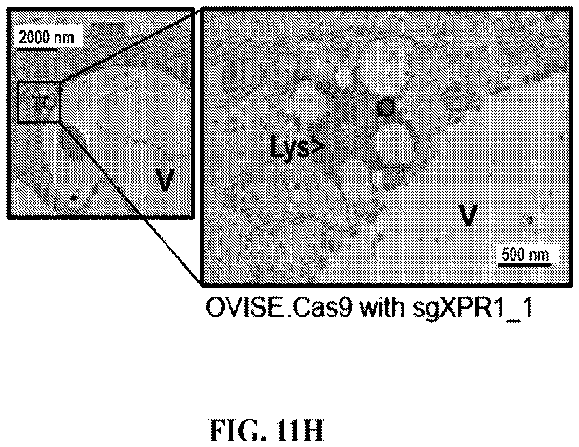

**FIG. 2** - **Validation of the XPR1 dependency in SLC34A2-overexpressing ovarian cancer cell lines using shRNA. a.** Bottom, a bubble plot showing the expression of SLC34A2 across all cancer cell lines, with the degree of dependency on XPR1 encoded by the size and color of the point (more dependent lines have larger points with deeper hues). Note that lung cancer cell lines do not display dependency on XPR1, despite high expression of SLC34A2, and that some highly dependent cell lines do not express high levels of SLC34A2. Top, the Pearson correlation (R²) between SLC34A2 mRNA expression and XPR1 dependency within every lineage represented by at least 10 different cell lines. **b.** Three different cell lines were engineered with doxycycline-inducible shRNA targeting XPR1 (shXPR1_2 and shXPR1_4) or seed-matched control shRNA (shSeed_2 and shSeed_4) which have the same seed sequence but should not suppress XPR1 mRNA. Three days after induction of shRNA, cellular lysates were analyzed by protein levels for KIDINS220 (see **figure 11****),** SLC34A2 (see **figure 9****),** and XPR1. Protein levels normalized to vinculin are expressed below each band. **c.** Viability effect of suppression of XPR1 using shRNA reagents is highly penetrant. Cells were plated at low density and treated with doxycycline to induce expression of shRNA. 14 days after plating, surviving cells were stained with crystal violet. Note that shXPR1 reagents effectively suppress growth, but shSeed reagents have no effect on cellular growth. **d.** Seven days after induction of shRNA, viability was measured Quantification of using Cell Titer Glo (Promega). IGROV1 and OVISE both express high levels of SLC34A2 and are predicted to be dependent on XPR1; A2780 does not.

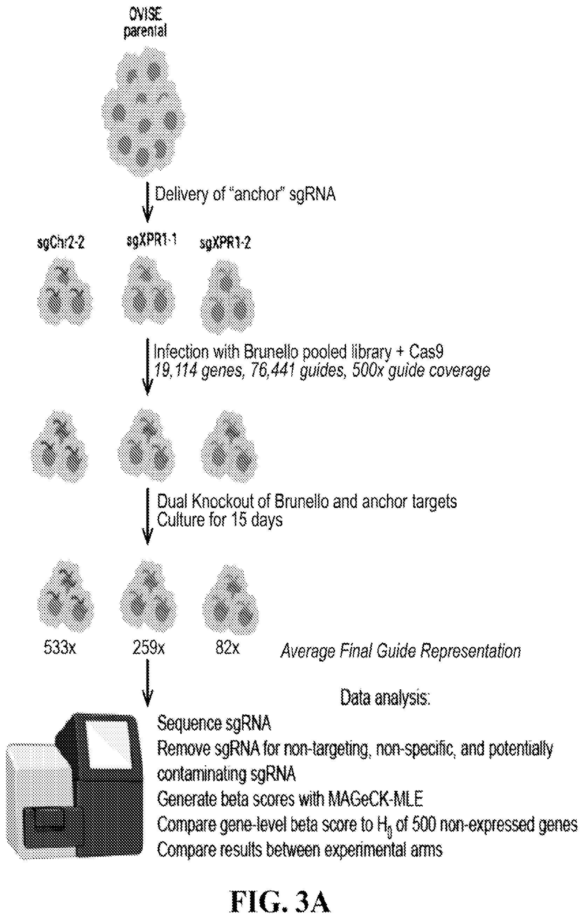

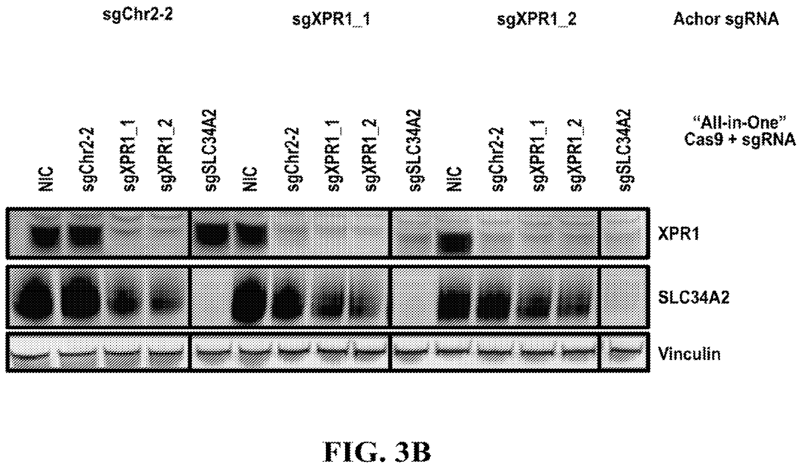

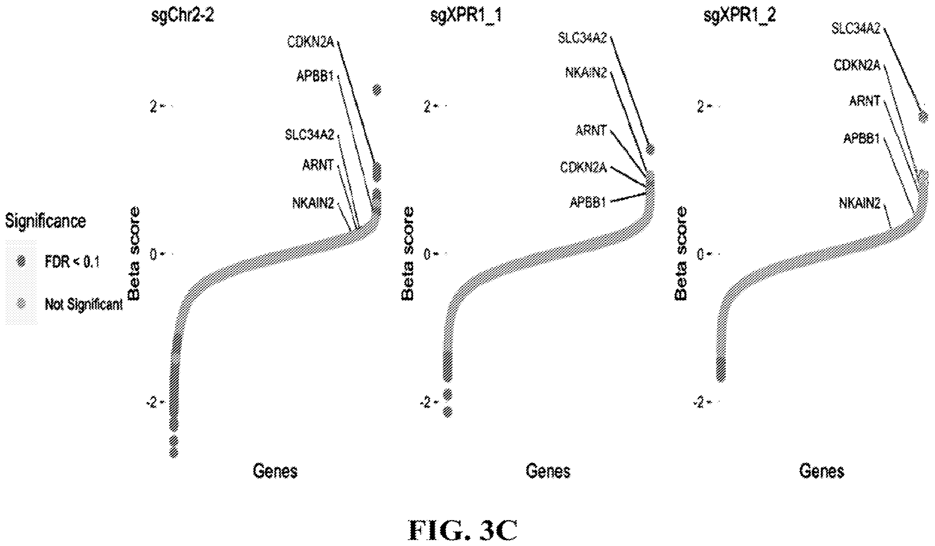

**FIG. 3** **- A genome-scale CRISPR/Cas9 screen validates the relationship between XPR1 dependency in the context of high expression of SLC34A2. a.** Outline of the experimental method for a genome-scale, dual-knockout modifier screen. OVISE (without Cas9 expression) is engineered to stably express sgRNA targeting XPR1. Upon introduction of "all-in-one" lentivirus, containing both Cas9 ORF and a second sgRNA, both genes are simultaneously cut by Cas9. Applicants used three sgRNA: one targeting a gene desert on chromosome 2 (sgChr2-2) and two targeting XPR1 (sgXPR1_1 and sgXPR1_2) and infecting the cells with the Brunello genome-scale sgRNA library. 15 days after infection, Applicants collected cell pellets (far smaller in the sgXPR1 arms because of XPR1-dependent cell death) and sequenced the sgRNA. b. Western confirmation of dual-knockout of XPR1 and SLC34A2. The three cell lines used in the genome-scale screen were infected with "all-in-one" lentivirus expressing control-, XPR1-, or SLC34A2-targeting sgRNA. Note that in the sgXPR1 "anchor" cell lines, XPR1 is suppressed with the control virus, indicating that the first infection provides XPR1-targeting sgRNA and the second infection provides Cas9 protein. NIC stands for "no-infection control'. c. Arm-level results of the genome-scale modifier screen. See methods for full analysis details. Beta-scores represent the extent to which a gene was enriched or depleted relative to all other genes.

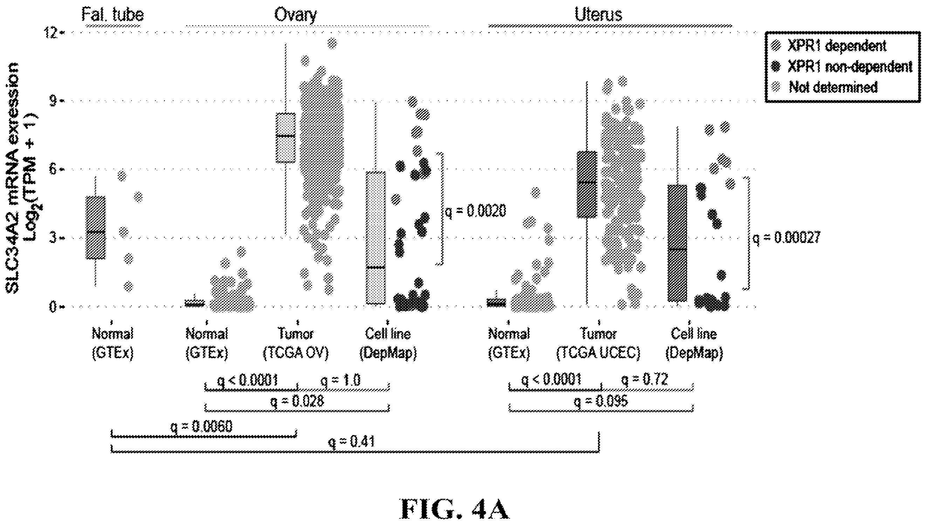

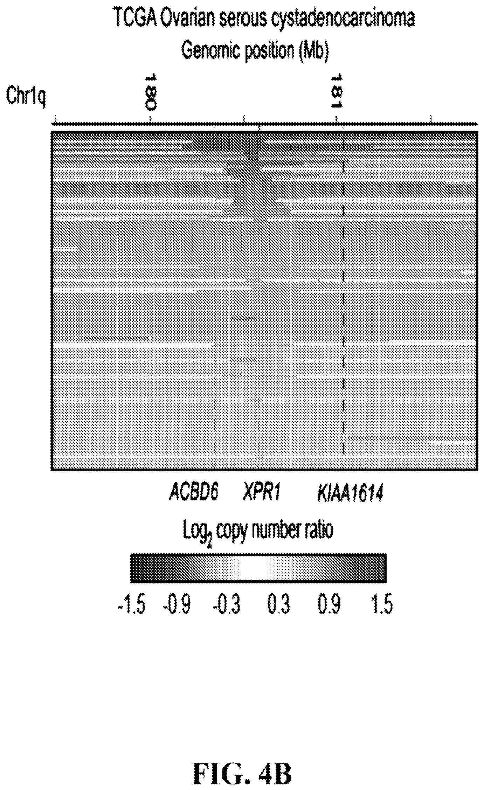

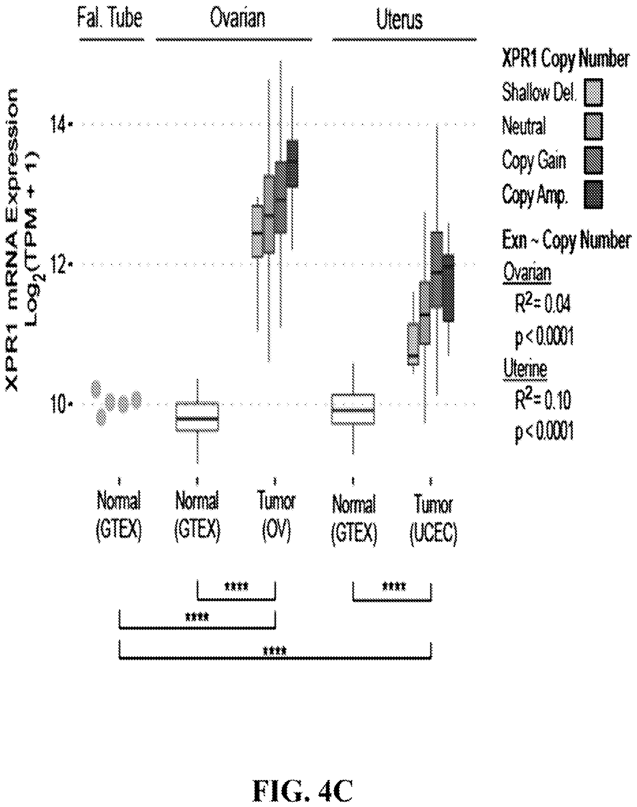

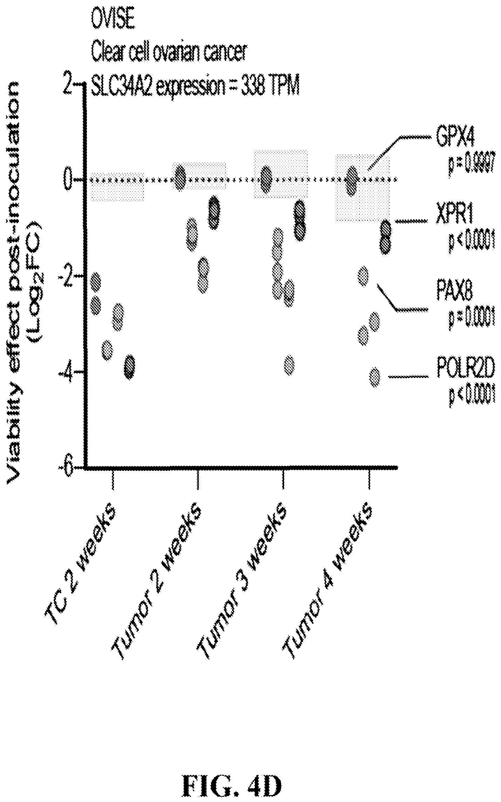

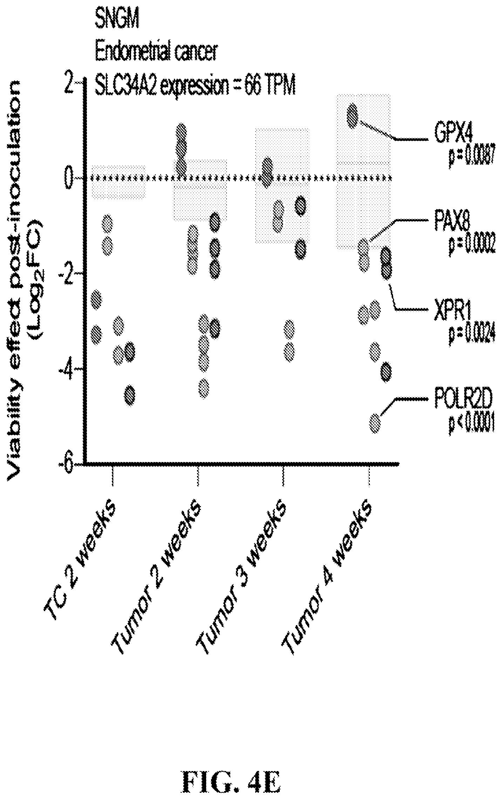

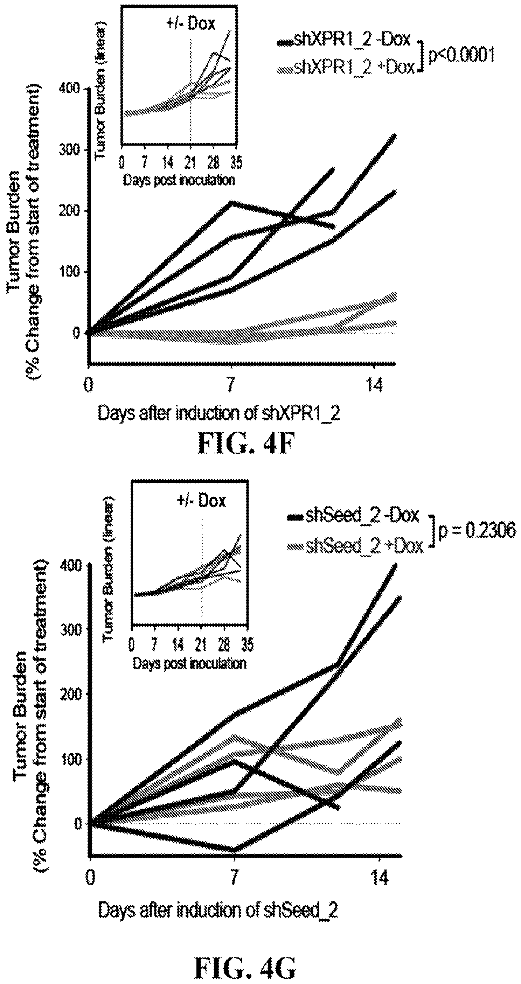

**FIG. 4** **- XPR1 and SLC34A2 are overexpressed in patient samples and XPR1 dependency is retained in mouse xenograft studies. a.** SLC34A2 mRNA expression is compared in normal (GTEx), tumor (TCGA), and cancer cell line (CCLE) samples. For CCLE cell lines, the XPR1-dependency status (CERES < -0.5) is indicated. Note that Fallopian tube is the tissue of origin for many ovarian/uterine cancers. **b.** XPR1 copy number heatmap for a ~2.5 Mb region of chromosome 1 indicating XPR1 amplification in serous ovarian cancer (TCGA OV). **c.** As in panel a, XPR1 mRNA expression is compared across the indicated tissues. For TCGA samples, the XPR1 copy number status (GISTIC) is indicated. **d.** In a CRISPR/Cas9 competition tumor formation assay in the ovarian cancer cell line OVISE, the depletion of the XPR1 and the indicated genes is plotted. GPX4 was used as a metabolic dependency gene. PAX8 is a benchmark dependency in many ovarian cancers. POLR2D is a pan-essential positive control. The gray boxes represent the variability in 15 different control sgRNA which target non-gene sites in the human genome. **e.** Same as panel d, but with the uterine cancer cell lines SNGM. **f.** The tumor growth of a model of disseminated ovarian carcinomatosis after XPR1 suppression using doxycycline-inducible shXPR1_2. The inset shows the full growth curves on a linear scale, while the larger image is the percent growth after treatment. **g.** Same as in f, but with the non-targeting shSeed_2.

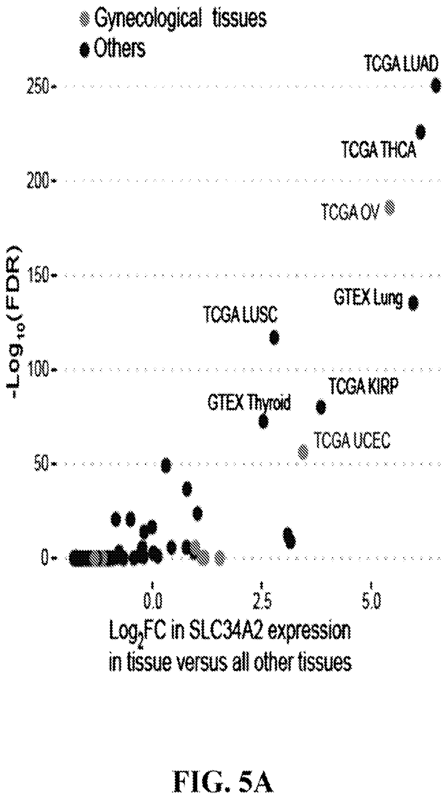

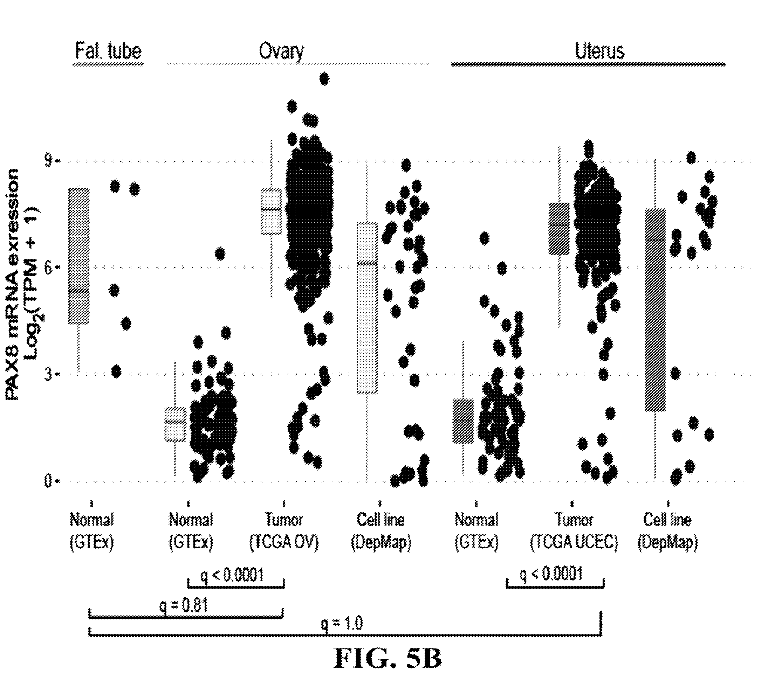

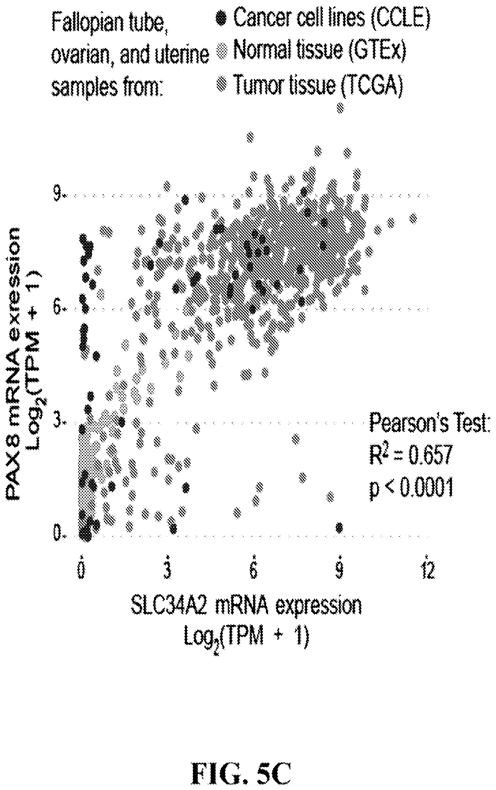

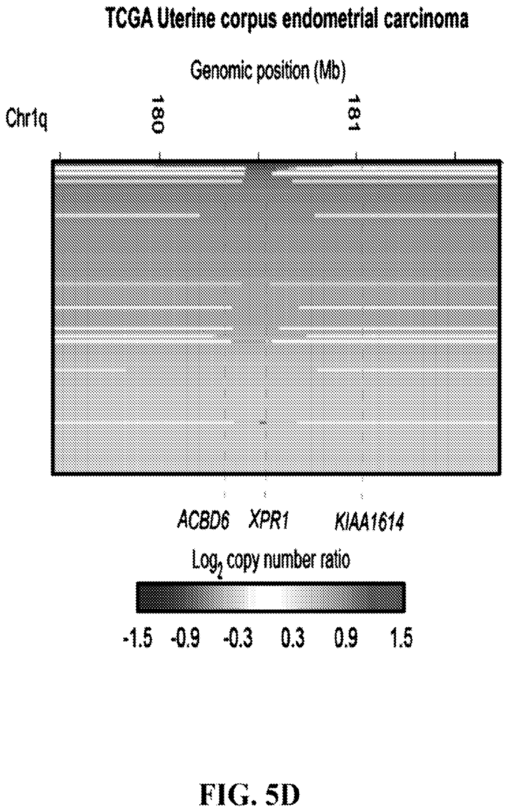

**FIG. 5** **- SLC34A2 and XPR1 overexpression in ovarian cancer are likely driven by PAX8. a.** Comparison of SLC34A2 expression across tissues. Using the combined GTEx, TCGA, and CCLE dataset as in **Figure 4a** **and c,** the differential expression of SLC34A2 in each tissue relative to all other tissues is compared. The relevant gynecological tissues (fallopian tube, ovary, and uterus) are highlighted in teal. **b.** PAX8 expression is increased in tumor samples relative to normal tissues. In the same way as in **Figure 4a****,** PAX8 expression is compared across the indicated tissue samples. q-values indicate the likelihood of the indicated populations having the same PAX8 expression according to a Wilcoxon ranked sums test with Bonferroni correction for multiple comparisons. **c.** PAX8 and SLC34A2 expression are highly correlated in fallopian tube, ovarian and uterine tissues. **d.** XPR1 copy number heatmap for a ~2.5 Mb region of chromosome 1 indicating XPR1 amplification in TCGA Uterine Corpus Endometrial Carcinoma²⁸. Each patient sample is represented by a horizontal line. Red indicates copy gain and blue indicates copy loss. Dashed vertical lines are the location of indicated genes. Data are a subset of the samples with rank ordered by highest copy gain to indicate both focal and chromosome arm-level gains.

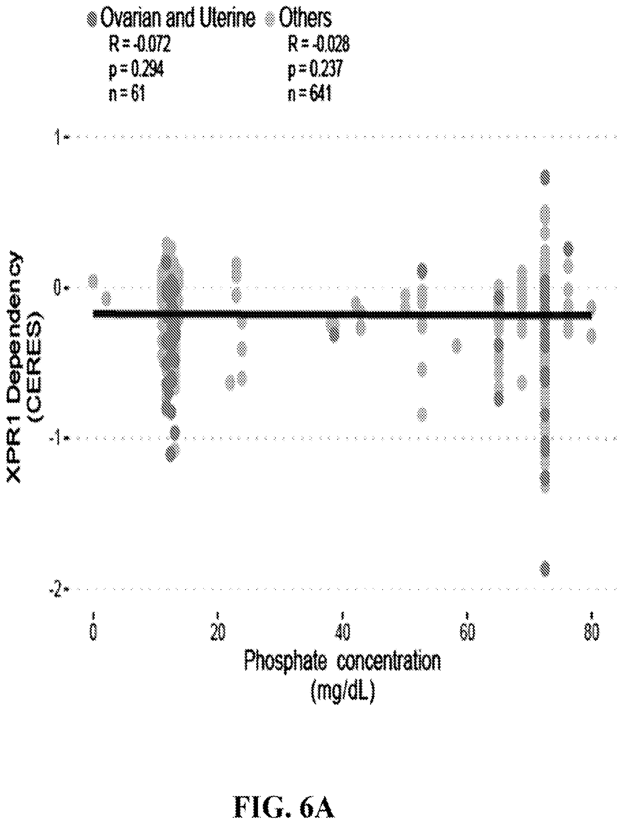

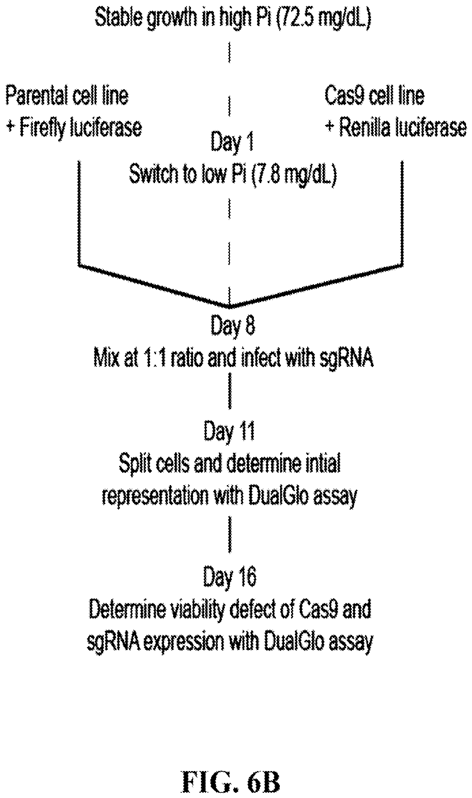

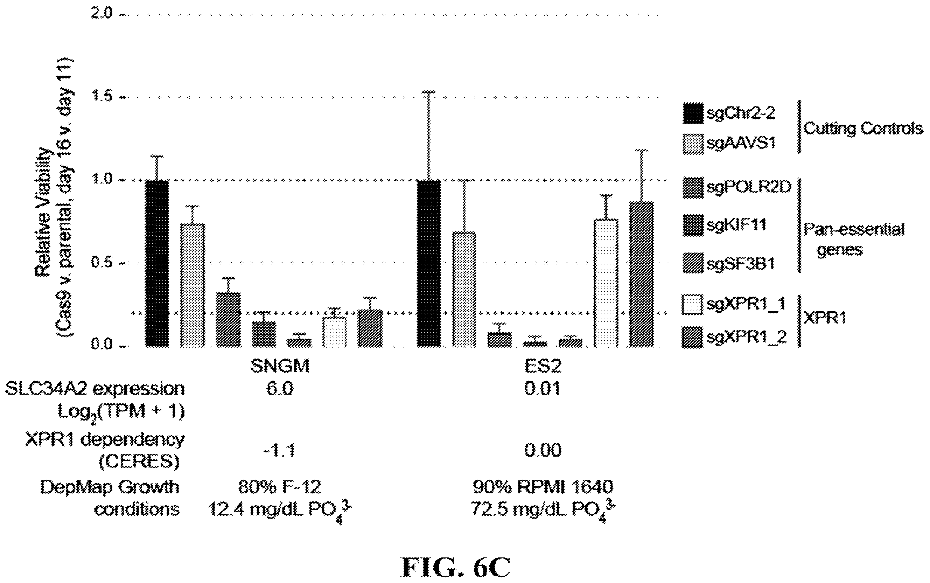

**FIG. 6** **- The XPR1 dependency is not affected by phosphate levels in the tissue culture medium. a.** The concentration of phosphate in the growth medium of Dependency Map cell lines does not determine the extent of XPR1 dependency. Concentrations of phosphate were estimated from manufacturer formulations (see methods). **b.** Experimental procedure for manipulating tissue culture medium and assessing its effect on XPR1 dependency. The same parental cancer cell line was engineered to express firefly luciferase and Cas9, or renilla luciferase alone. After one week of growing the cell lines in low-phosphate RPMI 1640, the two variants were mixed together and infected with sgRNA-encoding lentivirus. After selection for lentivirus-infected cell lines, the initial representation of Cas9:parental cells was determined by measuring the ratio of Firefly:Renilla luciferase using a DualGlo assay (Promega). One week after infection (Day 16 of the protocol), the extent to which genetic perturbation was detrimental to cell viability was determined using the DualGlo. **c.** The XPR1 dependency is maintained in a low phosphate medium condition. SNGM (endometrial cell line dependent on XPR1) and ES2 (clear cell ovarian cell line without XPR1 dependency) were profiled in the assay outlined in panel b. Note that the CERES score - displayed below the plot - represents the viability defect of the cell line 21 days after knockout of XPR1 and growth in the indicated growth medium.

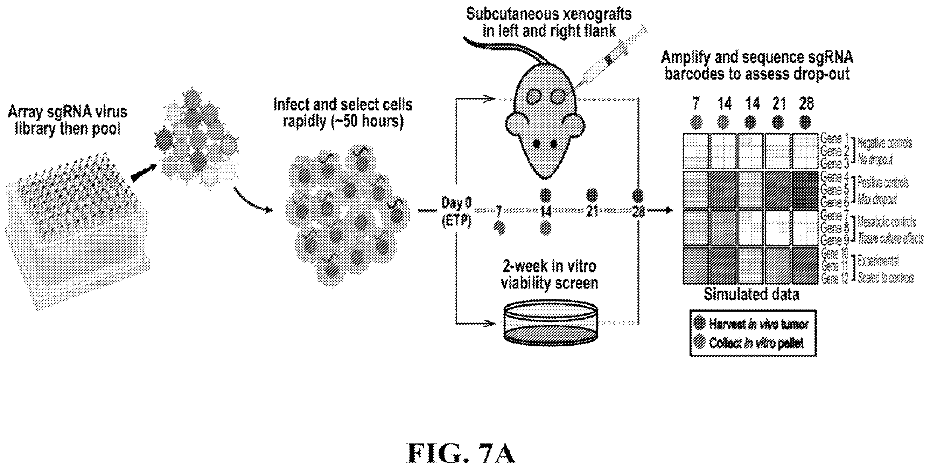

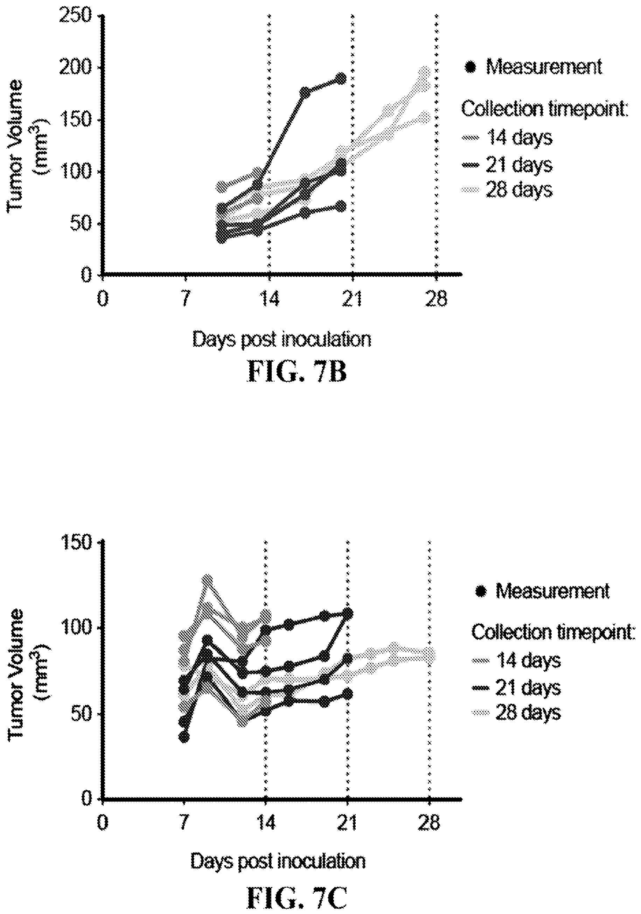

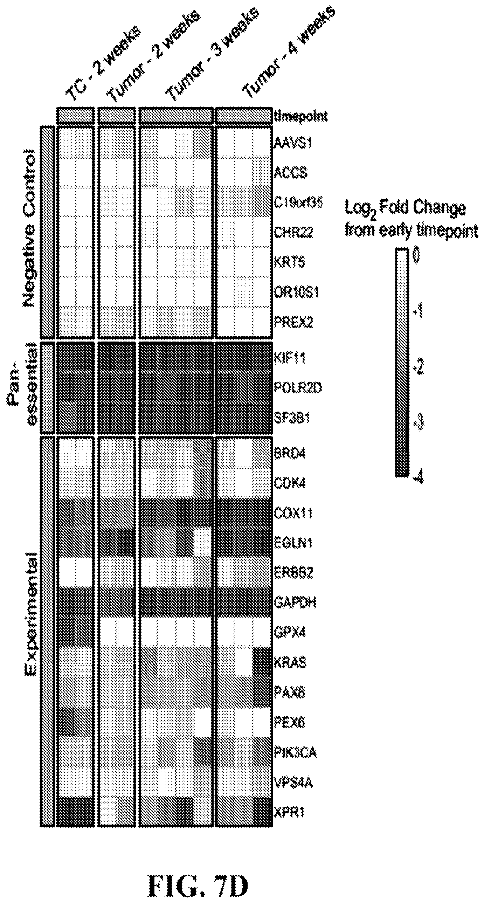

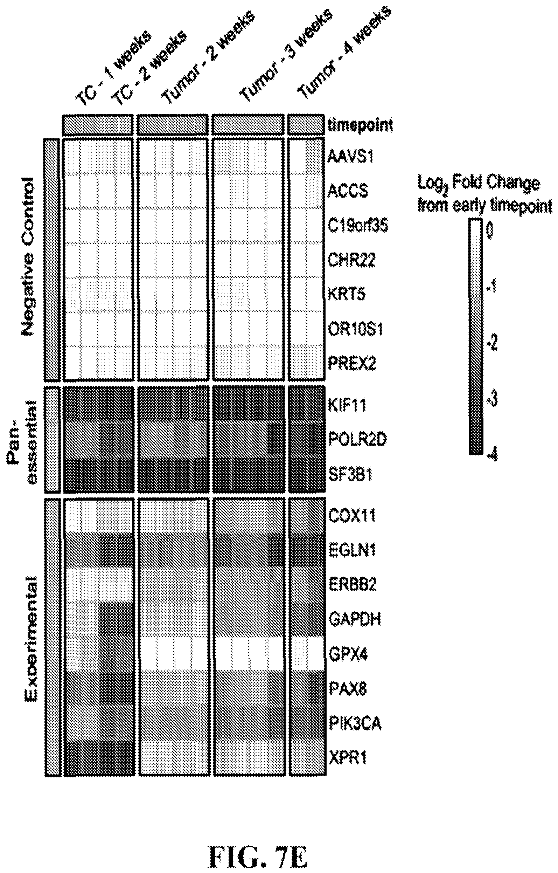

**FIG. 7** **- In vivo CRISPR/Cas9 competition assays for target validation in mouse xenografts. a.** Experimental design for in vivo competition assays. Using a rapid infection and selection protocol, pooled sgRNA can be introduced via lentivirus into cancer cell lines and inoculated as subcutaneous xenograft and the effect of gene knockout can be evaluated in a more physiologically-relevant environment than tissue culture. **b.** After rapid infection with pooled sgRNA, 8 million SNGM cells were inoculated as subcutaneous xenografts and allowed to grow. Tumor tissue was harvested at the indicated time points. c. Same as in d, but for the experiment using the OVISE cancer cell line. **d.** sgRNA abundance in tumor xenografts was evaluated by PCR and next-generation sequencing analysis, and the fold change compared to the early time point is shown as a heatmap for all of the negative control genes as well as any gene with a >4 fold change in abundance in any of the screens. Same as in d, but for the experiment using the OVISE cancer cell line.

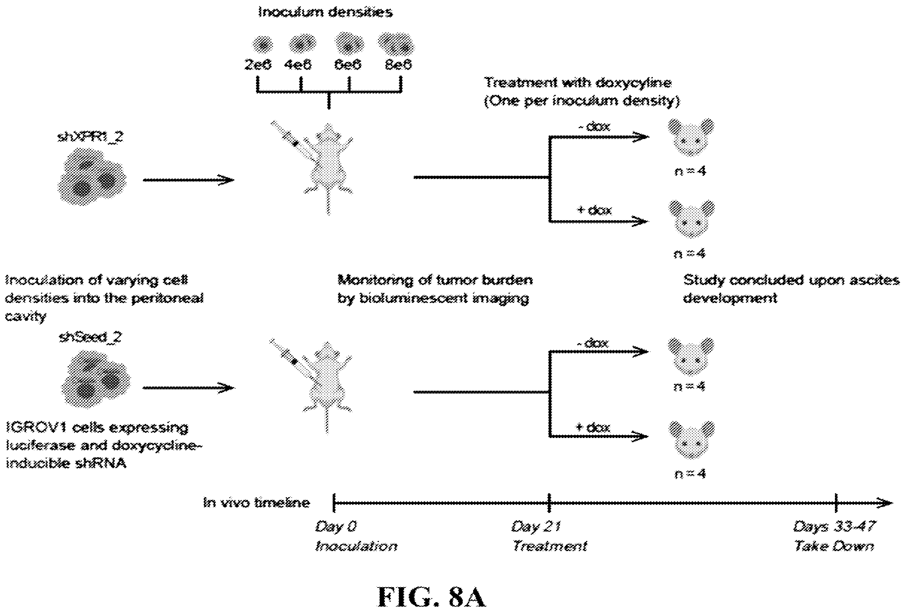

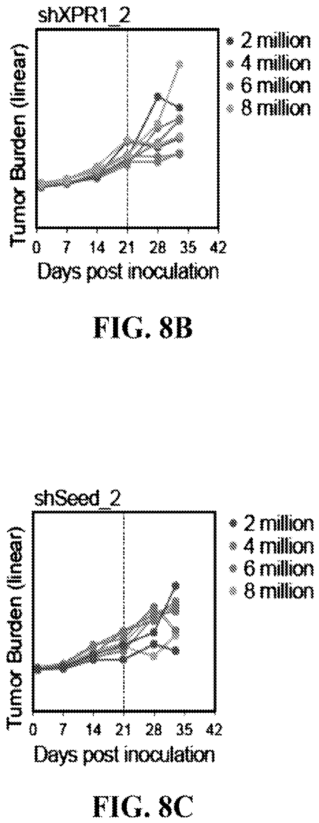

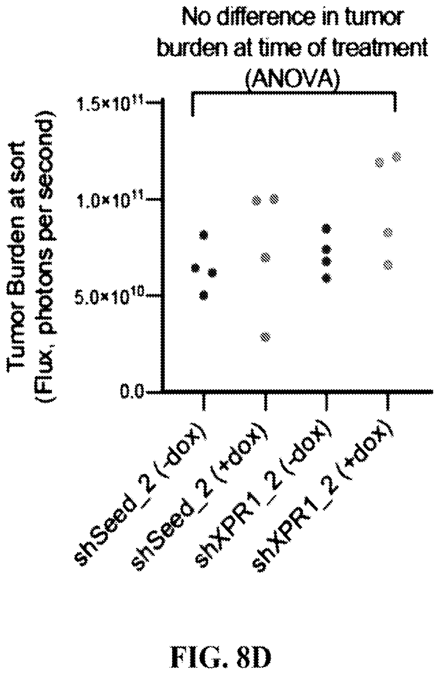

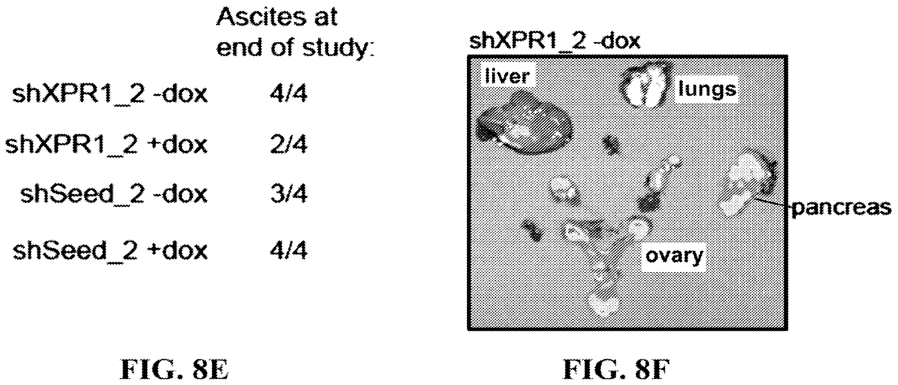

**FIG. 8** **- XPR1 suppression halts growth in disseminated ovarian carcinomatosis. a.** Experimental design to assess the effect of XPR1 suppression on established intraperitoneal ovarian carcinomatosis. IGROV1 constitutively expressing luciferase were engineered to inducibly express shXPR1_2 (to suppress XPR1) or shSeed_2 (control). Because the in vivo growth kinetics for the model were unknown, 4 different cell densities were inoculated in the peritoneal cavity of mice and tumor growth was monitored using bioluminescent imaging. After three weeks of tumor growth, one mouse from each inoculation group was fed with doxycycline chow to induce expression of shRNA. Animals developed ascites within 2-3 weeks after treatment, and so the study was terminated. **b.** The growth rate of IGROV1 cells engineered with shXPR1_2 was not dependent on the number of cells inoculated. **c.** Same as in panel b, but with IGROV1 cells engineered with shSeed_2. **d.** At the time of treatment, the tumor burden was equivalent across the four different groups. **e.** Number of animals per treatment and shRNA group which had developed ascites. **f.** IGROV1 cells, constitutively expressing luciferase, are detected on the indicated organs after study termination. Here, the cells were taken from a mouse from the shXPR1_2 -Dox group.
**FIG. 9** - **Transcriptional profiling highlights a phosphate homeostatic response to XPR1 inactivation in SLC34A2 overexpressing ovarian cancer. a.** At various time-points after treatment with doxycycline and induction of shRNA, the intracellular phosphate was measured in OVISE and IGROV1 cell lines. **b.** UMAP projection of MixSeq scRNA-sequencing results to compare multiplexed cancer cells after XPR1 inactivation. **c.** Middle, the log-fold change of the top 500 differentially expressed genes after regressing out the effect of cell cycle. Left, summary annotations for each cell line include XPR1 dependency (XPR1 CERES), the overall transcriptional change (Average LFC), and the degree of cell cycle arrest observed in the single cell data (ΔG0/G1). Right, the correlation between cell lines of the overall transcriptional change. **d.** The measured transcriptional change in the indicated genes are plotted for the highly correlated cell lines (see panel c) on the left and the other five cell lines on the right. **e.** Quantitation of SLC34A2 protein levels (measured by Protein Simple) after XPR1 inactivation. **f.** Radioactive phosphate uptake was measured in the OVISE ovarian cancer cell line after inactivation of the indicated genes.

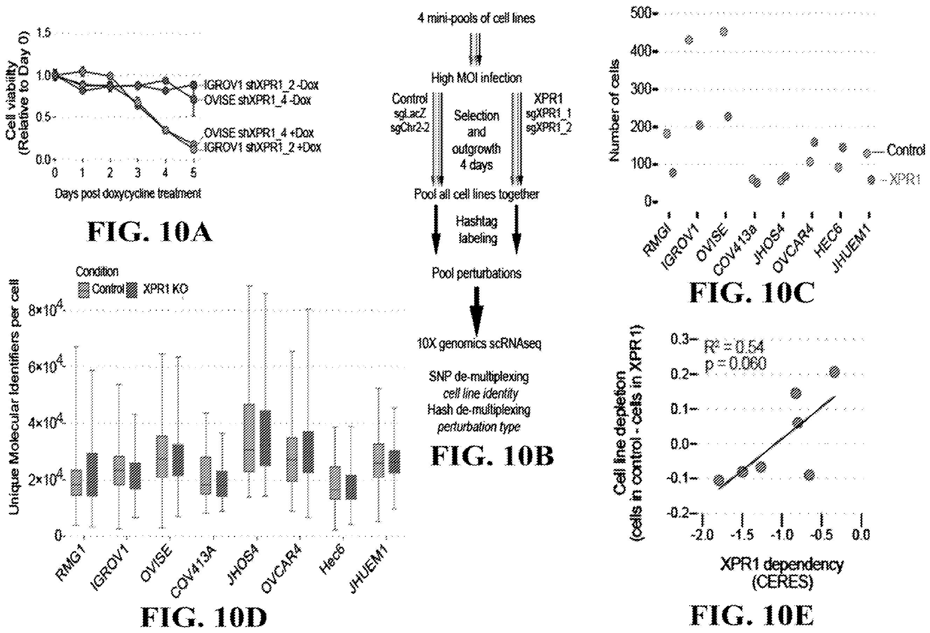

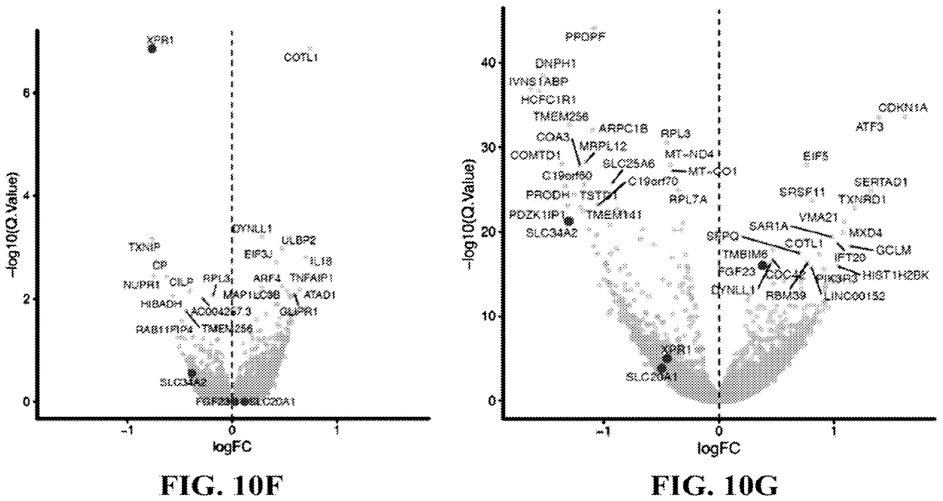

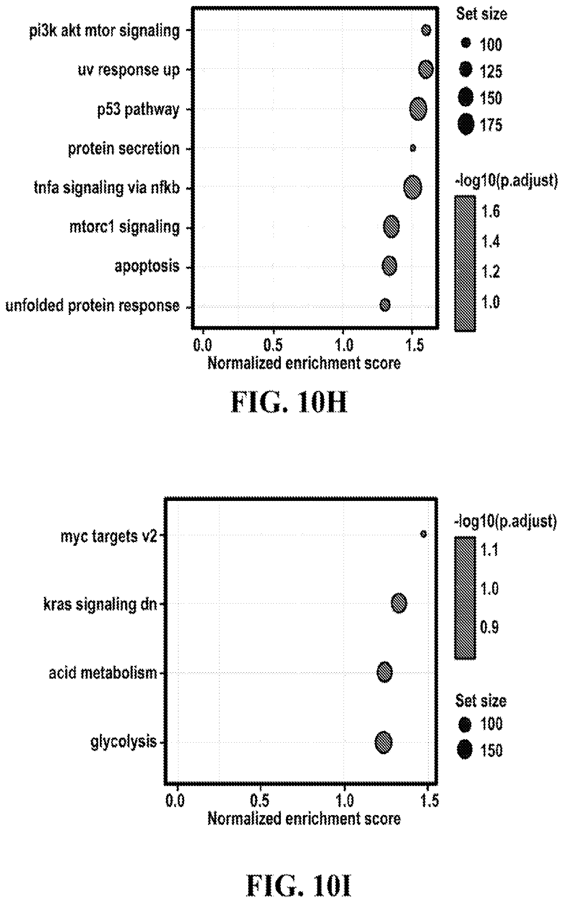

**FIG. 10** **- Transcriptional profiling reveals a phosphate-related homeostatic response after XPR1 suppression. a.** The viability of cells (as measured by total protein content) was measured in parallel with total phosphate. The total intracellular phosphate reported in **Figure 9a** refers to total phosphate measured divided by cellular viability. **b.** Experimental workflow to determine the transcriptional profile of XPR1 inactivation across many different cancer cell lines. Cell lines were pooled according to their doubling time and infected in small pools with control or XPR1 sgRNA at a high multiplicity of infection (MOI) in order to maximize penetrance of XPR1 inactivation. The day after infection, cells were selected with puromycin and then grown until the fourth day after infection. At this point, the cells were counted and pooled per perturbation and incubated with cell-surface labeling antibodies containing RNA "hash-tags." Cells across perturbations were then pooled and subjected to the 10X genomics single cell RNA sequencing (scRNAseq) pipeline. The single nucleotide polymorphisms (SNP) profile for each cell was used to assign it to a particular cell line, while the "hash-tag" barcode was used to determine which perturbation that cell had received. **c.** The total number of cells per cell line. Differences are likely due to outgrowth of individual cell lines within each pool or differences in sensitivity to the high MOI used. **d.** The total number of unique transcripts measured for each cell, as measured by unique molecular identifiers (UMIs) which were included in the preparation of the library before amplification and next generation sequencing. **e.** The number of cells collected per cell line was compared between the two conditions (control and XPR1 inactivation) to determine if a viability defect is observed in the ~4 day timeframe of the experiment. **f.** After cell cycle regression, genes which were increased in expression after XPR1 inactivation within dependent cell lines (IGROV1, EFE184, OVISE, RMGI, OVMANA, and OVCAR4) were analyzed by gene set enrichment. **g.** Same as in f, but for genes which were decreased after XPR1 inactivation. **h.** Four days after induction of shXPR1_2 (IGROV1) or shXPR1_4 (OVISE) using doxycycline, the amount of secreted FGF23 was measured in the conditioned medium using ELISA. Note that SLC34A2 suppression is observed at this time point.

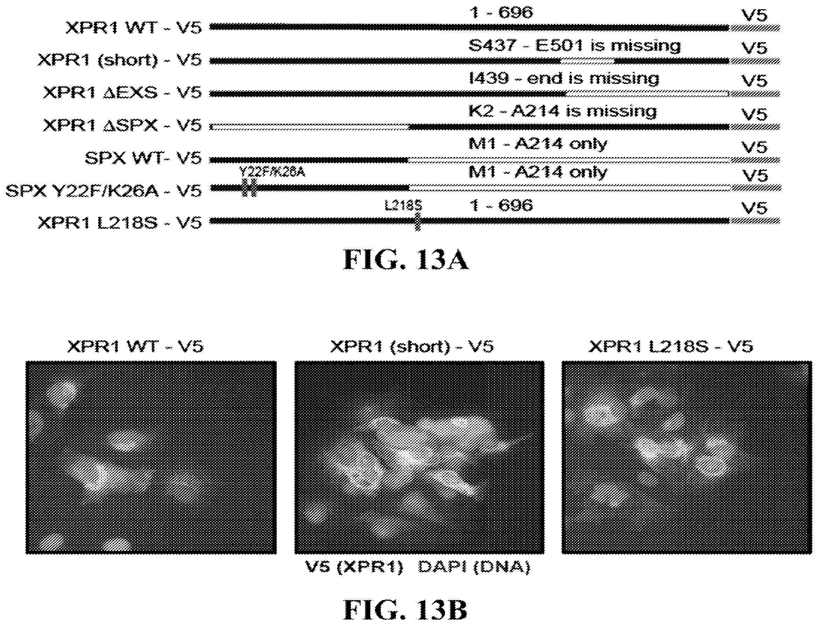

**FIG. 11** **- The phosphate efflux capacity of XPR1 is required for viability in SLC34A2-overexpressing cancer cell lines. a.** Across 737 cancer cell screened (DepMap Avana 20Q1), the viability defects of XPR1 or KIDINS220 inactivation is plotted. **b.** After expression of the indicated XPR1 open reading frames in HEK-293Ts, the interaction between the V5-tagged ORF and KIDINS220 was evaluated using co-immunoprecipitation. See **Figure 13a** for the design of these constructs. **c.** After genetic inactivation of XPR1 or KIDINS220, the localization of XPR1-V5 was determined using immunofluorescence. Note that sgXPR1_1 inactivates both endogenous XPR1 and the XPR1-V5 ORF, and so no staining should be expected. **d.** Three days after genetic inactivation of XPR1 or KIDINS220, cells were loaded with radioactive phosphate, and then efflux was measured for 30 minutes after. **e.** The indicated XPR1 constructs were tested for their ability to rescue knockout of endogenous XPR1. The L218S mutation in XPR1 has previously been shown to only have ~50% the efflux capacity of XPR1. **f.** Phase contrast images of 'vacuole-like' phenotype 4-5 days after XPR1 inactivation. Arrowheads indicate the location of 'vacuole-like' structures. Scale bars = 200 um. **g.** The acidic dye Lysotracker was used to stain live cells five days after inactivation of XPR1. **h.** Transmission electron micrographs of "vacuole-like" structures (labeled V) or lysosomes (Lys) in OVISE cancer cells after XPR1 inactivation.

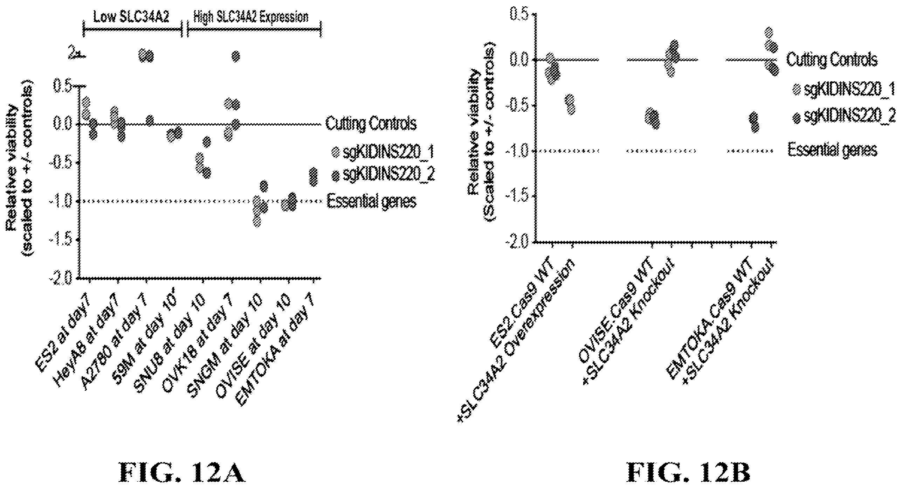

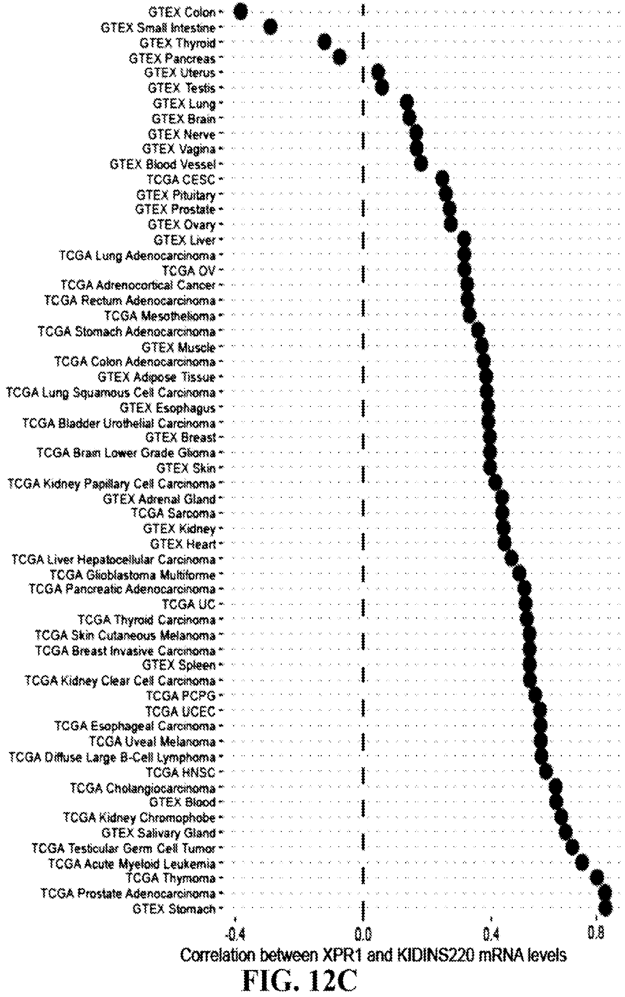

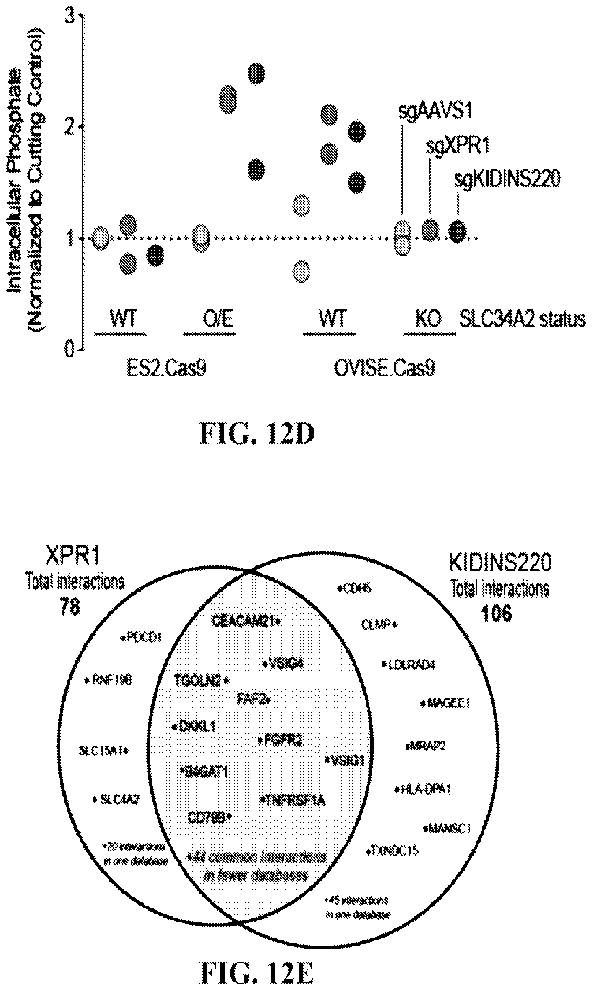

**FIG. 12** **- XPR1 and KIDINS220 are co-expressed and co-precipitated in large-scale datasets. a.** Validation of KIDINS220 dependency was performed in ovarian and uterine cancer cell lines with a range of SLC34A2 expression as in **Figure 1d****. b.** SLC34A2 expression is necessary and sufficient for KIDINS220 dependency, performed as in **Figure 1f****. c.** XPR1 and KIDINS220 mRNA levels are highly correlated across many tissues. Using GTEx, TCGA, and CCLE data, the Pearson correlation was calculated. **d.** XPR1 and KIDINS220 suppression both cause increased intracellular phosphate, which is dependent on SLC34A2 expression. Five days after infection with the indicated sgRNA, intracellular phosphate was determined as in **Figure 9a****. e.** High throughput protein:protein interaction databases implicate XPR1 and KIDINS220 as part of a protein complex. The interacting partners of XPR1 and KIDINS220 were downloaded from the BioGrid and Bioplex databases and compared. Genes which were present in the interactomes of XPR1 or KIDINS220 consistently across multiple datasets are called out as text.

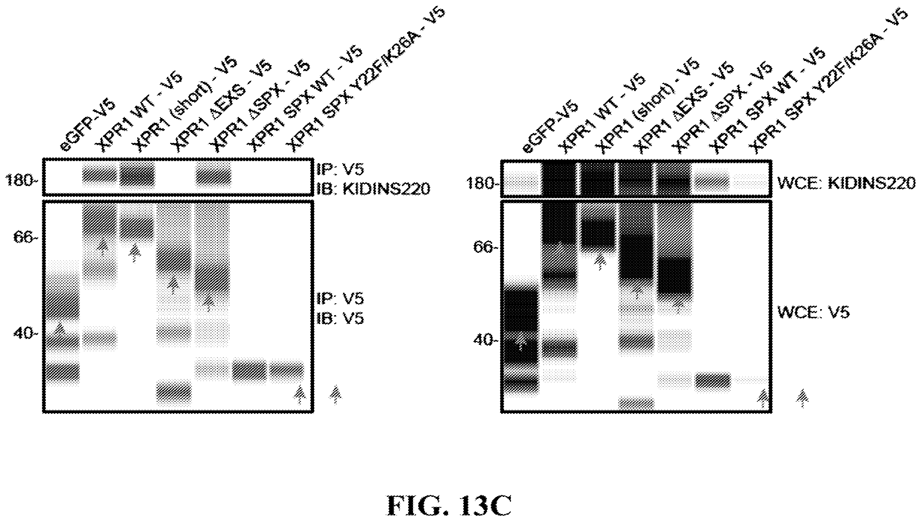

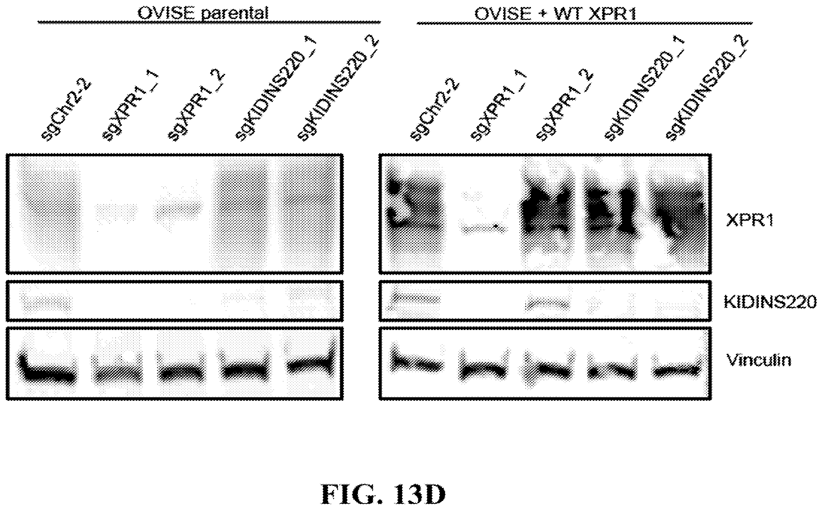

**FIG. 13** **- XPR1 domain mutants vary in localization and KIDINS220 a.** XPR1 WT refers to the 696 amino acid protein produced by NM_004736 (the only isoform detected) , while XPR1 (short) refers to the 631 amino acid product of NM_001135669. All constructs have C-terminal V5 tags for immunoprecipitation, western blotting, and immunofluorescent detection. **b.** Immunofluorescent localization of some of the XPR1 mutants tested using the V5 epitope tag. Left, WT XPR1 localizes to the secretory pathways as well as puncta within the cytoplasm. Middle, XPR1 (short) staining appears to be far more diffuse. The L218S mutation has the same localization pattern as WT XPR1, consistent with proper trafficking, similar to what has been observed previously (see main text). **c.** Co-immunoprecipitation of XPR1 domain mutants with endogenous KIDINS220 in 293s. This is an uncropped version of **Figure 9b****,** including the SPX domain only constructs (lanes 6-7). Right, the whole cell extract (WCE) from the experiment, indicating differing expression levels of KIDINS220. It should be noted that much of the background signal is attributed to immunoblotting for endogenous XPR1 in the same experiment. Green arrows indicate the expected molecular weight of the ORF, in contrast to degradation products. **d.** Western blot validation of guide-resistant ORF. OVISE.Cas9 cells (parental, left, or overexpressing the WT XPR1 ORF, right, used in **Figure 9e****)** were infected with the indicted sgRNA and harvested 5 days after infection. The XPR1 ORF includes a mutation to block binding of sgXPR1_2 but not sgXPR1_1 (note the suppression of both isoforms with sgXPR1_1 but only endogenous XPR1 with sgXPR1_2).

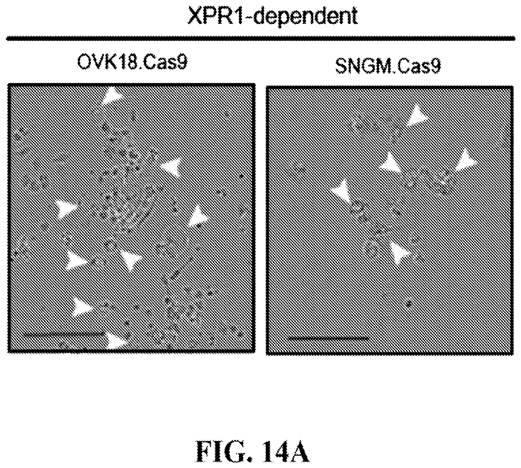

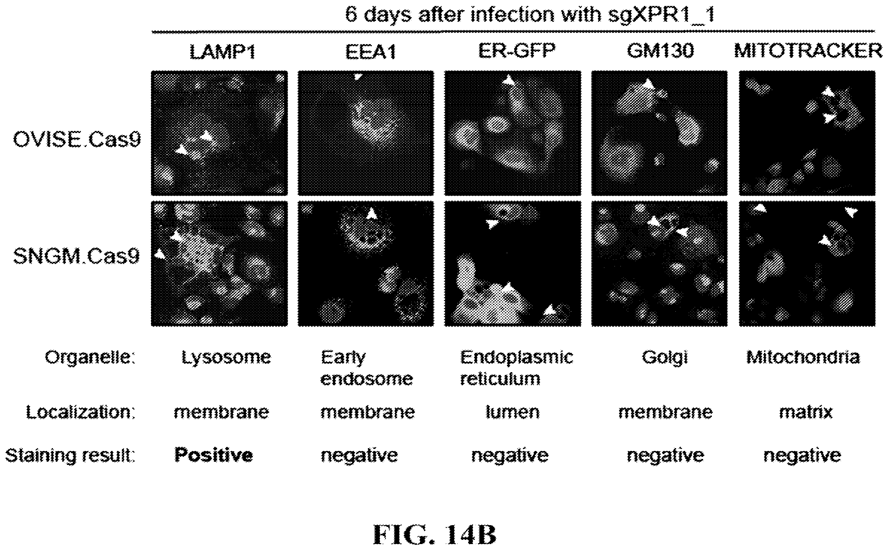

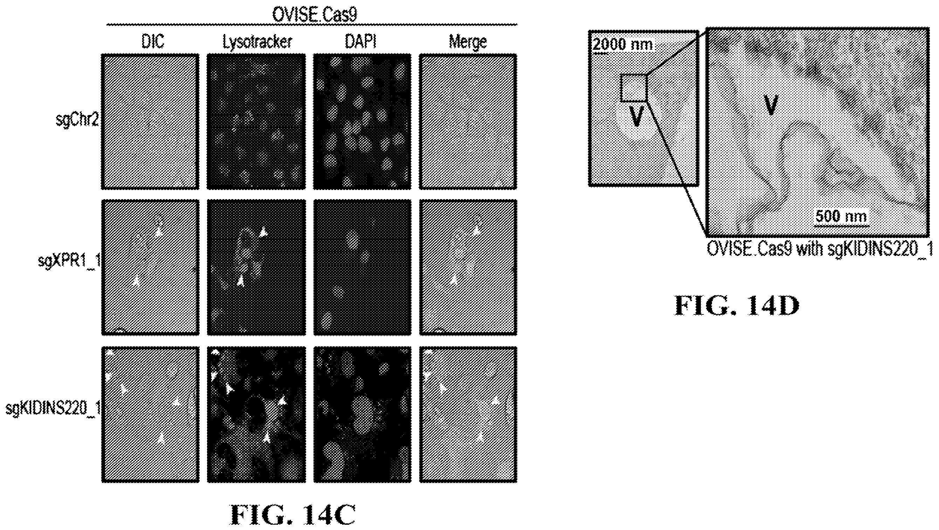

**FIG. 14** **- Common organellar stains do not label "vacuoles". a.** 6 days after infection with lentivirus encoding sgXPR1_2, OVISE.Cas9 and SNGM.Cas9 cell lines were stained and imaged using the indicated dyes and stains. Arrowheads indicate the location of vacuole structures by phase contrast (not pictured). Positive staining was only observed for the lysosomal dye LAMP1. **b.** Phase contrast images of 'vacuole-like' phenotype 4-5 days after XPR1 inactivation. Arrowheads indicate the location of 'vacuole-like' structures. Scale bars = 200 um. **c.** Live cell DIC and confocal immunofluorescence images of XPR1 dependent cell line OVISE 5 days after CRISPR inactivation of XPR1 or KIDINS220 vs control sgRNA (sgChr2-2). Acidic organelles were detected with Lysotracker (red) and DNA with DAPI (blue). Arrowheads indicate the location of 'vacuole-like' structures. **d.** Transmission electron micrographs of OVISE.Cas9 5 days after XPR1 inactivation. Scale bars are indicated within the figure. Lysosome and "vacuole-like" structures are indicated by "Lys" and "V" respectively. **e.** Same as in c, but with KIDINS220 inactivation.

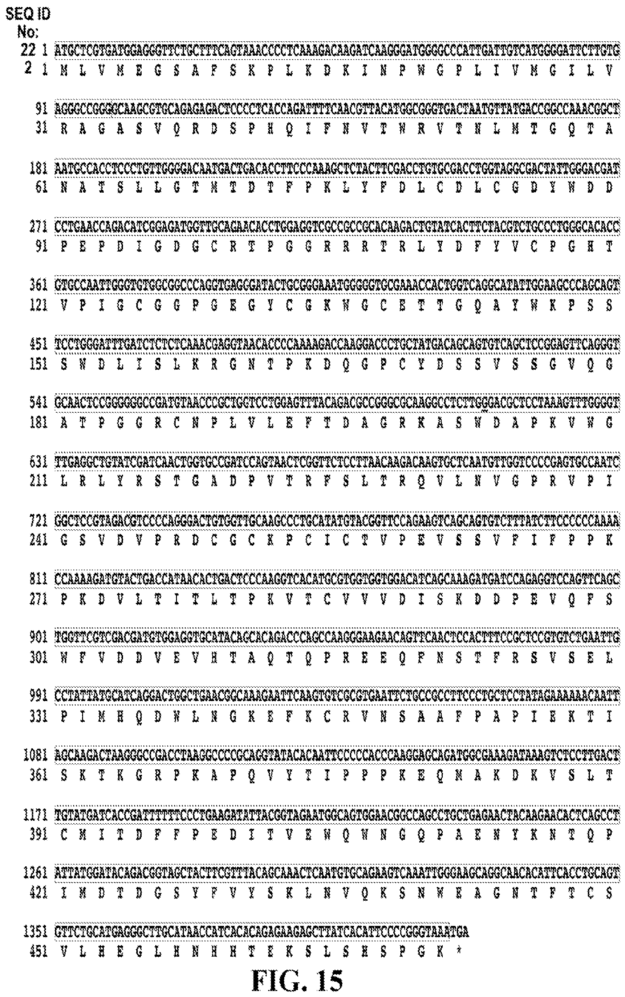

**FIG. 15** - Exemplary design of RBD fusion protein (51.2 kDa). The protein includes 33 residue signal sequence, 207 residue RBD from NZB strain, 4 residue linker (GSVD), and 227 residue Fc from mouse IgG1.

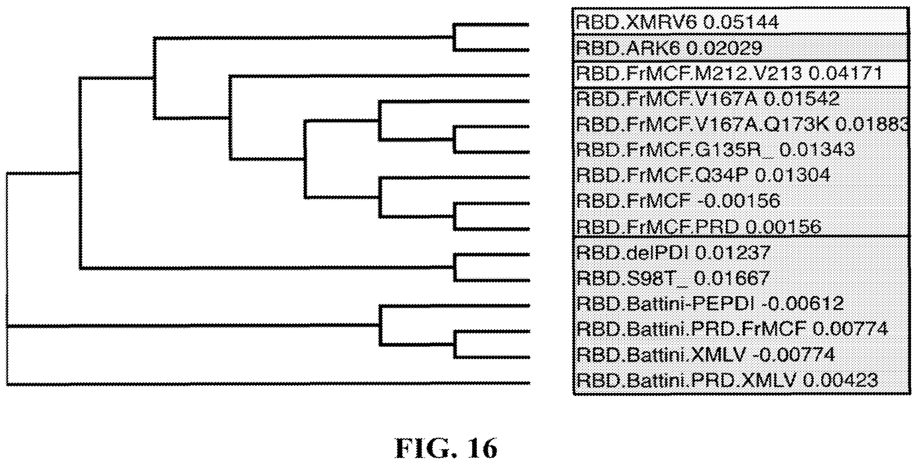

**FIG. 16** - Taxonomy of other viral strains that can infect murine XPR1.

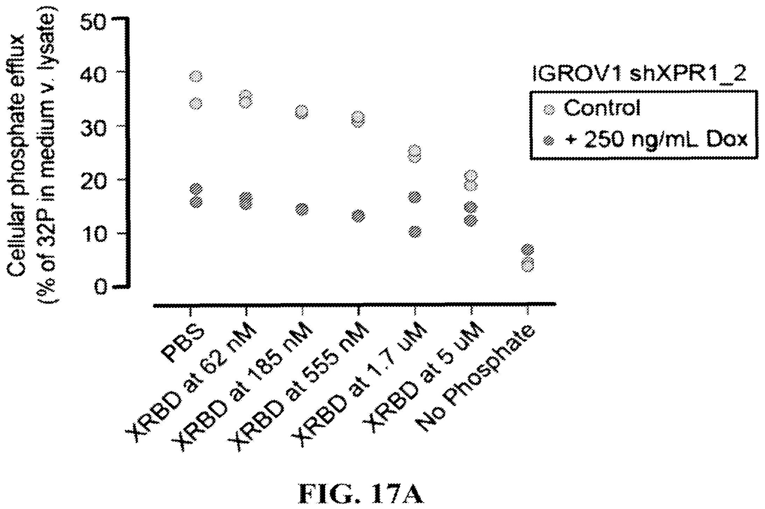

**FIG. 17A-17G** **- XRBD is a drug-like inhibitor of the XPR1 and KIDINS220 protein complex. a)** XRBD protein inhibits XPR1-dependent phosphate efflux. Phosphate efflux was measured by incubating IGROV1 ovarian cancer cell lines with RPMI without phosphate supplemented with ³²P-labeled phosphate, washing the cells extensively, and then incubating the cells in standard RPMI (~10 mM phosphate). After 30 minutes, the conditioned medium was collected, and the cells were washed and then lysed. Phosphate efflux was calculated as the percent of ³²P measured in the conditioned medium relative to the total 32P measured in the lysate and conditioned medium. Where indicated, the cells were pretreated for 72 hours with 250 ng/mL Doxycycline to induce XPR1 suppression via shXPR1_2. Where indicated, the cells were treated with varying doses of XRBD protein during the ³²P uptake and efflux portions of the experiment. Where indicated, "no phosphate" RPMI was used during the efflux portion to prevent the cells from exporting any ³²P. **b)** Western blot confirmation of stable knockout of XPR1 or KIDINS220 in 293T cells. 293T cells were transiently transfected with an 'all-in-one' plasmid containing both Cas9 and sgRNA targeting XPR1 or KIDINS220. Clonal populations were isolated by limiting dilution and analyzed by western blot. Wildtype human XPR1 (hXPR1) was re-expressed in the knockout background as a control. **c)** XRBD flow cytometry analysis of the 293T cells analyzed in b. The cells were incubated with 20 nM XRBD-mFc for 30 minutes at 37c, followed by extensive washing and incubation with an antimouse secondary conjugated to AlexaFluor488 (Invitrogen). Left, histograms from at least 10,000 single cells. Right, the median fluorescent intensity of the populations shown on left is displayed as a heatmap. **d)** XRBD treatment causes viability defects in ovarian cancer cell lines. The indicated cell lines were treated with the XRBD protein at the indicated concentrations and cellular viability was assessed five days later by Cell Titre Glo (CTG, Promega). Right, a heatmap comparing each cell lines' XPR1 inactivation sensitivity (assessed by CRISPR viability assays) and XRBD sensitivity (decrease in cellular viability after five day treatment with the top dose of XRBD relative to vehicle control). Below, the Pearson correlation coefficient between XPR1 inactivation sensitivity and XRBD treatment. **e)** Western blot analysis and XRBD treatment in a small panel of lung cancer cell lines. 5 days after inactivation of XPR1 or SLC34A2 in the indicated cell lines, XPR1 or SLC34A2 protein levels were analyzed by western blot. Below, cellular viability was assessed as in d. **f)** Glycerol gradient sedimentation analysis of XPR1-contatiing native protein complexes with or without KIDINS220 inactivation. 293T cells, or the stable KIDINS220 knockout cell lines profiled in b were lysed, and 1 mg of lysate was layered onto 10-30% glycerol gradients and spun at 55,000 RPM in a SW55 rotor for 3.5 hours at 4c to separate protein complexes by molecular weight. 24 fractions were collected from each gradient in which low molecular weight complexes correspond to low fraction numbers (i.e., 1-7) and larger protein complexes are at larger numbers. XPR1 was detected in fractions by immunoblot. **g)** RT-PCR validation of FGF23. 2 or 4 days after induction of the indicated shRNA with doxycycline, RNA was extracted from cells, reverse-transcribed into cDNA, and levels of XPR1, FGF23, or Vinculin (housekeeping control) were quantified by gene-specific primers. The data shown were normalized to Vinculin and are displayed as the Log2 fold-change relative to the matched timepoint without doxycycline treatment (Ctl).

The figures herein are for illustrative purposes only and are not necessarily drawn to scale.

### DETAILED DESCRIPTION OF THE EXAMPLE EMBODIMENTS

### General Definitions

Unless defined otherwise, technical and scientific terms used herein have the same meaning as commonly understood by one of ordinary skill in the art to which this disclosure pertains. Definitions of common terms and techniques in molecular biology may be found in Molecular Cloning: A Laboratory Manual, 2nd edition (1989) (Sambrook, Fritsch, and Maniatis); Molecular Cloning: A Laboratory Manual, 4th edition (2012) (Green and Sambrook); Current Protocols in Molecular Biology (1987) (F.M. Ausubel et al. eds.); the series Methods in Enzymology (Academic Press, Inc.): PCR 2: A Practical Approach (1995) (M.J. MacPherson, B.D. Hames, and G.R. Taylor eds.): Antibodies, A Laboratory Manual (1988) (Harlow and Lane, eds.): Antibodies A Laboratory Manual, 2nd edition 2013 (E.A. Greenfield ed.); Animal Cell Culture (1987) (R.I. Freshney, ed.); Benjamin Lewin, Genes IX, published by Jones and Bartlet, 2008 (ISBN 0763752223); Kendrew et al. (eds.), The Encyclopedia of Molecular Biology, published by Blackwell Science Ltd., 1994 (ISBN 0632021829); Robert A. Meyers (ed.), Molecular Biology and Biotechnology: a Comprehensive Desk Reference, published by VCH Publishers, Inc., 1995 (ISBN 9780471185710); Singleton et al., Dictionary of Microbiology and Molecular Biology 2nd ed., J. Wiley & Sons (New York, N.Y. 1994), March, Advanced Organic Chemistry Reactions, Mechanisms and Structure 4th ed., John Wiley & Sons (New York, N.Y. 1992); and Marten H. Hofker and Jan van Deursen, Transgenic Mouse Methods and Protocols, 2nd edition (2011).

As used herein, the singular forms "a", "an", and "the" include both singular and plural referents unless the context clearly dictates otherwise.

The term "optional" or "optionally" means that the subsequent described event, circumstance or substituent may or may not occur, and that the description includes instances where the event or circumstance occurs and instances where it does not.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of +/-10% or less, +/-5% or less, +/-1% or less, and +/-0.1% or less of and from the specified value, insofar such variations are appropriate to perform in the disclosed invention. It is to be understood that the value to which the modifier "about" or "approximately" refers is itself also specifically, and preferably, disclosed.

As used herein, a "biological sample" may contain whole cells and/or live cells and/or cell debris. The biological sample may contain (or be derived from) a "bodily fluid". The present invention encompasses embodiments wherein the bodily fluid is selected from amniotic fluid, aqueous humour, vitreous humour, bile, blood serum, breast milk, cerebrospinal fluid, cerumen (earwax), chyle, chyme, endolymph, perilymph, exudates, feces, female ejaculate, gastric acid, gastric juice, lymph, mucus (including nasal drainage and phlegm), pericardial fluid, peritoneal fluid, pleural fluid, pus, rheum, saliva, sebum (skin oil), semen, sputum, synovial fluid, sweat, tears, urine, vaginal secretion, vomit and mixtures of one or more thereof. Biological samples include cell cultures, bodily fluids, cell cultures from bodily fluids. Bodily fluids may be obtained from a mammal organism, for example by puncture, or other collecting or sampling procedures.

The terms "subject," "individual," and "patient" are used interchangeably herein to refer to a vertebrate, preferably a mammal, more preferably a human. Mammals include, but are not limited to, murines, simians, humans, farm animals, sport animals, and pets. Tissues, cells and their progeny of a biological entity obtained in vivo or cultured in vitro are also encompassed.

Various embodiments are described hereinafter.

Reference is made to Bondeson, et al., "Phosphate dysregulation via the XPR1:KIDINS220 protein complex is a therapeutic vulnerability in ovarian cancer" bioRxiv 2020.10.16.339374; doi.org/10.1101/2020.10.16.339374 (posted to bioRxiv on October 17, 2020).

### OVERVIEW

Exploiting cancer-specific metabolic states is an attractive strategy to kill cancer cells while sparing normal tissues¹. WO2017/060304 discloses a pharmaceutical composition for use for the treatment of pancreatic cancer comprising at least one RBD ligand. Inorganic phosphate is a fundamental component of DNA, an intermediate metabolite in numerous pathways, and a key signaling molecule, yet perturbing phosphate homeostasis has not been explored as a cancer therapeutic. By analyzing CRISPR/Cas9 loss of viability screens across many cancer cell lines, Applicants found that high expression of SLC34A2, a phosphate importer, renders cells sensitive to inactivation of the phosphate exporter XPR1. This surprising finding indicates that accumulation of inorganic phosphate may be toxic to cancer cells. Applicants extensively validated this synthetic lethal interaction in cancer cell lines and mouse xenografts, and found evidence that many primary ovarian cancer tumors would be sensitive to phosphate dysregulation. Mechanistically, Applicants also identified a transcriptional response aimed at restoring phosphate homeostasis. Applicants also identified an XPR1-binding partner (KIDINS220) required for phosphate efflux. Applicants also identified novel vacuole structures which Applicants hypothesize are associated with toxic accumulation of intracellular phosphate. Applicants also show that a viral XPR1 binding protein, receptor-binding domain (RBD), inhibits XPR1-dependent phosphate efflux and is a drug-like inhibitor of XPR1. Overall, these data illustrate novel mechanisms by which ovarian cancer cells maintain phosphate homeostasis, and that the perturbation of these mechanisms is an unappreciated therapeutic vulnerability in ovarian cancer.

Embodiments disclosed herein provide methods of treating and diagnosing cancers based on identification of the dependency on XPR1:KIDINS220-mediated phosphate export in tumor cells (e.g., tumors that overexpress SLC34A2). In an example embodiment, receptor-binding domain (RBD) inhibits XPR1-dependent phosphate efflux and is a drug-like inhibitor of XPR1 that can be used to treat sensitive tumors. Sensitive tumors express increased SLC34A2, a phosphate importer, and do not sufficiently suppress phosphate import in response to XPR1/phosphate efflux inhibition to prevent cell growth inhibition. Applicants further identified genes that co-vary (e.g., covariation) with SLC34A2 that can also be used alone or in combination to detect cancers that are vulnerable to inhibition of XPR1:KIDINS220-mediated phosphate export (e.g., PAX8). As used herein, the term "co-vary" refers to genes that are upregulated and downregulated together. As used herein "correlation" between genes refers to genes that co-vary. Applicants identified genes that are differentially expressed in response to XPR1 inhibition. For example, SLC34A2 and SLC20A1 are suppressed and FGF23 is upregulated. Applicants determined that the tumor cells alter the expression in order to restore phosphate homeostasis. Thus, targeting these mechanisms can further increase the vulnerability of sensitive tumors.

As used herein, XPR1 refers to xenotropic and polytropic retrovirus receptor 1 (Also known as: IBGC6, SLC53A1, SYG1, X3). Exemplary sequences include the following NCBI accession numbers: NM_004736.4, NM_001135669.2, NM_001328662.2, NP_004727.2, NP_001129141.1 and NP_001315591.1. XPR1 includes a SPX domain (N-terminal) and EXS domain (C-terminal) that can be targeted, in addition to targeting the entire protein, by a therapeutic agent (e.g., small molecules, antibodies) or agent for detecting expression (e.g., antibodies). XPR1 is a phosphate exporter in metazoans, a function that does not require the SPX domain (see, e.g., Giovannini, et al., Inorganic phosphate export by the retrovirus receptor XPR1 in metazoans. Cell Rep. 2013;3(6):1866-1873; Legati, et al. Mutations in XPR1 cause primary familial brain calcification associated with altered phosphate export. Nat Genet. 2015;47(6):579-581; Ansermet, et al., Renal Fanconi Syndrome and Hypophosphatemic Rickets in the Absence of Xenotropic and Polytropic Retroviral Receptor in the Nephron . J Am Soc Nephrol. 2017;28(4):1073-1078).

As used herein, KIDINS220 refers to kinase D interacting substrate 220 (Also known as: ARMS, SINO). Exemplary sequences include the following NCBI accession numbers: NM_020738.4, NM_001348729.2, NM_001348731.2, NM_001348732.2, NM_001348734.2, NM_001348735.2, NM_001348736.2, NM_001348738.2, NM_001348739.2, NM_001348740.2, NM_001348741.2, NM_001348742.2, NM_001348743.2, NM_001348745.2, NP_065789.1, NP_001335658.1, NP_001335660.1, NP_001335661.1, NP_001335663.1, NP_001335664.1, NP_001335665.1, NP_001335667.1, NP_001335668.1, NP_001335669.1, NP_001335670.1, NP_001335671.1, NP_001335672.1, and NP_001335674.1. KIDINS220 includes an Ankyrin repeat-containing domain (N-terminal) and KAP family P-loop domain that can be targeted by a therapeutic agent (e.g., small molecules, antibodies) or agent for detecting expression (e.g., antibodies). The P-loop domain is characterized by two conserved motifs, termed the Walker A and B motifs. The Walker A motif, also known as the Walker loop, or P-loop, or phosphate-binding loop, is a motif in proteins that is associated with phosphate binding. The Walker B motif is a motif in most P-loop proteins situated well downstream of the A-motif.

As used herein, SLC34A2 refers to solute carrier family 34 member 2 (Also known as: NAPI-3B, NAPI-IIb, NPTIIb, PULAM). Exemplary sequences include the following NCBI accession numbers: NM_006424.3, NM_001177998.2, NM_001177999.1, NP_006415.3, NP_001171469.2, and NP_001171470.1. Synthetic peptides derived from a complementarity determining region hypervariable domain amino acid sequence of a humanized monoclonal antibody to NaPi2B transporter has been described for inhibiting tumor growth or treating cancer (US 9,193,797 B2).

As used herein, SLC20A1 refers to solute carrier family 20 member 1 (Also known as: GLVR1, Glvr-1, PIT1, PiT-1). Exemplary sequences include the following NCBI accession numbers: NM_005415.5 and NP_005406.3.

As used herein, PAX8 refers to paired box 8. Exemplary sequences include the following NCBI accession numbers: NM_003466.4, NM_013952.4, NM_013953.4, NM_013992.4, NP_003457.1, NP_039246.1, NP_039247.1, and NP_054698.1.

As used herein, FGF23 refers to fibroblast growth factor 23 (Also known as: ADHR, FGFN, HFTC2, HPDR2, HYPF, PHPTC). Exemplary sequences include the following NCBI accession numbers: NM_020638.3 and NP_065689.1.

The present invention may be useful for the treatment of any cancer dependent on XPR1:KIDINS220-mediated phosphate export. In other preferred embodiments, the cancer has increased expression of SLC34A2 as compared to normal tissue (e.g., ovarian cancer, uterine cancer, breast cancer, bile duct cancer, liver and lung cancer). In more preferred embodiments, the cancer is ovarian or uterine cancer. Detection of SLC34A2 expression is further described in the diagnostic methods section herein.

Exemplary cancers that may benefit from treatment with one or more inhibitors of XPR1:KIDINS220-mediated phosphate export include, without limitation, liquid tumors such as leukemia (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), polycythemia vera, lymphoma (e.g., Hodgkin's disease, non-Hodgkin's disease), Waldenstrom's macroglobulinemia, heavy chain disease, or multiple myeloma. The cancer may include, without limitation, solid tumors such as sarcomas and carcinomas. Examples of solid tumors include, but are not limited to fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, epithelial carcinoma, bronchogenic carcinoma, hepatoma, colorectal cancer (e.g., colon cancer, rectal cancer), anal cancer, pancreatic cancer (e.g., pancreatic adenocarcinoma, islet cell carcinoma, neuroendocrine tumors), breast cancer (e.g., ductal carcinoma, lobular carcinoma, inflammatory breast cancer, clear cell carcinoma, mucinous carcinoma), ovarian carcinoma (e.g., ovarian epithelial carcinoma or surface epithelial-stromal tumour including serous tumour, endometrioid tumor and mucinous cystadenocarcinoma, sex-cord-stromal tumor), prostate cancer, liver and bile duct carcinoma (e.g., hepatocelluar carcinoma, cholangiocarcinoma, hemangioma), choriocarcinoma, seminoma, embryonal carcinoma, kidney cancer (e.g., renal cell carcinoma, clear cell carcinoma, Wilm's tumor, nephroblastoma), cervical cancer, uterine cancer (e.g., endometrial adenocarcinoma, uterine papillary serous carcinoma, uterine clear-cell carcinoma, uterine sarcomas and leiomyosarcomas, mixed mullerian tumors), testicular cancer, germ cell tumor, lung cancer (e.g., lung adenocarcinoma, squamous cell carcinoma, large cell carcinoma, bronchioloalveolar carcinoma, non-small-cell carcinoma, small cell carcinoma, mesothelioma), bladder carcinoma, signet ring cell carcinoma, cancer of the head and neck (e.g., squamous cell carcinomas), esophageal carcinoma (e.g., esophageal adenocarcinoma), tumors of the brain (e.g., glioma, glioblastoma, astrocytoma, medulloblastoma, craniopharyngioma, ependymoma, pinealoma, hemangioblastoma, acoustic neuroma, oligodenroglioma, schwannoma, meningioma), neuroblastoma, retinoblastoma, neuroendocrine tumor, melanoma, cancer of the stomach (e.g., stomach adenocarcinoma, gastrointestinal stromal tumor), or carcinoids. Lymphoproliferative disorders are also considered to be proliferative diseases. In certain example embodiments, the cancer is ovarian cancer. In certain other example embodiments, the cancer is uterine cancer.

In another aspect, embodiments disclosed herein are directed to a method of treating cancer based on determining if the subject has a tumor sensitive to phosphate dysregulation and administering one or more therapeutic agents that target XPR1:KIDINS2200-mediated phosphate export if the subject has a tumor sensitive to phosphate dysregulation. In another aspect, embodiments disclosed herein provide methods of determining whether a subject has a tumor sensitive to phosphate dysregulation by detecting expression of SLC34A2 in a tumor sample or detecting amplification in XPR1 copy number in a tumor sample.

### THERAPEUTIC METHODS OF TARGETING CANCER DEPENDENCIES

### Therapeutic Agents

In certain embodiments, the present invention provides for one or more therapeutic agents targeting XPR1:KIDINS220-mediated phosphate export. In preferred embodiments, the therapeutic agents are inhibitors of XPR1:KIDINS220-mediated phosphate export. In certain embodiments, the therapeutic agent blocks or disrupts the XPR1:KIDINS220 protein complex from functioning to export inorganic phosphate from a tumor cell. In certain embodiments, the therapeutic agent blocks the XPR1:KIDINS220 protein complex from forming. In certain embodiments, the therapeutic agent targets an extracellular domain of XPR1. In certain embodiments, the therapeutic agent targets the SPX domain or EXS domain of XPR1. In certain embodiments, the therapeutic agent targets the Ankyrin repeat-containing domain or KAP family P-loop domain (including the Walker A/B motifs) of KIDIN220.

In certain embodiments, targeting XPR1:KIDINS220-mediated phosphate export may be made in combination with the current standard of care and may provide for improved treatment and/or less toxicity. In certain embodiments, the one or more therapeutic agents comprise therapeutic polypeptides, a small molecule inhibitor, small molecule degrader (e.g., ATTEC, AUTAC, LYTAC, or PROTAC), antibody, antibody fragment, antibody-like protein scaffold, aptamer, genetic modifying agents (e.g., CRISPR-Cas systems, TALENs, Zinc Finger Nucleases, Meganucleases), RNAi or any combination thereof. In one example embodiment, the one or more therapeutic agents comprise one or more therapeutic polypeptides. In another example embodiment, the therapeutic polypeptides are envelop receptor-binding domains (RBD). In another example embodiment, the one or more therapeutic agents comprises one or more antibodies specific to XPR1, specific to KIDINS220, or specific to the XPR1/KIDINS2200 complex.

The terms "therapeutic agent", "therapeutic capable agent" or "treatment agent" are used interchangeably and refer to a molecule or compound, or combination of molecules or compounds, that confers some beneficial effect upon administration to a subject. The beneficial effect includes enablement of diagnostic determinations; amelioration of a disease, symptom, disorder, or pathological condition; reducing or preventing the onset of a disease, symptom, disorder or condition; and generally counteracting a disease, symptom, disorder or pathological condition.

As used herein, "treatment" or "treating," or "palliating" or "ameliorating" are used interchangeably. These terms refer to an approach for obtaining beneficial or desired results including but not limited to a therapeutic benefit and/or a prophylactic benefit. By therapeutic benefit is meant any therapeutically relevant improvement in or effect on one or more diseases, conditions, or symptoms under treatment. For prophylactic benefit, the compositions may be administered to a subject at risk of developing a particular disease, condition, or symptom, or to a subject reporting one or more of the physiological symptoms of a disease, even though the disease, condition, or symptom may not have yet been manifested. As used herein "treating" includes ameliorating, curing, preventing it from becoming worse, slowing the rate of progression, or preventing the disorder from re-occurring (i.e., to prevent a relapse).

The term "effective amount" or "therapeutically effective amount" refers to the amount of an agent that is sufficient to effect beneficial or desired results. The therapeutically effective amount may vary depending upon one or more of: the subject and disease condition being treated, the weight and age of the subject, the severity of the disease condition, the manner of administration and the like, which can readily be determined by one of ordinary skill in the art. The term also applies to a dose that will provide an image for detection by any one of the imaging methods described herein. The specific dose may vary depending on one or more of: the particular agent chosen, the dosing regimen to be followed, whether it is administered in combination with other compounds, timing of administration, the tissue to be imaged, and the physical delivery system in which it is carried.

For example, in methods for treating cancer in a subject, an effective amount of a combination of inhibitors targeting epigenetic genes is any amount that provides an anti-cancer effect, such as reduces or prevents proliferation of a cancer cell or is cytotoxic towards a cancer cell. In certain embodiments, the effective amount of an inhibitor targeting an epigenetic gene is reduced when an inhibitor is administered concomitantly or in combination with one or more additional inhibitors targeting epigenetic genes as compared to the effective amount of the inhibitor when administered in the absence of one or more additional inhibitors targeting epigenetic genes. In certain embodiments, the inhibitor targeting an epigenetic gene does not reduce or prevent proliferation of a cancer cell when administered in the absence of one or more additional inhibitors targeting epigenetic genes.

### Therapeutic Polypeptides

In one example embodiment, a method of targeting cancer phosphate dependency comprises administering a therapeutic polypeptide. In certain embodiments, a polypeptide therapeutic is used to inhibit the XPR1:KIDINS220-mediated phosphate export. The therapeutic polypeptide may inhibit XPR1:KIDINS220-mediated phosphate export by binding to XPR1, KIDINS220, or the XPR1/KIDINS200 complex. In one example embodiment, the therapeutic polypeptide is an envelope-receptor-binding domain ("RBD"). In another example embodiment, the therapeutic polypeptide is an antibody or fragment or variant thereof.

### Envelope-Receptor-Binding Domain (RBD)

In certain embodiments, the protein therapeutic is a receptor binding protein "RBD" protein that can bind to and inhibit the XPR1:KIDINS220 protein complex. In certain example embodiments, the RBD protein is derived from an enveloped virus glycoprotein and capable of interacting with the XPR1 membrane receptor. XPR1 is the xenotropic and polytropic receptor for a variety of murine leukemia viruses (MLV or MuLV). In certain embodiments, "RBD" is an about 238 residue fragment of the envelope glycoprotein for a retrovirus, such as X-MLV, and fragment inhibits XPR1 phosphate efflux (see, e.g., Giovannini, et al., Inorganic phosphate export by the retrovirus receptor XPR1 in metazoans. Cell Rep. 2013;3(6):1866-1873). The RBD proteins can be modified as described herein. In certain embodiments, the RBD can be a fusion protein. For example, the RBD can be an Fc fusion protein to increase stability *in vivo.* In certain embodiments, the Fc domain promotes dimerization of the RBD fusion protein. In certain embodiments, the RBD protein can be a fusion protein linked to glutathione S-transferase (GST), and/or serum albumin (e.g., HSA or MSA). In certain embodiments, the GST domain promotes dimerization of the RBD fusion protein (see, e.g., Tudyka and Skerra, Glutathione S-transferase Can Be Used as a C-terminal, Enzymatically Active Dimerization Module for a Recombinant Protease Inhibitor, and Functionally Secreted Into the Periplasm of Escherichia Coli. Protein Science (1997), 6:2180-2187). Structural analyses have revealed that glutathione S-transferase (GST) can form homodimers (Ji et al., Biochemistry. 1992 Oct 27; 31(42):10169-84; Reinemer et al., J Mol Biol. 1992 Sep 5; 227(1):214-26; and Kaplan et al., Protein Sci. 1997 Feb; 6(2):399-406).

**FIG. 15** shows an exemplary RBD Fc fusion protein; MLVMEGSAFSKPLKDKINPWGPLIVMGILVRAGASVQRDSPHQIFNVTWRVTNLMTGQTANA TSLLGTMTDTFPKLYFDLCDLVGDYWDDPEPDIGDGCRTPGGRRRTRLYDFYVCPGHTVPIG CGGPGEGYCGKWGCETTGQAYWKPSSSWDLISLKRGNTPKDQGPCYDSSVSSGVQGATPGGR CNPLVLEFTDAGRKASWDAPKVWGLRLYRSTGADPVTRFSLTRQVLNVGPRVPIGSVDVPRD CGCKPCICTVPEVSSVFIFPPKPKDVLTITLTPKVTCVVVDISKDDPEVQFSWFVDDVEVHT AQTQPREEQFNSTFRSVSELPIMHQDWLNGKEFKCRVNSAAFPAPIEKTISKTKGRPKAPQV YTIPPPKEQMAKDKVSLTCMITDFFPEDITVEWQWNGQPAENYKNTQPIMDTDGSYFVYSKL NVQKSNWEAGNTFTCSVLHEGLHNHHTEKSLSHSPGK **(SEQ ID NO:** 2). In certain embodiments, the mFc tag promotes dimerization of RBD. In certain embodiments, the mFc tags promote recycling and stability of the RBD fusion protein. Giovannini, et al., 2013 discloses KoRV, A-MLV, X-MLV (NZB-IU-6 strain), and PERV-A RBD C-terminally fused to a mouse immunoglobulin (Ig) G1 Fc fragment and produced in HEK293T cells. Multiple MULVs use XPR1 with different species cross-reactivity: X-MLVs only infect XPR1^{hum}; P-MLVs use both XPR1^{mus} and XPR1^{hum}. The RBD protein can be derived from xenotropic or polytropic murine leukemia retrovirus (X- and P-MLV) Env. FIG. 16 shows RBD proteins from other viral strains that can infect through murine XPR1 and that are applicable to the present invention. As used herein, an RBD protein that is derived may be any non-natural hybrid of an RBD protein from an enveloped virus glycoprotein (e.g., non-natural hybrids X- and P-MLVs). As used herein "hybrid protein" refers to any protein that contains segments or parts from any two or more different proteins (e.g., non-natural hybrids containing parts from both X- and P-ML Vs), including a non-naturally occurring modified protein.

### Gene Therapy Embodiments For RBD

In one example embodiment, gene therapy may be used to express RBD in tumor cells or the tumor microenvironment. In certain embodiments, gene therapy is used for subjects having tumors that overexpress SLC34A2. As used herein, the terms "gene therapy," "gene delivery," "gene transfer" and "genetic modification" are used interchangeably and refer to modifying or manipulating the expression of a gene to alter the biological properties of living cells for therapeutic use.

In one example embodiment, a vector for use in gene therapy comprises a sequence encoding an RBD protein and is used to deliver said sequence to tumor cells. The vector may further comprise one or more regulatory elements to control expression of the gene. The vector may further comprise regulatory/control elements, e.g., promoters, enhancers, introns, polyadenylation signals, Kozak consensus sequences, or internal ribosome entry sites (IRES). The vector may further comprise a targeting moiety that directs the vector specifically to tumor cells or the tumor microenvironment. In another example embodiment, the vector may comprise a viral vector with a tropism specific for tumors.

In general, and throughout this specification, the term "vector" refers to a nucleic acid molecule capable of transporting another nucleic acid to which it has been linked. Vectors include, but are not limited to, nucleic acid molecules that are single-stranded, double-stranded, or partially double-stranded; nucleic acid molecules that comprise one or more free ends, no free ends (e.g., circular); nucleic acid molecules that comprise DNA, RNA, or both; and other varieties of polynucleotides known in the art. There are no limitations regarding the type of vector that can be used. The vector can be a cloning vector, suitable for propagation and for obtaining polynucleotides, gene constructs or expression vectors incorporated to several heterologous organisms. Suitable vectors include eukaryotic expression vectors based on viral vectors (e.g. adenoviruses, adeno- associated viruses as well as retroviruses and lentiviruses), as well as non-viral vectors such as plasmids.

In one example embodiment, the vector is a viral vector, wherein virally-derived DNA or RNA sequences are present in the vector for packaging into a virus (e.g., retroviruses, replication defective retroviruses, adenoviruses, replication defective adenoviruses, and adeno-associated viruses). Viral vectors also include polynucleotides carried by a virus for transfection into a host cell. Certain vectors are capable of autonomous replication in a host cell into which they are introduced (e.g., episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operably linked. Such vectors are referred to herein as "expression vectors." Vectors for and that result in expression in a eukaryotic cell can be referred to herein as "eukaryotic expression vectors." In another example embodiment, the vector integrates the gene into the cell genome or is maintained episomally.

In one example embodiment, the vector is a "plasmid," which refers to a circular double stranded DNA loop into which additional DNA segments can be inserted, such as by standard molecular cloning techniques.

In one example embodiment, the vector is an mRNA vector (see, e.g., Sahin, U, Kariko, K and Tureci, O (2014). mRNA-based therapeutics - developing a new class of drugs. Nat Rev Drug Discov 13: 759-780; Weissman D, Karikó K. mRNA: Fulfilling the Promise of Gene Therapy. Mol Ther. 2015;23(9):1416-1417. doi:10.1038/mt.2015.138; Kowalski PS, Rudra A, Miao L, Anderson DG. Delivering the Messenger: Advances in Technologies for Therapeutic mRNA Delivery. Mol Ther. 2019;27(4):710-728. doi:10.1016/j.ymthe.2019.02.012; Magadum A, Kaur K, Zangi L. mRNA-Based Protein Replacement Therapy for the Heart. Mol Ther. 2019;27(4):785-793. doi:10.1016/j.ymthe.2018.11.018; Reichmuth AM, Oberli MA, Jaklenec A, Langer R, Blankschtein D. mRNA vaccine delivery using lipid nanoparticles Ther Deliv. 2016;7(5):319-334. doi:10.4155/tde-2016-0006; and Khalil AS, Yu X, Umhoefer JM, et al. Single-dose mRNA therapy via biomaterial-mediated sequestration of overexpressed proteins. Sci Adv. 2020;6(27):eaba2422). In an exemplary embodiment, mRNA encoding for an RBD protein is delivered using lipid nanoparticles (see, e.g., Reichmuth, et al., 2016) and administered directly to a tumor. In an exemplary embodiment, mRNA encoding for an RBD protein is delivered using biomaterial-mediated sequestration (see, e.g., Khalil, et al., 2020) and administered directly to tumor tissue. Sequences present in mRNA molecules, as described further herein, are applicable to mRNA vectors (e.g., Kozak consensus sequence, miRNA target sites and WPRE).

### Regulatory Elements

Recombinant expression vectors can comprise a nucleic acid of the invention in a form suitable for expression of the nucleic acid in a host cell, which means that the recombinant expression vectors include one or more regulatory elements, which may be selected on the basis of the host cells to be used for expression, that is operably-linked to the nucleic acid sequence to be expressed. Within a recombinant expression vector, "operably linked" is intended to mean that the nucleotide sequence of interest is linked to the regulatory element(s) in a manner that allows for expression of the nucleotide sequence (e.g., in an in vitro transcription/translation system or in a host cell when the vector is introduced into the host cell). The term "operably linked" as used herein also refers to the functional relationship and position of a promoter sequence relative to a polynucleotide of interest (e.g., a promoter or enhancer is operably linked to a coding sequence if it affects the transcription of that sequence). Typically, an operably linked promoter is contiguous with the sequence of interest. However, enhancers need not be contiguous with the sequence of interest to control its expression. The term "promoter", as used herein, refers to a nucleic acid fragment that functions to control the transcription of one or more polynucleotides, located upstream of the polynucleotide sequence(s), and which is structurally identified by the presence of a binding site for DNA-dependent RNA polymerase, transcription initiation sites, and any other DNA sequences including, but not limited to, transcription factor binding sites, repressor, and activator protein binding sites, and any other sequences of nucleotides known in the art to act directly or indirectly to regulate the amount of transcription from the promoter. A "tissue-specific" promoter is only active in specific types of differentiated cells or tissues.

In another embodiment, the vector of the invention further comprises expression control sequences including, but not limited to, appropriate transcription sequences (i.e., initiation, termination, promoter, and enhancer), efficient RNA processing signals (e.g., splicing and polyadenylation (polyA) signals), sequences that stabilize cytoplasmic mRNA, sequences that enhance translation efficiency (i.e. Kozak consensus sequence), and sequences that enhance protein stability. A great number of expression control sequences, including promoters which are native, constitutive, inducible, or tissue-specific are known in the art and may be utilized according to the present invention.

In another embodiment, the vector of the invention further comprises a post-transcriptional regulatory region. In a preferred embodiment, the post-transcriptional regulatory region is the Woodchuck Hepatitis Virus post-transcriptional region (WPRE) or functional variants and fragments thereof and the PPT-CTS or functional variants and fragments thereof (see, e.g., Zufferey R, et al., J. Virol. 1999; 73:2886-2892; and Kappes J, et al., WO 2001/044481). In a particular embodiment, the post-transcriptional regulatory region is WPRE. The term "Woodchuck hepatitis virus posttranscriptional regulatory element" or "WPRE", as used herein, refers to a DNA sequence that, when transcribed, creates a tertiary structure capable of enhancing the expression of a gene (see, e.g., Lee Y, et ah, Exp. Physiol. 2005; 90(l):33-37 and Donello J, et al, J. Virol. 1998; 72(6):5085-5092).

The term "regulatory element" is intended to include promoters, enhancers, internal ribosomal entry sites (IRES), and other expression control elements (e.g., transcription termination signals, such as polyadenylation signals and poly-U sequences). Such regulatory elements are described, for example, in Goeddel, GENE EXPRESSION TECHNOLOGY: METHODS IN ENZYMOLOGY 185, Academic Press, San Diego, Calif. (1990).

Regulatory elements include those that direct constitutive expression of a nucleotide sequence in many types of host cell and those that direct expression of the nucleotide sequence only in certain host cells (e.g., tissue-specific regulatory sequences). A tissue-specific promoter may direct expression primarily in a desired tissue of interest. Regulatory elements may also direct expression in a temporal-dependent manner, such as in a cell-cycle dependent or developmental stage-dependent manner, which may or may not also be tissue or cell-type specific. In some embodiments, a vector comprises one or more pol III promoter (e.g., 1, 2, 3, 4, 5, or more pol III promoters), one or more pol II promoters (e.g., 1, 2, 3, 4, 5, or more pol II promoters), one or more pol I promoters (e.g., 1, 2, 3, 4, 5, or more pol I promoters), or combinations thereof. Also encompassed by the term "regulatory element" are enhancer elements. It will be appreciated by those skilled in the art that the design of the expression vector can depend on such factors as the choice of the host cell to be transformed, the level of expression desired, etc. A vector can be introduced into host cells to thereby produce transcripts, proteins, or peptides, including fusion proteins or peptides, encoded by nucleic acids as described herein (e.g., RBD).

In one embodiment, the vector contains at least one target sequence of at least one miRNA expressed in non-tumor tissue. The term "microRNAs" or "miRNAs", as used herein, are small (~22-nt), evolutionarily conserved, regulatory RNAs involved in RNA-mediated gene silencing at the post-transcriptional level (see, e.g., Barrel DP. Cell 2004; 116: 281-297). Through base pairing with complementary regions (most often in the 3' untranslated region (3'UTR) of cellular messenger RNA (mRNA)), miRNAs can act to suppress mRNA translation or, upon high-sequence homology, cause the catalytic degradation of mRNA. Because of the highly differential tissue expression of many miRNAs, cellular miRNAs can be exploited to mediate tissue-specific targeting of gene therapy vectors. By engineering tandem copies of target elements perfectly complementary to tissue-specific miRNAs (miRT).

### Antibodies

In certain embodiments, the one or more agents is an antibody. In certain embodiments, the antibody blocks or disrupts the XPR1:KIDINS220 protein complex from functioning to export inorganic phosphate from a tumor cell. In example embodiments, the antibody is specific for XPR1, KIDINS220, or the XPR1/KIDINS220 protein complex. In certain embodiments, the antibody blocks the XPR1:KIDINS220 protein complex from forming. In certain embodiments, the antibody targets XPR1 (see, e.g., WO2020153467A1). In certain embodiments, the antibody targets an extracellular domain of XPR1. In certain embodiments, the antibody targets the SPX domain or EXS domain of XPR1. In certain embodiments, XPR1 antibodies target the region spanning amino acid 529 to the end of the protein, which is commonly available from different vendors. In certain embodiments, the antibody targets a Walker A/B motif of KIDINS220. In certain embodiments, the antibody targets a Walker A/B motif or KAP family P-loop domain of KIDINS220 to block phosphate binding.

Antibodies recognizing XPR1 or KIDINS220 have been generated and are commercially available (see, e.g., ThermoFisher website: Cat #21856-1-AP, Cat #PA5-116475, Cat #PA5-100152, Cat #PA5-97897, Cat #PA5-22116, Cat #MA5-32869, Cat #PA5-82511, Cat #PA5-111894, Cat #66748-1-IG, Cat #A303-002A, Cat #A303-003A, Cat #MA1-90667, Cat #PA1-4229, Cat #BS-7041R, Cat #A303-002A-M, Cat #RA19019, Cat #R A 19020; and Cat #PA5-82552, Cat #PA5-21908, Cat #PA5-34338, Cat #14174-1-AP, Cat #PA5-34337, Cat #PA1-23547). One skilled in the art could easily generate a therapeutic antibody to block XPR1 or KIDINS220 to inhibit phosphate efflux (see, e.g., Lu, RM., Hwang, YC., Liu, IJ. et al. Development of therapeutic antibodies for the treatment of diseases. J Biomed Sci 27, 1 (2020)).

The term "antibody" is used interchangeably with the term "immunoglobulin" herein, and includes intact antibodies, fragments of antibodies, e.g., Fab, F(ab')2 fragments, and intact antibodies and fragments that have been mutated either in their constant and/or variable region (e.g., mutations to produce chimeric, partially humanized, or fully humanized antibodies, as well as to produce antibodies with a desired trait, e.g., enhanced binding and/or reduced FcR binding). The term "fragment" refers to a part or portion of an antibody or antibody chain comprising fewer amino acid residues than an intact or complete antibody or antibody chain. Fragments can be obtained via chemical or enzymatic treatment of an intact or complete antibody or antibody chain. Fragments can also be obtained by recombinant means. Exemplary fragments include a nanobody, Fab, Fab', (Fab')2, Fv, ScFv, diabody, triabody, tetrabody, Bis-scFv, minibody, Fab2, or Fab3 fragment, Fabc, Fd, dAb, V_{HH} and scFv and/or Fv fragments.

As used herein, a preparation of antibody protein having less than about 50% of non-antibody protein (also referred to herein as a "contaminating protein"), or of chemical precursors, is considered to be "substantially free." 40%, 30%, 20%, 10% and more preferably 5% (by dry weight), of non-antibody protein, or of chemical precursors is considered to be substantially free. When the antibody protein or biologically active portion thereof is recombinantly produced, it is also preferably substantially free of culture medium, i.e., culture medium represents less than about 30%, preferably less than about 20%, more preferably less than about 10%, and most preferably less than about 5% of the volume or mass of the protein preparation.

The term "antigen-binding fragment" refers to a polypeptide fragment of an immunoglobulin or antibody that binds antigen or competes with intact antibody (i.e., with the intact antibody from which they were derived) for antigen binding (i.e., specific binding). As such these antibodies or fragments thereof are included in the scope of the invention, provided that the antibody or fragment binds specifically to a target molecule.

It is intended that the term "antibody" encompass any Ig class or any Ig subclass (e.g. the IgG1, IgG2, IgG3, and IgG4 subclassess of IgG) obtained from any source (e.g., humans and non-human primates, and in rodents, lagomorphs, caprines, bovines, equines, ovines, etc.).

The term "Ig class" or "immunoglobulin class", as used herein, refers to the five classes of immunoglobulin that have been identified in humans and higher mammals, IgG, IgM, IgA, IgD, and IgE. The term "Ig subclass" refers to the two subclasses of IgM (H and L), three subclasses of IgA (IgA1, IgA2, and secretory IgA), and four subclasses of IgG (IgG1, IgG2, IgG3, and IgG4) that have been identified in humans and higher mammals. The antibodies can exist in monomeric or polymeric form; for example, IgM antibodies exist in pentameric form, and IgA antibodies exist in monomeric, dimeric or multimeric form.

The term "IgG subclass" refers to the four subclasses of immunoglobulin class IgG - IgG1, IgG2, IgG3, and IgG4 that have been identified in humans and higher mammals by the heavy chains of the immunoglobulins, V1 - γ4, respectively. The term "single-chain immunoglobulin" or "single-chain antibody" (used interchangeably herein) refers to a protein having a two-polypeptide chain structure consisting of a heavy and a light chain, said chains being stabilized, for example, by interchain peptide linkers, which has the ability to specifically bind antigen. The term "domain" refers to a globular region of a heavy or light chain polypeptide comprising peptide loops (e.g., comprising 3 to 4 peptide loops) stabilized, for example, by β pleated sheet and/or intrachain disulfide bond. Domains are further referred to herein as "constant" or "variable", based on the relative lack of sequence variation within the domains of various class members in the case of a "constant" domain, or the significant variation within the domains of various class members in the case of a "variable" domain. Antibody or polypeptide "domains" are often referred to interchangeably in the art as antibody or polypeptide "regions". The "constant" domains of an antibody light chain are referred to interchangeably as "light chain constant regions", "light chain constant domains", "CL" regions or "CL" domains. The "constant" domains of an antibody heavy chain are referred to interchangeably as "heavy chain constant regions", "heavy chain constant domains", "CH" regions or "CH" domains). The "variable" domains of an antibody light chain are referred to interchangeably as "light chain variable regions", "light chain variable domains", "VL" regions or "VL" domains). The "variable" domains of an antibody heavy chain are referred to interchangeably as "heavy chain constant regions", "heavy chain constant domains", "VH" regions or "VH" domains).

The term "region" can also refer to a part or portion of an antibody chain or antibody chain domain (e.g., a part or portion of a heavy or light chain or a part or portion of a constant or variable domain, as defined herein), as well as more discrete parts or portions of said chains or domains. For example, light and heavy chains or light and heavy chain variable domains include "complementarity determining regions" or "CDRs" interspersed among "framework regions" or "FRs", as defined herein.

The term "conformation" refers to the tertiary structure of a protein or polypeptide (e.g., an antibody, antibody chain, domain or region thereof). For example, the phrase "light (or heavy) chain conformation" refers to the tertiary structure of a light (or heavy) chain variable region, and the phrase "antibody conformation" or "antibody fragment conformation" refers to the tertiary structure of an antibody or fragment thereof.

The term "antibody-like protein scaffolds" or "engineered protein scaffolds" broadly encompasses proteinaceous non-immunoglobulin specific-binding agents, typically obtained by combinatorial engineering (such as site-directed random mutagenesis in combination with phage display or other molecular selection techniques). Usually, such scaffolds are derived from robust and small soluble monomeric proteins (such as Kunitz inhibitors or lipocalins) or from a stably folded extra-membrane domain of a cell surface receptor (such as protein A, fibronectin or the ankyrin repeat).

Such scaffolds have been extensively reviewed in Binz et al. (Engineering novel binding proteins from nonimmunoglobulin domains. Nat Biotechnol 2005, 23:1257-1268), Gebauer and Skerra (Engineered protein scaffolds as next-generation antibody therapeutics. Curr Opin Chem Biol. 2009, 13:245-55), Gill and Damle (Biopharmaceutical drug discovery using novel protein scaffolds. Curr Opin Biotechnol 2006, 17:653-658), Skerra (Engineered protein scaffolds for molecular recognition. J Mol Recognit 2000, 13:167-187), and Skerra (Alternative non-antibody scaffolds for molecular recognition. Curr Opin Biotechnol 2007, 18:295-304), and include without limitation affibodies, based on the Z-domain of staphylococcal protein A, a three-helix bundle of 58 residues providing an interface on two of its alpha-helices (Nygren, Alternative binding proteins: Affibody binding proteins developed from a small three-helix bundle scaffold. FEBS J 2008, 275:2668-2676); engineered Kunitz domains based on a small (ca. 58 residues) and robust, disulphide-crosslinked serine protease inhibitor, typically of human origin (e.g., LACI-D1), which can be engineered for different protease specificities (Nixon and Wood, Engineered protein inhibitors of proteases. Curr Opin Drug Discov Dev 2006, 9:261-268); monobodies or adnectins based on the 10th extracellular domain of human fibronectin III (10Fn3), which adopts an Ig-like beta-sandwich fold (94 residues) with 2-3 exposed loops, but lacks the central disulphide bridge (Koide and Koide, Monobodies: antibody mimics based on the scaffold of the fibronectin type III domain. Methods Mol Biol 2007, 352:95-109); anticalins derived from the lipocalins, a diverse family of eight-stranded beta-barrel proteins (ca. 180 residues) that naturally form binding sites for small ligands by means of four structurally variable loops at the open end, which are abundant in humans, insects, and many other organisms (Skerra, Alternative binding proteins: Anticalins-harnessing the structural plasticity of the lipocalin ligand pocket to engineer novel binding activities. FEBS J 2008, 275:2677-2683); DARPins, designed ankyrin repeat domains (166 residues), which provide a rigid interface arising from typically three repeated beta-turns (Stumpp et al., DARPins: a new generation of protein therapeutics. Drug Discov Today 2008, 13:695-701); avimers (multimerized LDLR-A module) (Silverman et al., Multivalent avimer proteins evolved by exon shuffling of a family of human receptor domains. Nat Biotechnol 2005, 23:1556-1561); and cysteine-rich knottin peptides (Kolmar, Alternative binding proteins: biological activity and therapeutic potential of cystine-knot miniproteins. FEBS J 2008, 275:2684-2690).

"Specific binding" of an antibody means that the antibody exhibits appreciable affinity for a particular antigen or epitope and, generally, does not exhibit significant cross reactivity. "Appreciable" binding includes binding with an affinity of at least 25 µM. Antibodies with affinities greater than 1 x 10⁷ M⁻¹ (or a dissociation coefficient of 1µM or less or a dissociation coefficient of 1nm or less) typically bind with correspondingly greater specificity. Values intermediate of those set forth herein are also intended to be within the scope of the present invention and antibodies of the invention bind with a range of affinities, for example, 100nM or less, 75nM or less, 50nM or less, 25nM or less, for example 10nM or less, 5nM or less, 1nM or less, or in embodiments 500pM or less, 100pM or less, 50pM or less or 25pM or less. An antibody that "does not exhibit significant crossreactivity" is one that will not appreciably bind to an entity other than its target (e.g., a different epitope or a different molecule). For example, an antibody that specifically binds to a target molecule will appreciably bind the target molecule but will not significantly react with non-target molecules or peptides. An antibody specific for a particular epitope will, for example, not significantly crossreact with remote epitopes on the same protein or peptide. Specific binding can be determined according to any art-recognized means for determining such binding. Preferably, specific binding is determined according to Scatchard analysis and/or competitive binding assays.

As used herein, the term "affinity" refers to the strength of the binding of a single antigen-combining site with an antigenic determinant. Affinity depends on the closeness of stereochemical fit between antibody combining sites and antigen determinants, on the size of the area of contact between them, on the distribution of charged and hydrophobic groups, etc. Antibody affinity can be measured by equilibrium dialysis or by the kinetic BIACORE^{™} method. The dissociation constant, Kd, and the association constant, Ka, are quantitative measures of affinity.

As used herein, the term "monoclonal antibody" refers to an antibody derived from a clonal population of antibody-producing cells (e.g., B lymphocytes or B cells) which is homogeneous in structure and antigen specificity. The term "polyclonal antibody" refers to a plurality of antibodies originating from different clonal populations of antibody-producing cells which are heterogeneous in their structure and epitope specificity but which recognize a common antigen. Monoclonal and polyclonal antibodies may exist within bodily fluids, as crude preparations, or may be purified, as described herein.

The term "binding portion" of an antibody (or "antibody portion") includes one or more complete domains, e.g., a pair of complete domains, as well as fragments of an antibody that retain the ability to specifically bind to a target molecule. It has been shown that the binding function of an antibody can be performed by fragments of a full-length antibody. Binding fragments are produced by recombinant DNA techniques, or by enzymatic or chemical cleavage of intact immunoglobulins. Binding fragments include Fab, Fab', F(ab')2, Fabc, Fd, dAb, Fv, single chains, single-chain antibodies, e.g., scFv, and single domain antibodies.

"Humanized" forms of non-human (e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, FR residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all or at least one, and typically two, variable domains, in which all or substantially all of the hypervariable regions correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin.

Examples of portions of antibodies or epitope-binding proteins encompassed by the present definition include: (i) the Fab fragment, having V_{L}, C_{L}, V_{H} and C_{H}1 domains; (ii) the Fab' fragment, which is a Fab fragment having one or more cysteine residues at the C-terminus of the C_{H}1 domain; (iii) the Fd fragment having V_{H} and C_{H}1 domains; (iv) the Fd' fragment having V_{H} and C_{H}1 domains and one or more cysteine residues at the C-terminus of the CHI domain; (v) the Fv fragment having the V_{L} and V_{H} domains of a single arm of an antibody; (vi) the dAb fragment (Ward et al., 341 Nature 544 (1989)) which consists of a V_{H} domain or a V_{L} domain that binds antigen; (vii) isolated CDR regions or isolated CDR regions presented in a functional framework; (viii) F(ab')₂ fragments which are bivalent fragments including two Fab' fragments linked by a disulphide bridge at the hinge region; (ix) single chain antibody molecules (e.g., single chain Fv; scFv) (Bird et al., 242 Science 423 (1988); and Huston et al., 85 PNAS 5879 (1988)); (x) "diabodies" with two antigen binding sites, comprising a heavy chain variable domain (V_{H}) connected to a light chain variable domain (V_{L}) in the same polypeptide chain (see, e.g., EP 404,097; WO 93/11161; Hollinger et al., 90 PNAS 6444 (1993)); (xi) "linear antibodies" comprising a pair of tandem Fd segments (V_{H}-Cₕ1-V_{H}-Cₕ1) which, together with complementary light chain polypeptides, form a pair of antigen binding regions (Zapata et al., Protein Eng. 8(10):1057-62 (1995); and U.S. Patent No. 5,641,870).

As used herein, a "blocking" antibody or an antibody "antagonist" is one which inhibits or reduces biological activity of the antigen(s) it binds. In certain embodiments, the blocking antibodies or antagonist antibodies or portions thereof described herein completely inhibit the biological activity of the antigen(s).

Antibodies may act as agonists or antagonists of the recognized polypeptides. For example, the present invention includes antibodies which disrupt receptor/ligand interactions either partially or fully. The invention features both receptor-specific antibodies and ligand-specific antibodies. The invention also features receptor-specific antibodies which do not prevent ligand binding but prevent receptor activation. Receptor activation (i.e., signaling) may be determined by techniques described herein or otherwise known in the art. For example, receptor activation can be determined by detecting the phosphorylation (e.g., tyrosine or serine/threonine) of the receptor or of one of its down-stream substrates by immunoprecipitation followed by western blot analysis. In specific embodiments, antibodies are provided that inhibit ligand activity or receptor activity by at least 95%, at least 90%, at least 85%, at least 80%, at least 75%, at least 70%, at least 60%, or at least 50% of the activity in absence of the antibody.

The invention also features receptor-specific antibodies which both prevent ligand binding and receptor activation as well as antibodies that recognize the receptor-ligand complex. Likewise, encompassed by the invention are neutralizing antibodies which bind the ligand and prevent binding of the ligand to the receptor, as well as antibodies which bind the ligand, thereby preventing receptor activation, but do not prevent the ligand from binding the receptor. Further included in the invention are antibodies which activate the receptor. These antibodies may act as receptor agonists, i.e., potentiate or activate either all or a subset of the biological activities of the ligand-mediated receptor activation, for example, by inducing dimerization of the receptor. The antibodies may be specified as agonists, antagonists or inverse agonists for biological activities comprising the specific biological activities of the peptides disclosed herein. The antibody agonists and antagonists can be made using methods known in the art. See, e.g., PCT publication WO 96/40281; U.S. Pat. No. 5,811,097; Deng et al., Blood 92(6):1981-1988 (1998); Chen et al., Cancer Res. 58(16):3668-3678 (1998); Harrop et al., J. Immunol. 161(4):1786-1794 (1998); Zhu et al., Cancer Res. 58(15):3209-3214 (1998); Yoon et al., J. Immunol. 160(7):3170-3179 (1998); Prat et al., J. Cell. Sci. III (Pt2):237-247 (1998); Pitard et al., J. Immunol. Methods 205(2):177-190 (1997); Liautard et al., Cytokine 9(4):233-241 (1997); Carlson et al., J. Biol. Chem. 272(17):11295-11301 (1997); Taryman et al., Neuron 14(4):755-762 (1995); Muller et al., Structure 6(9):1153-1167 (1998); Bartunek et al., Cytokine 8(1):14-20 (1996).

The antibodies as defined for the present invention include derivatives that are modified, i.e., by the covalent attachment of any type of molecule to the antibody such that covalent attachment does not prevent the antibody from generating an anti-idiotypic response. For example, but not by way of limitation, the antibody derivatives include antibodies that have been modified, e.g., by glycosylation, acetylation, pegylation, phosphylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, metabolic synthesis of tunicamycin, etc. Additionally, the derivative may contain one or more non-classical amino acids.

Simple binding assays can be used to screen for or detect agents that bind to a target protein, or disrupt the interaction between proteins (e.g., a receptor and a ligand). Because certain targets of the present invention are transmembrane proteins, assays that use the soluble forms of these proteins rather than full-length protein can be used, in some embodiments. Soluble forms include, for example, those lacking the transmembrane domain and/or those comprising the IgV domain or fragments thereof which retain their ability to bind their cognate binding partners. Further, agents that inhibit or enhance protein interactions for use in the compositions and methods described herein, can include recombinant peptido-mimetics.

Detection methods useful in screening assays include antibody-based methods, detection of a reporter moiety, detection of cytokines as described herein, and detection of a gene signature as described herein.

Another variation of assays to determine binding of a receptor protein to a ligand protein is through the use of affinity biosensor methods. Such methods may be based on the piezoelectric effect, electrochemistry, or optical methods, such as ellipsometry, optical wave guidance, and surface plasmon resonance (SPR).

### Therapeutic Polypeptide Modifications

In certain example embodiments, the therapeutic polypeptides of the present invention may be modified, such that they acquire advantageous properties for therapeutic use (e.g., stability and specificity), but maintain their biological activity. Therapeutic proteins may be modified to increase stability or to provide characteristics that improve efficacy of the protein when administered to a subject *in vivo.* As used herein in reference to therapeutic proteins, the terms "modified", "modification" and the like refer to one or more changes that enhance a desired property of the therapeutic protein, where the change does not alter the primary amino acid sequence of the therapeutic protein. "Modification" includes a covalent chemical modification that does not alter the primary amino acid sequence of the therapeutic protein itself. Such desired properties include, for example, prolonging the *in vivo* half-life, increasing the stability, reducing the clearance, altering the immunogenicity or allergenicity, enabling the raising of particular antibodies, or cellular targeting. Changes to a therapeutic protein that may be carried out include, but are not limited to, conjugation to a carrier protein, conjugation to a ligand, conjugation to an antibody, PEGylation, polysialylation HESylation, recombinant PEG mimetics, Fc fusion, albumin fusion, nanoparticle attachment, nanoparticulate encapsulation, cholesterol fusion, iron fusion, acylation, amidation, glycosylation, side chain oxidation, phosphorylation, biotinylation, the addition of a surface active material, the addition of amino acid mimetics, or the addition of unnatural amino acids. Modified therapeutic proteins also include analogs. By "analog" is meant a molecule that is not identical, but has analogous functional or structural features. For example, a therapeutic protein analog retains the biological activity of a corresponding naturally-occurring polypeptide, while having certain biochemical modifications that enhance the analog's function relative to a naturally-occurring polypeptide. Such biochemical modifications could increase the analog's protease resistance, membrane permeability, or half-life, without altering, for example, ligand binding. An analog may include an unnatural amino acid.

Modified proteins may include a spacer or a linker. The terms "spacer" or "linker" as used in reference to a fusion protein refers to a peptide that joins the proteins comprising a fusion protein. Generally, a spacer has no specific biological activity other than to join or to preserve some minimum distance or other spatial relationship between the proteins. However, in certain embodiments, the constituent amino acids of a spacer may be selected to influence some property of the molecule such as the folding, net charge, or hydrophobicity of the molecule.

Suitable linkers for use in an embodiment of the present invention are well known to those of skill in the art and include, but are not limited to, straight or branched-chain carbon linkers, heterocyclic carbon linkers, or peptide linkers. The linker is used to separate two peptides by a distance sufficient to ensure that, in a preferred embodiment, each peptide properly folds. Preferred peptide linker sequences adopt a flexible extended conformation and do not exhibit a propensity for developing an ordered secondary structure. Typical amino acids in flexible protein regions include Gly, Asn and Ser. Virtually any permutation of amino acid sequences containing Gly, Asn and Ser would be expected to satisfy the above criteria for a linker sequence. Other near neutral amino acids, such as Thr and Ala, also may be used in the linker sequence. Still other amino acid sequences that may be used as linkers are disclosed in Maratea et al. (1985), Gene 40: 39-46; Murphy et al. (1986) Proc. Nat'l. Acad. Sci. USA 83 : 8258-62; U.S. Pat. No. 4,935,233; and U.S. Pat. No. 4,751, 180.

The clinical effectiveness of protein therapeutics is often limited by short plasma half- life and susceptibility to protease degradation. Studies of various therapeutic proteins (e.g., filgrastim) have shown that such difficulties may be overcome by various modifications, including conjugating or linking the polypeptide sequence to any of a variety of nonproteinaceous polymers, e.g., polyethylene glycol (PEG), polypropylene glycol, or polyoxyalkylenes (see, for example, typically via a linking moiety covalently bound to both the protein and the nonproteinaceous polymer, e.g., a PEG).

It is well known that the properties of certain proteins can be modulated by attachment of polyethylene glycol (PEG) polymers, which increases the hydrodynamic volume of the protein and thereby slows its clearance by kidney filtration. (See, e.g., Clark et al., J. Biol. Chem. 271: 21969-21977 (1996)). Such PEG- conjugated biomolecules have been shown to possess clinically useful properties, including better physical and thermal stability, protection against susceptibility to enzymatic degradation, increased solubility, longer in vivo circulating half-life and decreased clearance, reduced immunogenicity and antigenicity, and reduced toxicity. Therefore, it is envisioned that certain agents can be PEGylated (e.g., on peptide residues) to provide enhanced therapeutic benefits such as, for example, increased efficacy by extending half-life *in vivo.* In certain embodiments, PEGylation of the agents may be used to extend the serum half-life of the agents and allow for particular agents to be capable of crossing the blood-brain barrier. Thus, in one embodiment, PEGylating XPR1:KIDINS220 antagonists improve the pharmacokinetics and pharmacodynamics of the antagonists.

In regard to peptide PEGylation methods, reference is made to Lu et al., Int. J. Pept. Protein Res.43: 127-38 (1994); Lu et al., Pept. Res. 6: 140-6 (1993); Felix et al., Int. J. Pept. Protein Res. 46: 253-64 (1995); Gaertner et al., Bioconjug. Chem. 7: 38-44 (1996); Tsutsumi et al., Thromb. Haemost. 77: 168-73 (1997); Francis et al., hit. J. Hematol. 68: 1-18 (1998); Roberts et al., J. Pharm. Sci. 87: 1440-45 (1998); and Tan et al., Protein Expr. Purif. 12: 45-52

(1998). Polyethylene glycol or PEG is meant to encompass any of the forms of PEG that have been used to derivatize other proteins, including, but not limited to, mono-(C1-10) alkoxy or aryloxy-polyethylene glycol. Suitable PEG moieties include, for example, 40 kDa methoxy poly(ethylene glycol) propionaldehyde (Dow, Midland, Mich.); 60 kDa methoxy poly(ethylene glycol) propionaldehyde (Dow, Midland, Mich.); 40 kDa methoxy poly(ethylene glycol) maleimido-propionamide (Dow, Midland, Mich.); 31 kDa alpha-methyl-w-(3-oxopropoxy), polyoxyethylene (NOF Corporation, Tokyo); mPEG2-NHS-40k (Nektar); mPEG2-MAL-40k (Nektar), SUNBRIGHT GL2-400MA ((PEG)240kDa) (NOF Corporation, Tokyo), SUNBRIGHT ME-200MA (PEG20kDa) (NOF Corporation, Tokyo). The PEG groups are generally attached to the peptide (e.g., RBD) via acylation or alkylation through a reactive group on the PEG moiety (for example, a maleimide, an aldehyde, amino, thiol, or ester group) to a reactive group on the peptide (for example, an aldehyde, amino, thiol, a maleimide, or ester group).

The PEG molecule(s) may be covalently attached to any Lys, Cys, or K(CO(CH2)2SH) residues at any position in a peptide. In certain embodiments, the RBD proteins described herein can be PEGylated directly to any amino acid at the N-terminus by way of the N-terminal amino group. A "linker arm" may be added to a peptide to facilitate PEGylation. PEGylation at the thiol side-chain of cysteine has been widely reported (see, e.g., Caliceti & Veronese, Adv. Drug Deliv. Rev. 55: 1261-77 (2003)). If there is no cysteine residue in the peptide, a cysteine residue can be introduced through substitution or by adding a cysteine to the N-terminal amino acid. In certain embodiments, proteins are PEGylated through the side chains of a cysteine residue added to the N-terminal amino acid.

In exemplary embodiments, the PEG molecule(s) may be covalently attached to an amide group in the C-terminus of a peptide, such as in the RBD protein. In certain embodiments, the PEG molecule used in modifying an agent of the present invention is branched while in other embodiments, the PEG molecule may be linear. In particular aspects, the PEG molecule is between 1 kDa and 100 kDa in molecular weight. In further aspects, the PEG molecule is selected from 10, 20, 30, 40, 50, 60, and 80 kDa. In further still aspects, it is selected from 20, 40, or 60 kDa. Where there are two PEG molecules covalently attached to the agent of the present invention, each is 1 to 40 kDa and in particular aspects, they have molecular weights of 20 and 20 kDa, 10 and 30 kDa, 30 and 30 kDa, 20 and 40 kDa, or 40 and 40 kDa. In particular aspects, the agent (e.g., XPR1:KIDINS220 antagonists) contain mPEG-cysteine. The mPEG in mPEG-cysteine can have various molecular weights. The range of the molecular weight is preferably 5 kDa to 200 kDa, more preferably 5 kDa to 100 kDa, and further preferably 20 kDa to 60 kDA. The mPEG can be linear or branched.

The present disclosure also contemplates the use of PEG Mimetics. Recombinant PEG mimetics have been developed that retain the attributes of PEG (e.g., enhanced serum half- life) while conferring several additional advantageous properties. By way of example, simple polypeptide chains (comprising, for example, Ala, Glu, Gly, Pro, Ser and Thr) capable of forming an extended conformation similar to PEG can be produced recombinantly already fused to the therapeutic protein (e.g., Amunix' XTEN technology; Mountain View, CA). This obviates the need for an additional conjugation step during the manufacturing process. Moreover, established molecular biology techniques enable control of the side chain composition of the polypeptide chains, allowing optimization of immunogenicity and manufacturing properties.

Glycosylation can dramatically affect the physical properties of proteins and can also be important in protein stability, secretion, and subcellular localization (see, e.g., Solá and Griebenow, Glycosylation of Therapeutic Proteins: An Effective Strategy to Optimize Efficacy. BioDrugs. 2010; 24(1): 9-21). Proper glycosylation can be essential for biological activity. In fact, some genes from eukaryotic organisms, when expressed in bacteria (e.g., E. coli) which lack cellular processes for glycosylating proteins, yield proteins that are recovered with little or no activity by virtue of their lack of glycosylation. For purposes of the present disclosure, "glycosylation" is meant to broadly refer to the enzymatic process that attaches glycans to proteins, lipids or other organic molecules. The use of the term "glycosylation" in conjunction with the present disclosure is generally intended to mean adding or deleting one or more carbohydrate moieties (either by removing the underlying glycosylation site or by deleting the glycosylation by chemical and/or enzymatic means), and/or adding one or more glycosylation sites that may or may not be present in the native sequence. In addition, the phrase includes qualitative changes in the glycosylation of the native proteins involving a change in the nature and proportions of the various carbohydrate moieties present.

Addition of glycosylation sites can be accomplished by altering the amino acid sequence. The alteration to the polypeptide may be made, for example, by the addition of, or substitution by, one or more serine or threonine residues (for O-linked glycosylation sites) or asparagine residues (for N-linked glycosylation sites). The structures of N-linked and O- linked oligosaccharides and the sugar residues found in each type may be different. One type of sugar that is commonly found on both is N-acetylneuraminic acid (hereafter referred to as sialic acid). Sialic acid is usually the terminal residue of both N-linked and O-linked oligosaccharides and, by virtue of its negative charge, may confer acidic properties to the glycoprotein. A particular embodiment of the present disclosure comprises the generation and use of N-glycosylation variants.

The present disclosure also contemplates the use of polysialylation, the conjugation of peptides and proteins to the naturally occurring, biodegradable a-(2→8) linked polysialic acid ("PSA") in order to improve their stability and in vivo pharmacokinetics. PSA is a biodegradable, non-toxic natural polymer that is highly hydrophilic, giving it a high apparent molecular weight in the blood which increases its serum half-life. In addition, polysialylation of a range of peptide and protein therapeutics has led to markedly reduced proteolysis, retention of activity in vivo activity, and reduction in immunogenicity and antigenicity (see, e.g., G. Gregoriadis et al., Int. J. Pharmaceutics 300(1-2): 125-30). As with modifications with other conjugates (e.g., PEG), various techniques for site-specific polysialylation are available (see, e.g., T. Lindhout et al., PNAS 108(18)7397-7402 (2011)).

Additional suitable components and molecules for conjugation include, for example, thyroglobulin; albumins such as human serum albumin (HAS); tetanus toxoid; Diphtheria toxoid; polyamino acids such as poly(D-lysine:D-glutamic acid); VP6 polypeptides of rotaviruses; influenza virus hemaglutinin, influenza virus nucleoprotein; Keyhole Limpet Hemocyanin (KLH); and hepatitis B virus core protein and surface antigen; or any combination of the foregoing.

Fusion of albumin to one or more polypeptides of the present disclosure can, for example, be achieved by genetic manipulation, such that the DNA coding for HSA, or a fragment thereof, is joined to the DNA coding for the one or more polypeptide sequences. Albumin itself may be modified to extend its circulating half-life. Fusion of the modified albumin to one or more Polypeptides can be attained by the genetic manipulation techniques described above or by chemical conjugation; the resulting fusion molecule has a half- life that exceeds that of fusions with non-modified albumin. (See WO2011/051489).

Several albumin - binding strategies have been developed as alternatives for direct fusion, including albumin binding through a conjugated fatty acid chain (acylation). Because serum albumin is a transport protein for fatty acids, these natural ligands with albumin - binding activity have been used for half-life extension of small protein therapeutics. For example, insulin determir (LEVEMIR), an approved product for diabetes, comprises a myristyl chain conjugated to a genetically-modified insulin, resulting in a long- acting insulin analog.

Another type of modification is to conjugate (e.g., link) one or more additional components or molecules at the N- and/or C-terminus of a polypeptide sequence, such as another protein, or a carrier molecule. Thus, an exemplary polypeptide sequence can be provided as a conjugate with another component or molecule. A conjugate modification may result in a polypeptide sequence that retains activity with an additional or complementary function or activity of the second molecule. For example, a polypeptide sequence may be conjugated to a molecule, e.g., to facilitate solubility, storage, in vivo or shelf half-life or stability, reduction in immunogenicity, delayed or controlled release in vivo, etc. Other functions or activities include a conjugate that reduces toxicity relative to an unconjugated polypeptide sequence, a conjugate that targets a type of cell or organ more efficiently than an unconjugated polypeptide sequence, or a drug to further counter the causes or effects associated with a disorder or disease as set forth herein.

The present disclosure contemplates the use of other modifications, currently known or developed in the future, of the Polypeptides to improve one or more properties. One such method for prolonging the circulation half-life, increasing the stability, reducing the clearance, or altering the immunogenicity or allergenicity of a polypeptide of the present disclosure involves modification of the polypeptide sequences by hesylation, which utilizes hydroxyethyl starch derivatives linked to other molecules in order to modify the molecule's characteristics. Various aspects of hesylation are described in, for example, U.S. Patent Appln. Nos. 2007/0134197 and 2006/0258607.

In particular embodiments, the agents (e.g., XPR1:KIDINS220 antagonists, RBD) include a protecting group covalently joined to the N-terminal amino group. In exemplary embodiments, a protecting group covalently joined to the N-terminal amino group of the proteins reduces the reactivity of the amino terminus under *in vivo* conditions. Amino protecting groups include -C1-10 alkyl, -C1-10 substituted alkyl, -C2-10 alkenyl, -C2-10 substituted alkenyl, aryl, -C1-6 alkyl aryl, -C(O)-(CH2)1-6-COOH, -C(O)-C1-6 alkyl, -C(O)-aryl, -C(O)-O-C1-6 alkyl, or -C(O)-O-aryl. In particular embodiments, the amino terminus protecting group is selected from the group consisting of acetyl, propyl, succinyl, benzyl, benzyloxycarbonyl, and t-butyloxycarbonyl. In other embodiments, deamination of the N-terminal amino acid is another modification that may be used for reducing the reactivity of the amino terminus under *in vivo* conditions.

Chemically modified compositions of the agents (e.g., XPR1:KIDINS220 antagonists, RBD) wherein the agent is linked to a polymer are also included within the scope of the present invention. The polymer selected is usually modified to have a single reactive group, such as an active ester for acylation or an aldehyde for alkylation, so that the degree of polymerization may be controlled. Included within the scope of polymers is a mixture of polymers. Preferably, for therapeutic use of the end-product preparation, the polymer will be pharmaceutically acceptable. The polymer or mixture thereof may include but is not limited to polyethylene glycol (PEG), monomethoxy-polyethylene glycol, dextran, cellulose, or other carbohydrate-based polymers, poly-(N-vinyl pyrrolidone) polyethylene glycol, propylene glycol homopolymers, a polypropylene oxide/ethylene oxide co-polymer, polyoxyethylated polyols (for example, glycerol), and polyvinyl alcohol.

In other embodiments, the agents (e.g., XPR1:KIDINS220 antagonists, RBD) are modified by PEGylation, cholesterylation, or palmitoylation. The modification can be to any amino acid residue. In preferred embodiments, the modification is to the N-terminal amino acid of the agent (e.g., XPR1:KIDINS220 antagonists, RBD), either directly to the N-terminal amino acid or by way coupling to the thiol group of a cysteine residue added to the N-terminus or a linker added to the N-terminus such as trimesoyl tris(3,5- dibromosalicylate (Ttds). In certain embodiments, the N-terminus of the agent (e.g., XPR1:KIDINS220 antagonists, RBD) comprises a cysteine residue to which a protecting group is coupled to the N-terminal amino group of the cysteine residue and the cysteine thiolate group is derivatized with N-ethylmaleimide, PEG group, cholesterol group, or palmitoyl group. In other embodiments, an acetylated cysteine residue is added to the N-terminus of the agents, and the thiol group of the cysteine is derivatized with N-ethylmaleimide, PEG group, cholesterol group, or palmitoyl group. In certain embodiments, the agent of the present invention is a conjugate. In certain embodiments, the agent of the present invention is a polypeptide consisting of an amino acid sequence which is bound with a methoxypolyethylene glycol(s) via a linker.

Substitutions of amino acids may be used to modify an agent of the present invention. The phrase "substitution of amino acids" as used herein encompasses substitution of amino acids that are the result of both conservative and non-conservative substitutions. Conservative substitutions are the replacement of an amino acid residue by another similar residue in a polypeptide. Typical but not limiting conservative substitutions are the replacements, for one another, among the aliphatic amino acids Ala, Val, Leu and Ile; interchange of Ser and Thr containing hydroxy residues, interchange of the acidic residues Asp and Glu, interchange between the amide-containing residues Asn and Gln, interchange of the basic residues Lys and Arg, interchange of the aromatic residues Phe and Tyr, and interchange of the small-sized amino acids Ala, Ser, Thr, Met, and Gly. Non-conservative substitutions are the replacement, in a polypeptide, of an amino acid residue by another residue which is not biologically similar. For example, the replacement of an amino acid residue with another residue that has a substantially different charge, a substantially different hydrophobicity, or a substantially different spatial configuration.

One of skill in the art from this disclosure and the knowledge in the art will appreciate that there are a variety of ways in which to produce such therapeutic proteins. In general, such therapeutic proteins may be produced either in vitro or in vivo. Therapeutic proteins may be produced in vitro as peptides or polypeptides, which may then be formulated into a pharmaceutical composition and administered to a subject. Such in vitro production may occur by a variety of methods known to one of skill in the art such as, for example, peptide synthesis or expression of a peptide/polypeptide from a DNA or RNA molecule in any of a variety of bacterial, eukaryotic, or viral recombinant expression systems, followed by purification of the expressed peptide/polypeptide. Alternatively, therapeutic proteins may be produced in vivo by introducing molecules (e.g., DNA, RNA, viral expression systems, and the like) that encode therapeutic proteins into a subject, whereupon the encoded therapeutic proteins are expressed.

### Small Molecules

In certain embodiments, the one or more therapeutic agents comprise a small molecule that inhibits expression of XPR1, KINDINS220, inhibits formation of the XPR1:KINDINS220 complex, or inhibits phosphate efflux by the XPR1:KINDINS220 complex. The term "small molecule" refers to compounds, preferably organic compounds, with a size comparable to those organic molecules generally used in pharmaceuticals. The term excludes biological macromolecules (e.g., proteins, peptides, nucleic acids, etc.). Preferred small organic molecules range in size up to about 5000 Da, e.g., up to about 4000, preferably up to 3000 Da, more preferably up to 2000 Da, even more preferably up to about 1000 Da, e.g., up to about 900, 800, 700, 600 or up to about 500 Da. In certain embodiments, the small molecule may act as an antagonist or agonist (e.g., blocking an enzyme active site or activating a receptor by binding to a ligand binding site). In certain embodiments, the small molecule blocks or disrupts the XPR1:KIDINS220 protein complex from functioning to export inorganic phosphate from a tumor cell. In certain embodiments, the small molecule blocks the XPR1:KIDINS220 protein complex from forming.

In one example embodiment, a small molecule blocks a phosphate binding domain, such as the Walker A/B motif of KIDINS220 (see, e.g., Sandall CF, Ziehr BK, MacDonald JA. ATP-Binding and Hydrolysis in Inflammasome Activation. Molecules. 2020;25(19):4572). MCC950 is a diarylsulfonylurea-containing compound that suppresses ATP hydrolysis through direct binding of the ATP binding region of NLRP3, likely within the Walker B motif (see, e.g., Coll RC, Hill JR, Day CJ, et al. MCC950 directly targets the NLRP3 ATP-hydrolysis motif for inflammasome inhibition. Nat Chem Biol. 2019;15(6):556-559).

One type of small molecule applicable to the present invention is a degrader molecule (see, e.g., Ding, et al., Emerging New Concepts of Degrader Technologies, Trends Pharmacol Sci. 2020 Jul;41(7):464-474). The terms "degrader" and "degrader molecule" refer to all compounds capable of specifically targeting a protein for degradation (e.g., ATTEC, AUTAC, LYTAC, or PROTAC, reviewed in Ding, et al. 2020). Proteolysis Targeting Chimera (PROTAC) technology is a rapidly emerging alternative therapeutic strategy with the potential to address many of the challenges currently faced in modern drug development programs. PROTAC technology employs small molecules that recruit target proteins for ubiquitination and removal by the proteasome (see, e.g., Zhou et al., Discovery of a Small-Molecule Degrader of Bromodomain and Extra- Terminal (BET) Proteins with Picomolar Cellular Potencies and Capable of Achieving Tumor Regression. J. Med. Chem. 2018, 61, 462-481; Bondeson and Crews, Targeted Protein Degradation by Small Molecules, Annu Rev Pharmacol Toxicol. 2017 Jan 6; 57: 107-123; and Lai et al., Modular PROTAC Design for the Degradation of Oncogenic BCR-ABL Angew Chem Int Ed Engl. 2016 Jan 11; 55(2): 807-810). In certain embodiments, LYTACs are particularly advantageous for cell surface proteins as described herein (e.g., XPR1 and/or KIDINS220).

### Aptamers

In certain embodiments, the one or more agents is an aptamer. Nucleic acid aptamers are nucleic acid species that have been engineered through repeated rounds of *in vitro* selection or equivalently, SELEX (systematic evolution of ligands by exponential enrichment) to bind to various molecular targets such as small molecules, proteins, nucleic acids, cells, tissues and organisms. Nucleic acid aptamers have specific binding affinity to molecules through interactions other than classic Watson-Crick base pairing. Aptamers are useful in biotechnological and therapeutic applications as they offer molecular recognition properties similar to antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications. In certain embodiments, RNA aptamers may be expressed from a DNA construct. In other embodiments, a nucleic acid aptamer may be linked to another polynucleotide sequence. The polynucleotide sequence may be a double stranded DNA polynucleotide sequence. The aptamer may be covalently linked to one strand of the polynucleotide sequence. The aptamer may be ligated to the polynucleotide sequence. The polynucleotide sequence may be configured, such that the polynucleotide sequence may be linked to a solid support or ligated to another polynucleotide sequence.

Aptamers, like peptides generated by phage display or monoclonal antibodies ("mAbs"), are capable of specifically binding to selected targets and modulating the target's activity, e.g., through binding, aptamers may block their target's ability to function. A typical aptamer is 10-15 kDa in size (30-45 nucleotides), binds its target with sub-nanomolar affinity, and discriminates against closely related targets (e.g., aptamers will typically not bind other proteins from the same gene family). Structural studies have shown that aptamers are capable of using the same types of binding interactions (e.g., hydrogen bonding, electrostatic complementarity, hydrophobic contacts, steric exclusion) that drives affinity and specificity in antibody-antigen complexes.

Aptamers have a number of desirable characteristics for use in research and as therapeutics and diagnostics including high specificity and affinity, biological efficacy, and excellent pharmacokinetic properties. In addition, they offer specific competitive advantages over antibodies and other protein biologics. Aptamers are chemically synthesized and are readily scaled as needed to meet production demand for research, diagnostic or therapeutic applications. Aptamers are chemically robust. They are intrinsically adapted to regain activity following exposure to factors such as heat and denaturants and can be stored for extended periods (>1 yr) at room temperature as lyophilized powders. Not being bound by a theory, aptamers bound to a solid support or beads may be stored for extended periods.

Oligonucleotides in their phosphodiester form may be quickly degraded by intracellular and extracellular enzymes such as endonucleases and exonucleases. Aptamers can include modified nucleotides conferring improved characteristics on the ligand, such as improved *in vivo* stability or improved delivery characteristics. Examples of such modifications include chemical substitutions at the ribose and/or phosphate and/or base positions. SELEX identified nucleic acid ligands containing modified nucleotides are described, e.g., in U.S. Pat. No. 5,660,985, which describes oligonucleotides containing nucleotide derivatives chemically modified at the 2' position of ribose, 5 position of pyrimidines, and 8 position of purines, U.S. Pat. No. 5,756,703 which describes oligonucleotides containing various 2' -modified pyrimidines, and U.S. Pat. No. 5,580,737 which describes highly specific nucleic acid ligands containing one or more nucleotides modified with 2'-amino (2'-NH₂), 2'-fluoro (2'-F), and/or 2'-0-methyl (2'-OMe) substituents. Modifications of aptamers may also include, modifications at exocyclic amines, substitution of 4- thiouridine, substitution of 5-bromo or 5-iodo-uracil; backbone modifications, phosphorothioate or allyl phosphate modifications, methylations, and unusual base-pairing combinations such as the isobases isocytidine and isoguanosine. Modifications can also include 3' and 5' modifications such as capping. As used herein, the term phosphorothioate encompasses one or more non-bridging oxygen atoms in a phosphodiester bond replaced by one or more sulfur atoms. In further embodiments, the oligonucleotides comprise modified sugar groups, for example, one or more of the hydroxyl groups is replaced with halogen, aliphatic groups, or functionalized as ethers or amines. In one embodiment, the 2'-position of the furanose residue is substituted by any of an O-methyl, O-alkyl, O-allyl, S-alkyl, S-allyl, or halo group. Methods of synthesis of 2'-modified sugars are described, e.g., in Sproat, et al., Nucl. Acid Res. 19:733-738 (1991); Cotten, et al, Nucl. Acid Res. 19:2629-2635 (1991); and Hobbs, et al, Biochemistry 12:5138-5145 (1973). Other modifications are known to one of ordinary skill in the art. In certain embodiments, aptamers include aptamers with improved off-rates as described in International Patent Publication No. WO 2009012418, "Method for generating aptamers with improved off-rates,". In certain embodiments aptamers are chosen from a library of aptamers. Such libraries include, but are not limited to, those described in Rohloff et al., "Nucleic Acid Ligands With Protein-like Side Chains: Modified Aptamers and Their Use as Diagnostic and Therapeutic Agents," Molecular Therapy Nucleic Acids (2014) 3, e201. Aptamers are also commercially available (see, e.g., SomaLogic, Inc., Boulder, Colorado). In certain embodiments, the present invention may utilize any aptamer containing any modification as described herein.

### Programmable Genetic modifying agents

In certain example embodiments, a programmable nuclease may be used to edit a genomic region containing XPR1 or KIDINS220. Programmable genetic modifying agents are enzymes capable of being engineered to bind a specific target sequences. Example programmable genetic modifying agents include zinc finger nucleases, TALE nucleases (TALENS), meganucleases, and CRISPR-Cas systems. In the context of the present invention, programmable genetic modifying agents may be designed to target and/or modify genomic DNA or mRNA of XPR1 and/or KIDINS220. The modifications may reduce expression of XPR1 and/or KIDINS220, or may introduce structural or post-translations modifications that inhibit XPR1:KIDINS220 complex formation. Not being bound by a theory, normal cells are not vulnerable to XPR1:KIDINS220-mediated phosphate export inhibition, however, it is advantageous to temporarily inhibit XPR1:KIDINS220-mediated phosphate export. Thus, in certain embodiments, mRNA is targeted (e.g., RNA base editing).

### CRISPR-Cas Modification

CRISPR-Cas systems comprise an endonuclease (Cas protein) capable of forming a complex with a guide molecule. The guide molecule can be engineered to comprise a sequence complementary to a given target sequence (e.g., a target sequence within a region of XPR1 or KIDINS220). The guide molecule guides the complex to the target site where the Cas endonuclease introduce a single or double-stranded cut in the target sequence. Native cellular repair pathways, NHEJ and HDR, are used to repair the gut. NHEJ may introduce insertions or deletions at the cut site. Accordingly, CRISPR-Cas systems can be designed to introduce insertions or deletions that reduce or eliminate expression or interfere with XPR1/KINDS220 complex formation. Alternatively, template molecules can be delivered with CRISPR-Cas systems that utilize the HDR pathway to introduce insertions of desired template sequences. These insertions may introduce one or more mutations that reduce or eliminate expression or interfere with XPR1/KINDS220 complex formation. The insertions may remove or introduce post-translation modification sites, introduce premature stop codons, or disrupt splice sites that result in protein products with loss of function or reduced function. CRISPR-Cas systems may also be modified to work with additional functional domains. In such embodiments, the endonuclease activity of the Cas protein is eliminated to create a dead Cas (dCas). The dCas9 is then fused with a functional domain. The dCas-guide complex directs the functional domain to the target sequence, where the functional domain introduces a modification to a DNA or RNA target sequence. Modified CRISPR-Cas systems include DNA and RNA base editors, primer editors, and CRISPR associated transposase (CAST) systems, which are described in further detail below.

In general, a CRISPR-Cas or CRISPR system as used herein and in other documents, such as WO 2014/093622 (PCT/US2013/074667), refers collectively to transcripts and other elements involved in the expression of or directing the activity of CRISPR-associated ("Cas") genes, including sequences encoding a Cas gene, a tracr (trans-activating CRISPR) sequence (e.g., tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or "RNA(s)" as that term is herein used (e.g., RNA(s) to guide Cas, such as Cas9, e.g., CRISPR RNA and transactivating (tracr) RNA or a single guide RNA (sgRNA) (chimeric RNA)) or other sequences and transcripts from a CRISPR locus. In general, a CRISPR system is characterized by elements that promote the formation of a CRISPR complex at the site of a target sequence (also referred to as a protospacer in the context of an endogenous CRISPR system). See, e.g, Shmakov et al. (2015) "Discovery and Functional Characterization of Diverse Class 2 CRISPR-Cas Systems", Molecular Cell, DOI: dx.doi.org/10.1016/j.molcel.2015.10.008.

CRISPR-Cas systems can generally fall into two classes based on their architectures of their effector molecules, which are each further subdivided by type and subtype. The two class are Class 1 and Class 2. Class 1 CRISPR-Cas systems have effector modules composed of multiple Cas proteins, some of which form crRNA-binding complexes, while Class 2 CRISPR-Cas systems include a single, multi-domain crRNA-binding protein.

In some embodiments, the CRISPR-Cas system that can be used to modify a polynucleotide of the present invention described herein can be a Class 1 CRISPR-Cas system. In some embodiments, the CRISPR-Cas system that can be used to modify a polynucleotide of the present invention described herein can be a Class 2 CRISPR-Cas system.

### Class 1 CRISPR-Cas Systems

In some embodiments, the CRISPR-Cas system that can be used to modify a polynucleotide of the present invention described herein can be a Class 1 CRISPR-Cas system. Class 1 CRISPR-Cas systems are divided into types I, II, and IV. Makarova et al. 2020. Nat. Rev. 18: 67-83., particularly as described in Figure 1. Type I CRISPR-Cas systems are divided into 9 subtypes (I-A, I-B, I-C, I-D, I-E, I-F1, I-F2, I-F3, and IG). Makarova *et al*., 2020. Class 1, Type I CRISPR-Cas systems can contain a Cas3 protein that can have helicase activity. Type III CRISPR-Cas systems are divided into 6 subtypes (III-A, III-B, III-C, III-D, III-E, and III-F). Type III CRISPR-Cas systems can contain a Cas10 that can include an RNA recognition motif called Palm and a cyclase domain that can cleave polynucleotides. Makarova *et al*., 2020. Type IV CRISPR-Cas systems are divided into 3 subtypes. (IV-A, IV-B, and IV-C). Makarova *et al*., 2020. Class 1 systems also include CRISPR-Cas variants, including Type I-A, I-B, I-E, I-F and I-U variants, which can include variants carried by transposons and plasmids, including versions of subtype I-F encoded by a large family of Tn7-like transposon and smaller groups of Tn7-like transposons that encode similarly degraded subtype I-B systems. Peters et al., PNAS 114 (35) (2017); DOI: 10.1073/pnas.1709035114; *see also,* Makarova et al. 2018. The CRISPR Journal, v. 1, n5, Figure 5.

The Class 1 systems typically use a multi-protein effector complex, which can, in some embodiments, include ancillary proteins, such as one or more proteins in a complex referred to as a CRISPR-associated complex for antiviral defense (Cascade), one or more adaptation proteins (e.g., Cas1, Cas2, RNA nuclease), and/or one or more accessory proteins (e.g., Cas 4, DNA nuclease), CRISPR associated Rossman fold (CARF) domain containing proteins, and/or RNA transcriptase.

The backbone of the Class 1 CRISPR-Cas system effector complexes can be formed by RNA recognition motif domain-containing protein(s) of the repeat-associated mysterious proteins (RAMPs) family subunits (e.g., Cas 5, Cas6, and/or Cas7). RAMP proteins are characterized by having one or more RNA recognition motif domains. In some embodiments, multiple copies of RAMPs can be present. In some embodiments, the Class I CRISPR-Cas system can include 1, 2, 3, 4, 5, 6, 7, 8, 9, 10, 11, 12 or more Cas5, Cas6, and/or Cas 7 proteins. In some embodiments, the Cas6 protein is an RNAse, which can be responsible for pre-crRNA processing. When present in a Class 1 CRISPR-Cas system, Cas6 can be optionally physically associated with the effector complex.

Class 1 CRISPR-Cas system effector complexes can, in some embodiments, also include a large subunit. The large subunit can be composed of or include a Cas8 and/or Cas10 protein. *See,* e.g., Figures 1 and 2. Koonin EV, Makarova KS. 2019. Phil. Trans. R. Soc. B 374: 20180087, DOI: 10.1098/rstb.2018.0087 and Makarova et al. 2020.

Class 1 CRISPR-Cas system effector complexes can, in some embodiments, include a small subunit (for example, Cas11). *See,* e.g., Figures 1 and 2. Koonin EV, Makarova KS. 2019 Origins and Evolution of CRISPR-Cas systems. Phil. Trans. R. Soc. B 374: 20180087, DOI: 10.1098/rstb.2018.0087.

In some embodiments, the Class 1 CRISPR-Cas system can be a Type I CRISPR-Cas system. In some embodiments, the Type I CRISPR-Cas system can be a subtype I-A CRISPR-Cas system. In some embodiments, the Type I CRISPR-Cas system can be a subtype I-B CRISPR-Cas system. In some embodiments, the Type I CRISPR-Cas system can be a subtype I-C CRISPR-Cas system. In some embodiments, the Type I CRISPR-Cas system can be a subtype I-D CRISPR-Cas system. In some embodiments, the Type I CRISPR-Cas system can be a subtype I-E CRISPR-Cas system. In some embodiments, the Type I CRISPR-Cas system can be a subtype I-F1 CRISPR-Cas system. In some embodiments, the Type I CRISPR-Cas system can be a subtype I-F2 CRISPR-Cas system. In some embodiments, the Type I CRISPR-Cas system can be a subtype I-F3 CRISPR-Cas system. In some embodiments, the Type I CRISPR-Cas system can be a subtype I-G CRISPR-Cas system. In some embodiments, the Type I CRISPR-Cas system can be a CRISPR Cas variant, such as a Type I-A, I-B, I-E, IF and I-U variants, which can include variants carried by transposons and plasmids, including versions of subtype I-F encoded by a large family of Tn7-like transposon and smaller groups of Tn7-like transposons that encode similarly degraded subtype I-B systems as previously described.

In some embodiments, the Class 1 CRISPR-Cas system can be a Type III CRISPR-Cas system. In some embodiments, the Type III CRISPR-Cas system can be a subtype III-A CRISPR-Cas system. In some embodiments, the Type III CRISPR-Cas system can be a subtype III-B CRISPR-Cas system. In some embodiments, the Type III CRISPR-Cas system can be a subtype III-C CRISPR-Cas system. In some embodiments, the Type III CRISPR-Cas system can be a subtype III-D CRISPR-Cas system. In some embodiments, the Type III CRISPR-Cas system can be a subtype III-E CRISPR-Cas system. In some embodiments, the Type III CRISPR-Cas system can be a subtype III-F CRISPR-Cas system.

In some embodiments, the Class 1 CRISPR-Cas system can be a Type IV CRISPR-Cas-system. In some embodiments, the Type IV CRISPR-Cas system can be a subtype IV-A CRISPR-Cas system. In some embodiments, the Type IV CRISPR-Cas system can be a subtype IV-B CRISPR-Cas system. In some embodiments, the Type IV CRISPR-Cas system can be a subtype IV-C CRISPR-Cas system.

The effector complex of a Class 1 CRISPR-Cas system can, in some embodiments, include a Cas3 protein that is optionally fused to a Cas2 protein, a Cas4, a Cas5, a Cas6, a Cas7, a Cas8, a Cas10, a Cas11, or a combination thereof. In some embodiments, the effector complex of a Class 1 CRISPR-Cas system can have multiple copies, such as 1, 2, 3, 4, 5, 6, 7, 8, 9, 10, 11, 12, 13, or 14, of any one or more Cas proteins.

### Class 2 CRISPR-Cas Systems

The compositions, systems, and methods described in greater detail elsewhere herein can be designed and adapted for use with Class 2 CRISPR-Cas systems. Thus, in some embodiments, the CRISPR-Cas system is a Class 2 CRISPR-Cas system. Class 2 systems are distinguished from Class 1 systems in that they have a single, large, multi-domain effector protein. In certain example embodiments, the Class 2 system can be a Type II, Type V, or Type VI system, which are described in Makarova et al. "Evolutionary classification of CRISPR-Cas systems: a burst of class 2 and derived variants" Nature Reviews Microbiology, 18:67-81 (Feb 2020). Each type of Class 2 system is further divided into subtypes. *See* Markova et al. 2020, particularly at Figure. 2. Class 2, Type II systems can be divided into 4 subtypes: II-A, II-B, II-C1, and II-C2. Class 2, Type V systems can be divided into 17 subtypes: V-A, V-B1, V-B2, V-C, V-D, V-E, V-F1, V-F1(V-U3), V-F2, V-F3, V-G, V-H, V-I, V-K (V-U5), V-U1, V-U2, and V-U4. Class 2, Type IV systems can be divided into 5 subtypes: VI-A, VI-B1, VI-B2, VI-C, and VI-D.

The distinguishing feature of these types is that their effector complexes consist of a single, large, multi-domain protein. Type V systems differ from Type II effectors (e.g., Cas9), which contain two nuclear domains that are each responsible for the cleavage of one strand of the target DNA, with the HNH nuclease inserted inside the Ruv-C like nuclease domain sequence. The Type V systems (e.g., Cas12) only contain a RuvC-like nuclease domain that cleaves both strands. Type VI (Cas13) are unrelated to the effectors of Type II and V systems and contain two HEPN domains and target RNA. Cas13 proteins also display collateral activity that is triggered by target recognition. Some Type V systems have also been found to possess this collateral activity with two single-stranded DNA in *in vitro* contexts.

In some embodiments, the Class 2 system is a Type II system. In some embodiments, the Type II CRISPR-Cas system is a II-A CRISPR-Cas system. In some embodiments, the Type II CRISPR-Cas system is a II-B CRISPR-Cas system. In some embodiments, the Type II CRISPR-Cas system is a II-C1 CRISPR-Cas system. In some embodiments, the Type II CRISPR-Cas system is a II-C2 CRISPR-Cas system. In some embodiments, the Type II system is a Cas9 system. In some embodiments, the Type II system includes a Cas9.

In some embodiments, the Class 2 system is a Type V system. In some embodiments, the Type V CRISPR-Cas system is a V-A CRISPR-Cas system. In some embodiments, the Type V CRISPR-Cas system is a V-B1 CRISPR-Cas system. In some embodiments, the Type V CRISPR-Cas system is a V-B2 CRISPR-Cas system. In some embodiments, the Type V CRISPR-Cas system is a V-C CRISPR-Cas system. In some embodiments, the Type V CRISPR-Cas system is a V-D CRISPR-Cas system. In some embodiments, the Type V CRISPR-Cas system is a V-E CRISPR-Cas system. In some embodiments, the Type V CRISPR-Cas system is a V-F1 CRISPR-Cas system. In some embodiments, the Type V CRISPR-Cas system is a V-F1 (V-U3) CRISPR-Cas system. In some embodiments, the Type V CRISPR-Cas system is a V-F2 CRISPR-Cas system. In some embodiments, the Type V CRISPR-Cas system is a V-F3 CRISPR-Cas system. In some embodiments, the Type V CRISPR-Cas system is a V-G CRISPR-Cas system. In some embodiments, the Type V CRISPR-Cas system is a V-H CRISPR-Cas system. In some embodiments, the Type V CRISPR-Cas system is a V-I CRISPR-Cas system. In some embodiments, the Type V CRISPR-Cas system is a V-K (V-U5) CRISPR-Cas system. In some embodiments, the Type V CRISPR-Cas system is a V-U1 CRISPR-Cas system. In some embodiments, the Type V CRISPR-Cas system is a V-U2 CRISPR-Cas system. In some embodiments, the Type V CRISPR-Cas system is a V-U4 CRISPR-Cas system. In some embodiments, the Type V CRISPR-Cas system includes a Cas12a (Cpf1), Cas12b (C2c1), Cas12c (C2c3), CasX, and/or Cas14.

In some embodiments the Class 2 system is a Type VI system. In some embodiments, the Type VI CRISPR-Cas system is a VI-A CRISPR-Cas system. In some embodiments, the Type VI CRISPR-Cas system is a VI-B 1 CRISPR-Cas system. In some embodiments, the Type VI CRISPR-Cas system is a VI-B2 CRISPR-Cas system. In some embodiments, the Type VI CRISPR-Cas system is a VI-C CRISPR-Cas system. In some embodiments, the Type VI CRISPR-Cas system is a VI-D CRISPR-Cas system. In some embodiments, the Type VI CRISPR-Cas system includes a Cas13a (C2c2), Cas13b (Group 29/30), Cas13c, and/or Cas13d.

### Specialized Cas-based Systems

In some embodiments, the system is a Cas-based system that is capable of performing a specialized function or activity. For example, the Cas protein may be fused, operably coupled to, or otherwise associated with one or more functionals domains. In certain example embodiments, the Cas protein may be a catalytically dead Cas protein ("dCas") and/or have nickase activity. A nickase is a Cas protein that cuts only one strand of a double stranded target. In such embodiments, the dCas or nickase provide a sequence specific targeting functionality that delivers the functional domain to or proximate a target sequence. Example functional domains that may be fused to, operably coupled to, or otherwise associated with a Cas protein can be or include, but are not limited to a nuclear localization signal (NLS) domain, a nuclear export signal (NES) domain, a translational activation domain, a transcriptional activation domain (e.g. VP64, p65, MyoD1, HSF1, RTA, and SET7/9), a translation initiation domain, a transcriptional repression domain (e.g., a KRAB domain, NuE domain, NcoR domain, and a SID domain such as a SID4X domain), a nuclease domain (e.g., FokI), a histone modification domain (e.g., a histone acetyltransferase), a light inducible/controllable domain, a chemically inducible/controllable domain, a transposase domain, a homologous recombination machinery domain, a recombinase domain, an integrase domain, and combinations thereof. Methods for generating catalytically dead Cas9 or a nickase Cas9 (WO 2014/204725, Ran et al. Cell. 2013 Sept 12; 154(6):1380-1389), Cas12 (Liu et al. Nature Communications, 8, 2095 (2017), and Cas13 (WO 2019/005884, WO2019/060746) are known in the art.

In some embodiments, the functional domains can have one or more of the following activities: methylase activity, demethylase activity, translation activation activity, translation initiation activity, translation repression activity, transcription activation activity, transcription repression activity, transcription release factor activity, histone modification activity, nuclease activity, single-strand RNA cleavage activity, double-strand RNA cleavage activity, single-strand DNA cleavage activity, double-strand DNA cleavage activity, molecular switch activity, chemical inducibility, light inducibility, and nucleic acid binding activity. In some embodiments, the one or more functional domains may comprise epitope tags or reporters. Non-limiting examples of epitope tags include histidine (His) tags, V5 tags, FLAG tags, influenza hemagglutinin (HA) tags, Myc tags, VSV-G tags, and thioredoxin (Trx) tags. Examples of reporters include, but are not limited to, glutathione-S-transferase (GST), horseradish peroxidase (HRP), chloramphenicol acetyltransferase (CAT) beta-galactosidase, beta-glucuronidase, luciferase, green fluorescent protein (GFP), HcRed, DsRed, cyan fluorescent protein (CFP), yellow fluorescent protein (YFP), and auto-fluorescent proteins including blue fluorescent protein (BFP).

The one or more functional domain(s) may be positioned at, near, and/or in proximity to a terminus of the effector protein (e.g., a Cas protein). In embodiments having two or more functional domains, each of the two can be positioned at or near or in proximity to a terminus of the effector protein (e.g., a Cas protein). In some embodiments, such as those where the functional domain is operably coupled to the effector protein, the one or more functional domains can be tethered or linked via a suitable linker (including, but not limited to, GlySer linkers) to the effector protein (e.g., a Cas protein). When there is more than one functional domain, the functional domains can be same or different. In some embodiments, all the functional domains are the same. In some embodiments, all of the functional domains are different from each other. In some embodiments, at least two of the functional domains are different from each other. In some embodiments, at least two of the functional domains are the same as each other.

Other suitable functional domains can be found, for example, in International Application Publication No. WO 2019/018423.

### Split CRISPR-Cas systems

In some embodiments, the CRISPR-Cas system is a split CRISPR-Cas system. *See* e.g., Zetche et al., 2015. Nat. Biotechnol. 33(2): 139-142 and WO 2019/018423, the compositions and techniques of which can be used in and/or adapted for use with the present invention. Split CRISPR-Cas proteins are set forth herein and in documents in further detail herein. In certain embodiments, each part of a split CRISPR protein are attached to a member of a specific binding pair, and when bound with each other, the members of the specific binding pair maintain the parts of the CRISPR protein in proximity. In certain embodiments, each part of a split CRISPR protein is associated with an inducible binding pair. An inducible binding pair is one which is capable of being switched "on" or "off" by a protein or small molecule that binds to both members of the inducible binding pair. In some embodiments, CRISPR proteins may preferably split between domains, leaving domains intact. In particular embodiments, said Cas split domains (e.g., RuvC and HNH domains in the case of Cas9) can be simultaneously or sequentially introduced into the cell such that said split Cas domain(s) process the target nucleic acid sequence in the algae cell. The reduced size of the split Cas compared to the wild type Cas allows other methods of delivery of the systems to the cells, such as the use of cell penetrating peptides as described herein.

### DNA and RNA Base Editing

In some embodiments, a polynucleotide of the present invention described elsewhere herein can be modified using a base editing system. In some embodiments, a Cas protein is connected or fused to a nucleotide deaminase. Thus, in some embodiments the Cas-based system can be a base editing system. As used herein "base editing" refers generally to the process of polynucleotide modification via a CRISPR-Cas-based or Cas-based system that does not include excising nucleotides to make the modification. Base editing can convert base pairs at precise locations without generating excess undesired editing byproducts that can be made using traditional CRISPR-Cas systems.

In certain example embodiments, the nucleotide deaminase may be a DNA base editor used in combination with a DNA binding Cas protein such as, but not limited to, Class 2 Type II and Type V systems. Two classes of DNA base editors are generally known: cytosine base editors (CBEs) and adenine base editors (ABEs). CBEs convert a C•G base pair into a T•A base pair (Komor et al. 2016. Nature. 533:420-424; Nishida et al. 2016. Science. 353; and Li et al. Nat. Biotech. 36:324-327) and ABEs convert an A•T base pair to a G•C base pair. Collectively, CBEs and ABEs can mediate all four possible transition mutations (C to T, A to G, T to C, and G to A). Rees and Liu. 2018.Nat. Rev. Genet. 19(12): 770-788, particularly at Figures 1b, 2a-2c, 3a-3f, and Table 1. In some embodiments, the base editing system includes a CBE and/or an ABE. In some embodiments, a polynucleotide of the present invention described elsewhere herein can be modified using a base editing system. Rees and Liu. 2018. Nat. Rev. Gent. 19(12):770-788. Base editors also generally do not need a DNA donor template and/or rely on homology-directed repair. Komor et al. 2016. Nature. 533:420-424; Nishida et al. 2016. Science. 353; and Gaudeli et al. 2017. Nature. 551:464-471. Upon binding to a target locus in the DNA, base pairing between the guide RNA of the system and the target DNA strand leads to displacement of a small segment of ssDNA in an "R-loop". Nishimasu et al. Cell. 156:935-949. DNA bases within the ssDNA bubble are modified by the enzyme component, such as a deaminase. In some systems, the catalytically disabled Cas protein can be a variant or modified Cas can have nickase functionality and can generate a nick in the non-edited DNA strand to induce cells to repair the non-edited strand using the edited strand as a template. Komor et al. 2016. Nature. 533:420-424; Nishida et al. 2016. Science. 353; and Gaudeli et al. 2017. Nature. 551:464-471. Base editors may be further engineered to optimize conversion of nucleotides (e.g., A:T to G:C). Richter et al. 2020. Nature Biotechnology. doi.org/10.1038/s41587-020-0453-z.

Other Example Type V base editing systems are described in WO 2018/213708, WO 2018/213726, PCT/US2018/067207, PCT/US2018/067225, and PCT/US2018/067307.

In certain example embodiments, the base editing system may be a RNA base editing system. As with DNA base editors, a nucleotide deaminase capable of converting nucleotide bases may be fused to a Cas protein. However, in these embodiments, the Cas protein will need to be capable of binding RNA. Example RNA binding Cas proteins include, but are not limited to, RNA-binding Cas9s such as *Francisella novicida* Cas9 ("FnCas9"), and Class 2 Type VI Cas systems. The nucleotide deaminase may be a cytidine deaminase or an adenosine deaminase, or an adenosine deaminase engineered to have cytidine deaminase activity. In certain example embodiments, the RNA based editor may be used to delete or introduce a post-translation modification site in the expressed mRNA. In contrast to DNA base editors, whose edits are permanent in the modified cell, RNA base editors can provide edits where finer temporal control may be needed, for example in modulating a particular immune response. Example Type VI RNA-base editing systems are described in Cox et al. 2017. Science 358: 1019-1027, WO 2019/005884, WO 2019/005886, WO 2019/071048, PCT/US20018/05179, PCT/US2018/067207. An example FnCas9 system that may be adapted for RNA base editing purposes is described in WO 2016/106236.

An example method for delivery of base-editing systems, including use of a split-intein approach to divide CBE and ABE into reconstituble halves, is described in Levy et al. Nature Biomedical Engineering doi.org/10.1038/s41441-019-0505-5 (2019).

### Prime Editors

In some embodiments, a polynucleotide of the present invention described elsewhere herein can be modified using a prime editing system (See e.g., Anzalone et al. 2019. Nature. 576: 149-157). Like base editing systems, prime editing systems can be capable of targeted modification of a polynucleotide without generating double stranded breaks and does not require donor templates. Further prime editing systems can be capable of all 12 possible combination swaps. Prime editing can operate via a "search-and-replace" methodology and can mediate targeted insertions, deletions, all 12 possible base-to-base conversion, and combinations thereof. Generally, a prime editing system, as exemplified by PE1, PE2, and PE3 (*Id*.)*,* can include a reverse transcriptase fused or otherwise coupled or associated with an RNA-programmable nickase, and a prime-editing extended guide RNA (pegRNA) to facility direct copying of genetic information from the extension on the pegRNA into the target polynucleotide. Embodiments that can be used with the present invention include these and variants thereof. Prime editing can have the advantage of lower off-target activity than traditional CRIPSR-Cas systems along with few byproducts and greater or similar efficiency as compared to traditional CRISPR-Cas systems.

In some embodiments, the prime editing guide molecule can specify both the target polynucleotide information (e.g., sequence) and contain a new polynucleotide cargo that replaces target polynucleotides. To initiate transfer from the guide molecule to the target polynucleotide, the PE system can nick the target polynucleotide at a target side to expose a 3'hydroxyl group, which can prime reverse transcription of an edit-encoding extension region of the guide molecule (e.g., a prime editing guide molecule or peg guide molecule) directly into the target site in the target polynucleotide. See e.g., Anzalone et al. 2019. Nature. 576: 149-157, particularly at Figures 1b, 1c, related discussion, and Supplementary discussion.

In some embodiments, a prime editing system can be composed of a Cas polypeptide having nickase activity, a reverse transcriptase, and a guide molecule. The Cas polypeptide can lack nuclease activity. The guide molecule can include a target binding sequence as well as a primer binding sequence and a template containing the edited polynucleotide sequence. The guide molecule, Cas polypeptide, and/or reverse transcriptase can be coupled together or otherwise associate with each other to form an effector complex and edit a target sequence. In some embodiments, the Cas polypeptide is a Class 2, Type V Cas polypeptide. In some embodiments, the Cas polypeptide is a Cas9 polypeptide (e.g., is a Cas9 nickase). In some embodiments, the Cas polypeptide is fused to the reverse transcriptase. In some embodiments, the Cas polypeptide is linked to the reverse transcriptase.

In some embodiments, the prime editing system can be a PE1 system or variant thereof, a PE2 system or variant thereof, or a PE3 (e.g., PE3, PE3b) system. See e.g., Anzalone et al. 2019. Nature. 576: 149-157, particularly at pgs. 2-3, Figs. 2a, 3a-3f, 4a-4b, Extended data Figs. 3a-3b, 4,

The peg guide molecule can be about 10 to about 200 or more nucleotides in length, such as 10 to/or 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, or 200 or more nucleotides in length. Optimization of the peg guide molecule can be accomplished as described in Anzalone et al. 2019. Nature. 576: 149-157, particularly at pg. 3, Fig. 2a-2b, and Extended Data Figs. 5a-c.

### CRISPR Associated Transposase (CAST) Systems

In some embodiments, a polynucleotide of the present invention described elsewhere herein can be modified using a CRISPR Associated Transposase ("CAST") system. CAST system can include a Cas protein that is catalytically inactive, or engineered to be catalytically active, and further comprises a transposase (or subunits thereof) that catalyze RNA-guided DNA transposition. Such systems are able to insert DNA sequences at a target site in a DNA molecule without relying on host cell repair machinery. CAST systems can be Class1 or Class 2 CAST systems. An example Class 1 system is described in Klompe et al. Nature, doi: 10.1038/s41586-019-1323. An example Class 2 system is described in Strecker et al. Science. 10/1126/science. aax9181 (2019), and PCT/US2019/066835.

### Guide Molecules

The CRISPR-Cas or Cas-Based system described herein can, in some embodiments, include one or more guide molecules. The terms guide molecule, guide sequence and guide polynucleotide, refer to polynucleotides capable of guiding Cas to a target genomic locus and are used interchangeably as in foregoing cited documents such as WO 2014/093622 (PCT/US2013/074667). In general, a guide sequence is any polynucleotide sequence having sufficient complementarity with a target polynucleotide sequence to hybridize with the target sequence and direct sequence-specific binding of a CRISPR complex to the target sequence. The guide molecule can be a polynucleotide.

The ability of a guide sequence (within a nucleic acid-targeting guide RNA) to direct sequence-specific binding of a nucleic acid-targeting complex to a target nucleic acid sequence may be assessed by any suitable assay. For example, the components of a nucleic acid-targeting CRISPR system sufficient to form a nucleic acid-targeting complex, including the guide sequence to be tested, may be provided to a host cell having the corresponding target nucleic acid sequence, such as by transfection with vectors encoding the components of the nucleic acid-targeting complex, followed by an assessment of preferential targeting (e.g., cleavage) within the target nucleic acid sequence, such as by Surveyor assay (Qui et al. 2004. BioTechniques. 36(4)702-707). Similarly, cleavage of a target nucleic acid sequence may be evaluated in a test tube by providing the target nucleic acid sequence, components of a nucleic acid-targeting complex, including the guide sequence to be tested and a control guide sequence different from the test guide sequence, and comparing binding or rate of cleavage at the target sequence between the test and control guide sequence reactions. Other assays are possible and will occur to those skilled in the art.

In some embodiments, the guide molecule is an RNA. The guide molecule(s) (also referred to interchangeably herein as guide polynucleotide and guide sequence) that are included in the CRISPR-Cas or Cas based system can be any polynucleotide sequence having sufficient complementarity with a target nucleic acid sequence to hybridize with the target nucleic acid sequence and direct sequence-specific binding of a nucleic acid-targeting complex to the target nucleic acid sequence. In some embodiments, the degree of complementarity, when optimally aligned using a suitable alignment algorithm, can be about or more than about 50%, 60%, 75%, 80%, 85%, 90%, 95%, 97.5%, 99%, or more. Optimal alignment may be determined with the use of any suitable algorithm for aligning sequences, non-limiting examples of which include the Smith-Waterman algorithm, the Needleman-Wunsch algorithm, algorithms based on the Burrows-Wheeler Transform (e.g., the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and Maq (available at maq.sourceforge.net).

A guide sequence, and hence a nucleic acid-targeting guide may be selected to target any target nucleic acid sequence. The target sequence may be DNA. The target sequence may be any RNA sequence. In some embodiments, the target sequence may be a sequence within an RNA molecule selected from the group consisting of messenger RNA (mRNA), pre-mRNA, ribosomal RNA (rRNA), transfer RNA (tRNA), micro-RNA (miRNA), small interfering RNA (siRNA), small nuclear RNA (snRNA), small nucleolar RNA (snoRNA), double stranded RNA (dsRNA), non-coding RNA (ncRNA), long non-coding RNA (lncRNA), and small cytoplasmatic RNA (scRNA). In some preferred embodiments, the target sequence may be a sequence within an RNA molecule selected from the group consisting of mRNA, pre-mRNA, and rRNA. In some preferred embodiments, the target sequence may be a sequence within an RNA molecule selected from the group consisting of ncRNA, and lncRNA. In some more preferred embodiments, the target sequence may be a sequence within an mRNA molecule or a pre-mRNA molecule.

In some embodiments, a nucleic acid-targeting guide is selected to reduce the degree secondary structure within the nucleic acid-targeting guide. In some embodiments, about or less than about 75%, 50%, 40%, 30%, 25%, 20%, 15%, 10%, 5%, 1%, or fewer of the nucleotides of the nucleic acid-targeting guide participate in self-complementary base pairing when optimally folded. Optimal folding may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (*see* e.g., A.R. Gruber et al., 2008, Cell 106(1): 23-24; and PA Carr and GM Church, 2009, Nature Biotechnology 27(12): 1151-62).

In certain embodiments, a guide RNA or crRNA may comprise, consist essentially of, or consist of a direct repeat (DR) sequence and a guide sequence or spacer sequence. In certain embodiments, the guide RNA or crRNA may comprise, consist essentially of, or consist of a direct repeat sequence fused or linked to a guide sequence or spacer sequence. In certain embodiments, the direct repeat sequence may be located upstream (i.e., 5') from the guide sequence or spacer sequence. In other embodiments, the direct repeat sequence may be located downstream (i.e., 3') from the guide sequence or spacer sequence.

In certain embodiments, the crRNA comprises a stem loop, preferably a single stem loop. In certain embodiments, the direct repeat sequence forms a stem loop, preferably a single stem loop.

In certain embodiments, the spacer length of the guide RNA is from 15 to 35 nt. In certain embodiments, the spacer length of the guide RNA is at least 15 nucleotides. In certain embodiments, the spacer length is from 15 to 17 nt, e.g., 15, 16, or 17 nt, from 17 to 20 nt, e.g., 17, 18, 19, or 20 nt, from 20 to 24 nt, e.g., 20, 21, 22, 23, or 24 nt, from 23 to 25 nt, e.g., 23, 24, or 25 nt, from 24 to 27 nt, e.g., 24, 25, 26, or 27 nt, from 27 to 30 nt, e.g., 27, 28, 29, or 30 nt, from 30 to 35 nt, e.g., 30, 31, 32, 33, 34, or 35 nt, or 35 nt or longer.

The "tracrRNA" sequence or analogous terms includes any polynucleotide sequence that has sufficient complementarity with a crRNA sequence to hybridize. In some embodiments, the degree of complementarity between the tracrRNA sequence and crRNA sequence along the length of the shorter of the two when optimally aligned is about or more than about 25%, 30%, 40%, 50%, 60%, 70%, 80%, 90%, 95%, 97.5%, 99%, or higher. In some embodiments, the tracr sequence is about or more than about 5, 6, 7, 8, 9, 10, 11, 12, 13, 14, 15, 16, 17, 18, 19, 20, 25, 30, 40, 50, or more nucleotides in length. In some embodiments, the tracr sequence and crRNA sequence are contained within a single transcript, such that hybridization between the two produces a transcript having a secondary structure, such as a hairpin.

In general, degree of complementarity is with reference to the optimal alignment of the sca sequence and tracr sequence, along the length of the shorter of the two sequences. Optimal alignment may be determined by any suitable alignment algorithm, and may further account for secondary structures, such as self-complementarity within either the sca sequence or tracr sequence. In some embodiments, the degree of complementarity between the tracr sequence and sca sequence along the length of the shorter of the two when optimally aligned is about or more than about 25%, 30%, 40%, 50%, 60%, 70%, 80%, 90%, 95%, 97.5%, 99%, or higher.

In some embodiments, the degree of complementarity between a guide sequence and its corresponding target sequence can be about or more than about 50%, 60%, 75%, 80%, 85%, 90%, 95%, 97.5%, 99%, or 100%; a guide or RNA or sgRNA can be about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length; or guide or RNA or sgRNA can be less than about 75, 50, 45, 40, 35, 30, 25, 20, 15, 12, or fewer nucleotides in length; and tracr RNA can be 30 or 50 nucleotides in length. In some embodiments, the degree of complementarity between a guide sequence and its corresponding target sequence is greater than 94.5% or 95% or 95.5% or 96% or 96.5% or 97% or 97.5% or 98% or 98.5% or 99% or 99.5% or 99.9%, or 100%. Off target is less than 100% or 99.9% or 99.5% or 99% or 99% or 98.5% or 98% or 97.5% or 97% or 96.5% or 96% or 95.5% or 95% or 94.5% or 94% or 93% or 92% or 91% or 90% or 89% or 88% or 87% or 86% or 85% or 84% or 83% or 82% or 81% or 80% complementarity between the sequence and the guide, with it advantageous that off target is 100% or 99.9% or 99.5% or 99% or 99% or 98.5% or 98% or 97.5% or 97% or 96.5% or 96% or 95.5% or 95% or 94.5% complementarity between the sequence and the guide.

In some embodiments according to the invention, the guide RNA (capable of guiding Cas to a target locus) may comprise (1) a guide sequence capable of hybridizing to a genomic target locus in the eukaryotic cell; (2) a tracr sequence; and (3) a tracr mate sequence. All (1) to (3) may reside in a single RNA, i.e., an sgRNA (arranged in a 5' to 3' orientation), or the tracr RNA may be a different RNA than the RNA containing the guide and tracr sequence. The tracr hybridizes to the tracr mate sequence and directs the CRISPR/Cas complex to the target sequence. Where the tracr RNA is on a different RNA than the RNA containing the guide and tracr sequence, the length of each RNA may be optimized to be shortened from their respective native lengths, and each may be independently chemically modified to protect from degradation by cellular RNase or otherwise increase stability.

Many modifications to guide sequences are known in the art and are further contemplated within the context of this invention. Various modifications may be used to increase the specificity of binding to the target sequence and/or increase the activity of the Cas protein and/or reduce off-target effects. Example guide sequence modifications are described in PCT US2019/045582, specifically paragraphs [0178]-[0333].

### Target Sequences, PAMs, and PFSs

### Target Sequences

In the context of formation of a CRISPR complex, "target sequence" refers to a sequence to which a guide sequence is designed to have complementarity, where hybridization between a target sequence and a guide sequence promotes the formation of a CRISPR complex. A target sequence may comprise RNA polynucleotides. The term "target RNA" refers to an RNA polynucleotide being or comprising the target sequence. In other words, the target polynucleotide can be a polynucleotide or a part of a polynucleotide to which a part of the guide sequence is designed to have complementarity with and to which the effector function mediated by the complex comprising the CRISPR effector protein and a guide molecule is to be directed. In some embodiments, a target sequence is located in the nucleus or cytoplasm of a cell.

The guide sequence can specifically bind a target sequence in a target polynucleotide. The target polynucleotide may be DNA. The target polynucleotide may be RNA. The target polynucleotide can have one or more (e.g., 1, 2, 3, 4, 5, 6, 7, 8, 9, 10, etc. or more) target sequences. The target polynucleotide can be on a vector. The target polynucleotide can be genomic DNA. The target polynucleotide can be episomal. Other forms of the target polynucleotide are described elsewhere herein.

The target sequence may be DNA. The target sequence may be any RNA sequence. In some embodiments, the target sequence may be a sequence within an RNA molecule selected from the group consisting of messenger RNA (mRNA), pre-mRNA, ribosomal RNA (rRNA), transfer RNA (tRNA), micro-RNA (miRNA), small interfering RNA (siRNA), small nuclear RNA (snRNA), small nucleolar RNA (snoRNA), double stranded RNA (dsRNA), non-coding RNA (ncRNA), long non-coding RNA (lncRNA), and small cytoplasmatic RNA (scRNA). In some preferred embodiments, the target sequence (also referred to herein as a target polynucleotide) may be a sequence within an RNA molecule selected from the group consisting of mRNA, pre-mRNA, and rRNA. In some preferred embodiments, the target sequence may be a sequence within an RNA molecule selected from the group consisting of ncRNA, and lncRNA. In some more preferred embodiments, the target sequence may be a sequence within an mRNA molecule or a pre-mRNA molecule.

### PAM and PFS Elements

PAM elements are sequences that can be recognized and bound by Cas proteins. Cas proteins/effector complexes can then unwind the dsDNA at a position adjacent to the PAM element. It will be appreciated that Cas proteins and systems that include them that target RNA do not require PAM sequences (Marraffini et al. 2010. Nature. 463:568-571). Instead, many rely on PFSs, which are discussed elsewhere herein. In certain embodiments, the target sequence should be associated with a PAM (protospacer adjacent motif) or PFS (protospacer flanking sequence or site), that is, a short sequence recognized by the CRISPR complex. Depending on the nature of the CRISPR-Cas protein, the target sequence should be selected, such that its complementary sequence in the DNA duplex (also referred to herein as the non-target sequence) is upstream or downstream of the PAM. In the embodiments, the complementary sequence of the target sequence is downstream or 3' of the PAM or upstream or 5' of the PAM. The precise sequence and length requirements for the PAM differ depending on the Cas protein used, but PAMs are typically 2-5 base pair sequences adjacent the protospacer (that is, the target sequence). Examples of the natural PAM sequences for different Cas proteins are provided herein below and the skilled person will be able to identify further PAM sequences for use with a given Cas protein.

The ability to recognize different PAM sequences depends on the Cas polypeptide(s) included in the system. *See* e.g., Gleditzsch et al. 2019. RNA Biology. 16(4):504-517. **Table 3** below shows several Cas polypeptides and the PAM sequence they recognize.

| **Table 3 - Example PAM Sequences** | |
|---|---|
| Cas Protein | PAM Sequence |
| SpCas9 | NGG/NRG |
| SaCas9 | NGRRT or NGRRN |
| NmeCas9 | NNNNGATT |
| CjCas9 | NNNNRYAC |
| StCas9 | NNAGAAW |
| Cas12a (Cpf1) (including LbCpf1 and AsCpf1) | TTTV |
| Cas12b (C2c1) | TTT, TTA, and TTC |
| Cas12c (C2c3) | TA |
| Cas12d (CasY) | TA |
| Cas12e (CasX) | 5'-TTCN-3' |

In a preferred embodiment, the CRISPR effector protein may recognize a 3' PAM. In certain embodiments, the CRISPR effector protein may recognize a 3' PAM which is 5'H, wherein H is A, C or U.

Further, engineering of the PAM Interacting (PI) domain on the Cas protein may allow programing of PAM specificity, improve target site recognition fidelity, and increase the versatility of the CRISPR-Cas protein, for example as described for Cas9 in Kleinstiver BP et al. Engineered CRISPR-Cas9 nucleases with altered PAM specificities. Nature. 2015 Jul 23;523(7561):481-5. doi: 10.1038/nature14592. As further detailed herein, the skilled person will understand that Cas13 proteins may be modified analogously. Gao et al, "Engineered Cpf1 Enzymes with Altered PAM Specificities," bioRxiv 091611; doi: http://dx.doi.org/10.1101/091611 (Dec. 4, 2016). Doench et al. created a pool of sgRNAs, tiling across all possible target sites of a panel of six endogenous mouse and three endogenous human genes and quantitatively assessed their ability to produce null alleles of their target gene by antibody staining and flow cytometry. The authors showed that optimization of the PAM improved activity and also provided an on-line tool for designing sgRNAs.

PAM sequences can be identified in a polynucleotide using an appropriate design tool, which are commercially available as well as online. Such freely available tools include, but are not limited to, CRISPRFinder and CRISPRTarget. Mojica et al. 2009. Microbiol. 155(Pt. 3):733-740; Atschul et al. 1990. J. Mol. Biol. 215:403-410; Biswass et al. 2013 RNA Biol. 10:817-827; and Grissa et al. 2007. Nucleic Acid Res. 35:W52-57. Experimental approaches to PAM identification can include, but are not limited to, plasmid depletion assays (Jiang et al. 2013. Nat. Biotechnol. 31:233-239; Esvelt et al. 2013. Nat. Methods. 10:1116-1121; Kleinstiver et al. 2015. Nature. 523:481-485), screened by a high-throughput *in vivo* model called PAM-SCNAR (Pattanayak et al. 2013. Nat. Biotechnol. 31:839-843 and Leenay et al. 2016.Mol. Cell. 16:253), and negative screening (Zetsche et al. 2015. Cell. 163:759-771).

As previously mentioned, CRISPR-Cas systems that target RNA do not typically rely on PAM sequences. Instead, such systems typically recognize protospacer flanking sites (PFSs) instead of PAMs Thus, Type VI CRISPR-Cas systems typically recognize protospacer flanking sites (PFSs) instead of PAMs. PFSs represents an analogue to PAMs for RNA targets. Type VI CRISPR-Cas systems employ a Cas13. Some Cas13 proteins analyzed to date, such as Cas13a (C2c2) identified from Leptotrichia shahii (LShCAs13a) have a specific discrimination against G at the 3'end of the target RNA. The presence of a C at the corresponding crRNA repeat site can indicate that nucleotide pairing at this position is rejected. However, some Cas13 proteins (e.g., LwaCAs13a and PspCas13b) do not seem to have a PFS preference. See e.g., Gleditzsch et al. 2019. RNA Biology. 16(4):504-517.

Some Type VI proteins, such as subtype B, have 5'-recognition of D (G, T, A) and a 3'-motif requirement of NAN or NNA. One example is the Cas13b protein identified in Bergeyella zoohelcum (BzCas13b). See e.g., Gleditzsch et al. 2019. RNA Biology. 16(4):504-517.

Overall Type VI CRISPR-Cas systems appear to have less restrictive rules for substrate (e.g., target sequence) recognition than those that target DNA (e.g., Type V and type II).

### Zinc Finger Nucleases

In some embodiments, the polynucleotide is modified using a Zinc Finger nuclease or system thereof. One type of programmable DNA-binding domain is provided by artificial zinc-finger (ZF) technology, which involves arrays of ZF modules to target new DNA-binding sites in the genome. Each finger module in a ZF array targets three DNA bases. A customized array of individual zinc finger domains is assembled into a ZF protein (ZFP).

ZFPs can comprise a functional domain. The first synthetic zinc finger nucleases (ZFNs) were developed by fusing a ZF protein to the catalytic domain of the Type IIS restriction enzyme FokI. (Kim, Y. G. et al., 1994, Chimeric restriction endonuclease, Proc. Natl. Acad. Sci. U.S.A. 91, 883-887; Kim, Y. G. et al., 1996, Hybrid restriction enzymes: zinc finger fusions to Fok I cleavage domain. Proc. Natl. Acad. Sci. U.S.A. 93, 1156-1160). Increased cleavage specificity can be attained with decreased off target activity by use of paired ZFN heterodimers, each targeting different nucleotide sequences separated by a short spacer. (Doyon, Y. et al., 2011, Enhancing zinc-finger-nuclease activity with improved obligate heterodimeric architectures. Nat. Methods 8, 74-79). ZFPs can also be designed as transcription activators and repressors and have been used to target many genes in a wide variety of organisms. Exemplary methods of genome editing using ZFNs can be found for example in U.S. Patent Nos. 6,534,261, 6,607,882, 6,746,838, 6,794,136, 6,824,978, 6,866,997, 6,933,113, 6,979,539, 7,013,219, 7,030,215, 7,220,719, 7,241,573, 7,241,574, 7,585,849, 7,595,376, 6,903,185, and 6,479,626.

### TALE Nucleases

In some embodiments, a TALE nuclease or TALE nuclease system can be used to modify a polynucleotide. In some embodiments, the methods provided herein use isolated, non-naturally occurring, recombinant or engineered DNA binding proteins that comprise TALE monomers or TALE monomers or half monomers as a part of their organizational structure that enable the targeting of nucleic acid sequences with improved efficiency and expanded specificity.

Naturally occurring TALEs or "wild type TALEs" are nucleic acid binding proteins secreted by numerous species of proteobacteria. TALE polypeptides contain a nucleic acid binding domain composed of tandem repeats of highly conserved monomer polypeptides that are predominantly 33, 34 or 35 amino acids in length and that differ from each other mainly in amino acid positions 12 and 13. In advantageous embodiments the nucleic acid is DNA. As used herein, the term "polypeptide monomers", "TALE monomers" or "monomers" will be used to refer to the highly conserved repetitive polypeptide sequences within the TALE nucleic acid binding domain and the term "repeat variable di-residues" or "RVD" will be used to refer to the highly variable amino acids at positions 12 and 13 of the polypeptide monomers. As provided throughout the disclosure, the amino acid residues of the RVD are depicted using the IUPAC single letter code for amino acids. A general representation of a TALE monomer which is comprised within the DNA binding domain is X₁₋₁₁-(X₁₂X₁₃)-X₁₄₋₃₃ or 34 or 35, where the subscript indicates the amino acid position and X represents any amino acid. X₁₂X₁₃ indicate the RVDs. In some polypeptide monomers, the variable amino acid at position 13 is missing or absent and in such monomers, the RVD consists of a single amino acid. In such cases the RVD may be alternatively represented as X*, where X represents X₁₂ and (*) indicates that X₁₃ is absent. The DNA binding domain comprises several repeats of TALE monomers and this may be represented as (X₁₋₁₁-(X₁₂X₁₃)-X₁₄₋₃₃ or 34 or 35)_{z}, where in an advantageous embodiment, z is at least 5 to 40. In a further advantageous embodiment, z is at least 10 to 26.

The TALE monomers can have a nucleotide binding affinity that is determined by the identity of the amino acids in its RVD. For example, polypeptide monomers with an RVD of NI can preferentially bind to adenine (A), monomers with an RVD of NG can preferentially bind to thymine (T), monomers with an RVD of HD can preferentially bind to cytosine (C) and monomers with an RVD of NN can preferentially bind to both adenine (A) and guanine (G). In some embodiments, monomers with an RVD of IG can preferentially bind to T. Thus, the number and order of the polypeptide monomer repeats in the nucleic acid binding domain of a TALE determines its nucleic acid target specificity. In some embodiments, monomers with an RVD of NS can recognize all four base pairs and can bind to A, T, G or C. The structure and function of TALEs is further described in, for example, Moscou et al., Science 326:1501 (2009); Boch et al., Science 326:1509-1512 (2009); and Zhang et al., Nature Biotechnology 29:149-153 (2011).

The polypeptides used in methods of the invention can be isolated, non-naturally occurring, recombinant or engineered nucleic acid-binding proteins that have nucleic acid or DNA binding regions containing polypeptide monomer repeats that are designed to target specific nucleic acid sequences.

As described herein, polypeptide monomers having an RVD of HN or NH preferentially bind to guanine and thereby allow the generation of TALE polypeptides with high binding specificity for guanine containing target nucleic acid sequences. In some embodiments, polypeptide monomers having RVDs RN, NN, NK, SN, NH, KN, HN, NQ, HH, RG, KH, RH and SS can preferentially bind to guanine. In some embodiments, polypeptide monomers having RVDs RN, NK, NQ, HH, KH, RH, SS and SN can preferentially bind to guanine and can thus allow the generation of TALE polypeptides with high binding specificity for guanine containing target nucleic acid sequences. In some embodiments, polypeptide monomers having RVDs HH, KH, NH, NK, NQ, RH, RN and SS can preferentially bind to guanine and thereby allow the generation of TALE polypeptides with high binding specificity for guanine containing target nucleic acid sequences. In some embodiments, the RVDs that have high binding specificity for guanine are RN, NH RH and KH. Furthermore, polypeptide monomers having an RVD of NV can preferentially bind to adenine and guanine. In some embodiments, monomers having RVDs of H*, HA, KA, N*, NA, NC, NS, RA, and S* bind to adenine, guanine, cytosine and thymine with comparable affinity.

The predetermined N-terminal to C-terminal order of the one or more polypeptide monomers of the nucleic acid or DNA binding domain determines the corresponding predetermined target nucleic acid sequence to which the polypeptides of the invention will bind. As used herein the monomers and at least one or more half monomers are "specifically ordered to target" the genomic locus or gene of interest. In plant genomes, the natural TALE-binding sites always begin with a thymine (T), which may be specified by a cryptic signal within the non-repetitive N-terminus of the TALE polypeptide; in some cases, this region may be referred to as repeat 0. In animal genomes, TALE binding sites do not necessarily have to begin with a thymine (T) and polypeptides of the invention may target DNA sequences that begin with T, A, G or C. The tandem repeat of TALE monomers always ends with a half-length repeat or a stretch of sequence that may share identity with only the first 20 amino acids of a repetitive full-length TALE monomer and this half repeat may be referred to as a half-monomer. Therefore, it follows that the length of the nucleic acid or DNA being targeted is equal to the number of full monomers plus two.

As described in Zhang et al., Nature Biotechnology 29:149-153 (2011), TALE polypeptide binding efficiency may be increased by including amino acid sequences from the "capping regions" that are directly N-terminal or C-terminal of the DNA binding region of naturally occurring TALEs into the engineered TALEs at positions N-terminal or C-terminal of the engineered TALE DNA binding region. Thus, in certain embodiments, the TALE polypeptides described herein further comprise an N-terminal capping region and/or a C-terminal capping region.

An exemplary amino acid sequence of a N-terminal capping region is:

M D P I R S R T P S P A R E L L S G P Q P D G V Q P T A D R G V S P P A G
G P L D G L P A R R T M S R T R L P S P P A P S P A F S A D S F S D L L R Q F D P S L
F N T S L F D S L P P F G A H H T E A A T G E W D E V Q S G L R A A D A P P P T M
R V A V T A A R P P R A K P A P R R R A A Q P S D A S P A A Q V D L R T L G Y S Q
Q Q Q E K I K P K V R S T V A Q H H E A L V G H G F T H A H I V A L S Q H P A A L
G T V A V K Y Q D M I A A L P E A T H E A I V G V G K Q W S G A R A L E A L L T V
A G E L R G P P L Q L D T G Q L L K I A K R G G V T A V E A V H A W R N A L T G A
P L N (SEQ ID NO: 3)

An exemplary amino acid sequence of a C-terminal capping region is:

R P A L E S I V A Q L S R P D P A L A A L T N D H L V A L A C L G G R P A
L D A V K K G L P H A P A L I K R T N R R I P E R T S H R V A D H A Q V V R V L G
F F Q C H S H P A Q A F D D A M T Q F G M S R H G L L Q L F R R V G V T E L E A R
S G T L P P A S Q R W D R I L Q A S G M K R A K P S P T S T Q T P D Q A S L H A F A
D S L E R D L D A P S P M H E G D Q T R A S (SEQ ID NO: 4)

As used herein the predetermined "N-terminus" to "C terminus" orientation of the N-terminal capping region, the DNA binding domain comprising the repeat TALE monomers and the C-terminal capping region provide structural basis for the organization of different domains in the d-TALEs or polypeptides of the invention.

The entire N-terminal and/or C-terminal capping regions are not necessary to enhance the binding activity of the DNA binding region. Therefore, in certain embodiments, fragments of the N-terminal and/or C-terminal capping regions are included in the TALE polypeptides described herein.

In certain embodiments, the TALE polypeptides described herein contain a N-terminal capping region fragment that included at least 10, 20, 30, 40, 50, 54, 60, 70, 80, 87, 90, 94, 100, 102, 110, 117, 120, 130, 140, 147, 150, 160, 170, 180, 190, 200, 210, 220, 230, 240, 250, 260 or 270 amino acids of an N-terminal capping region. In certain embodiments, the N-terminal capping region fragment amino acids are of the C-terminus (the DNA-binding region proximal end) of an N-terminal capping region. As described in Zhang et al., Nature Biotechnology 29:149-153 (2011), N-terminal capping region fragments that include the C-terminal 240 amino acids enhance binding activity equal to the full length capping region, while fragments that include the C-terminal 147 amino acids retain greater than 80% of the efficacy of the full length capping region, and fragments that include the C-terminal 117 amino acids retain greater than 50% of the activity of the full-length capping region.

In some embodiments, the TALE polypeptides described herein contain a C-terminal capping region fragment that included at least 6, 10, 20, 30, 37, 40, 50, 60, 68, 70, 80, 90, 100, 110, 120, 127, 130, 140, 150, 155, 160, 170, 180 amino acids of a C-terminal capping region. In certain embodiments, the C-terminal capping region fragment amino acids are of the N-terminus (the DNA-binding region proximal end) of a C-terminal capping region. As described in Zhang et al., Nature Biotechnology 29:149-153 (2011), C-terminal capping region fragments that include the C-terminal 68 amino acids enhance binding activity equal to the full-length capping region, while fragments that include the C-terminal 20 amino acids retain greater than 50% of the efficacy of the full-length capping region.

In certain embodiments, the capping regions of the TALE polypeptides described herein do not need to have identical sequences to the capping region sequences provided herein. Thus, in some embodiments, the capping region of the TALE polypeptides described herein have sequences that are at least 50%, 60%, 70%, 80%, 85%, 90%, 91%, 92%, 93%, 94%, 95%, 96%, 97%, 98% or 99% identical or share identity to the capping region amino acid sequences provided herein. Sequence identity is related to sequence homology. Homology comparisons may be conducted by eye, or more usually, with the aid of readily available sequence comparison programs. These commercially available computer programs may calculate percent (%) homology between two or more sequences and may also calculate the sequence identity shared by two or more amino acid or nucleic acid sequences. In some preferred embodiments, the capping region of the TALE polypeptides described herein have sequences that are at least 95% identical or share identity to the capping region amino acid sequences provided herein.

Sequence homologies can be generated by any of a number of computer programs known in the art, which include but are not limited to BLAST or FASTA. Suitable computer programs for carrying out alignments like the GCG Wisconsin Bestfit package may also be used. Once the software has produced an optimal alignment, it is possible to calculate % homology, preferably % sequence identity. The software typically does this as part of the sequence comparison and generates a numerical result.

In some embodiments described herein, the TALE polypeptides of the invention include a nucleic acid binding domain linked to the one or more effector domains. The terms "effector domain" or "regulatory and functional domain" refer to a polypeptide sequence that has an activity other than binding to the nucleic acid sequence recognized by the nucleic acid binding domain. By combining a nucleic acid binding domain with one or more effector domains, the polypeptides of the invention may be used to target the one or more functions or activities mediated by the effector domain to a particular target DNA sequence to which the nucleic acid binding domain specifically binds.

In some embodiments of the TALE polypeptides described herein, the activity mediated by the effector domain is a biological activity. For example, in some embodiments the effector domain is a transcriptional inhibitor (i.e., a repressor domain), such as an mSin interaction domain (SID). SID4X domain or a Krüppel-associated box (KRAB) or fragments of the KRAB domain. In some embodiments the effector domain is an enhancer of transcription (i.e., an activation domain), such as the VP16, VP64 or p65 activation domain. In some embodiments, the nucleic acid binding is linked, for example, with an effector domain that includes but is not limited to a transposase, integrase, recombinase, resolvase, invertase, protease, DNA methyltransferase, DNA demethylase, histone acetylase, histone deacetylase, nuclease, transcriptional repressor, transcriptional activator, transcription factor recruiting, protein nuclear-localization signal or cellular uptake signal.

In some embodiments, the effector domain is a protein domain which exhibits activities which include but are not limited to transposase activity, integrase activity, recombinase activity, resolvase activity, invertase activity, protease activity, DNA methyltransferase activity, DNA demethylase activity, histone acetylase activity, histone deacetylase activity, nuclease activity, nuclear-localization signaling activity, transcriptional repressor activity, transcriptional activator activity, transcription factor recruiting activity, or cellular uptake signaling activity.

### Meganucleases

In some embodiments, a meganuclease or system thereof can be used to modify a polynucleotide. Meganucleases, which are endodeoxyribonucleases characterized by a large recognition site (double-stranded DNA sequences of 12 to 40 base pairs). Exemplary methods for using meganucleases can be found in US Patent Nos. 8,163,514, 8,133,697, 8,021,867, 8,119,361, 8,119,381, 8,124,369, and 8,129,134.

### SEQUENCES RELATED TO NUCLEUS TARGETING AND TRANSPORTATION

In some embodiments, one or more components (e.g., the Cas protein and/or deaminase, Zn Finger protein, TALE, or meganuclease) in the composition for engineering cells may comprise one or more sequences related to nucleus targeting and transportation. Such sequence may facilitate the one or more components in the composition for targeting a sequence within a cell. In order to improve targeting of the CRISPR-Cas protein and/or the nucleotide deaminase protein or catalytic domain thereof used in the methods of the present disclosure to the nucleus, it may be advantageous to provide one or both of these components with one or more nuclear localization sequences (NLSs).

In some embodiments, the NLSs used in the context of the present disclosure are heterologous to the proteins. Non-limiting examples of NLSs include an NLS sequence derived from: the NLS of the SV40 virus large T-antigen, having the amino acid sequence PKKKRKV (SEQ ID NO: 5) or PKKKRKVEAS (SEQ ID NO: 6); the NLS from nucleoplasmin (e.g., the nucleoplasmin bipartite NLS with the sequence KRPAATKKAGQAKKKK (SEQ ID NO: 7)); the c-myc NLS having the amino acid sequence PAAKRVKLD (SEQ ID NO: 8) or RQRRNELKRSP (SEQ ID NO: 9); the hRNPA1 M9 NLS having the sequence NQSSNFGPMKGGNFGGRSSGPYGGGGQYFAKPRNQGGY (SEQ ID NO: 10); the sequence RMRIZFKNKGKDTAELRRRRVEVSVELRKAKKDEQILKRRNV (SEQ ID NO: 11) of the IBB domain from importin-alpha; the sequences VSRKRPRP (SEQ ID NO: 12) and PPKKARED (SEQ ID NO: 13) of the myoma T protein; the sequence PQPKKKPL (SEQ ID NO: 14) of human p53; the sequence SALIKKKKKMAP (SEQ ID NO: 15) of mouse c-abl IV; the sequences DRLRR (SEQ ID NO: 16) and PKQKKRK (SEQ ID NO: 17) of the influenza virus NS1; the sequence RKLKKKIKKL (SEQ ID NO: 18) of the Hepatitis virus delta antigen; the sequence REKKKFLKRR (SEQ ID NO: 19) of the mouse Mx1 protein; the sequence KRKGDEVDGVDEVAKKKSKK (SEQ ID NO: 20) of the human poly(ADP-ribose) polymerase; and the sequence RKCLQAGMNLEARKTKK (SEQ ID NO: 21) of the steroid hormone receptors (human) glucocorticoid. In general, the one or more NLSs are of sufficient strength to drive accumulation of the DNA-targeting Cas protein in a detectable amount in the nucleus of a eukaryotic cell. In general, strength of nuclear localization activity may derive from the number of NLSs in the CRISPR-Cas protein, the particular NLS(s) used, or a combination of these factors. Detection of accumulation in the nucleus may be performed by any suitable technique. For example, a detectable marker may be fused to the nucleic acid-targeting protein, such that location within a cell may be visualized, such as in combination with a means for detecting the location of the nucleus (e.g., a stain specific for the nucleus such as DAPI). Cell nuclei may also be isolated from cells, the contents of which may then be analyzed by any suitable process for detecting protein, such as immunohistochemistry, Western blot, or enzyme activity assay. Accumulation in the nucleus may also be determined indirectly, such as by an assay for the effect of nucleic acid-targeting complex formation (e.g., assay for deaminase activity) at the target sequence, or assay for altered gene expression activity affected by DNA-targeting complex formation and/or DNA-targeting), as compared to a control not exposed to the CRISPR-Cas protein and deaminase protein, or exposed to a CRISPR-Cas and/or deaminase protein lacking the one or more NLSs.

The CRISPR-Cas and/or nucleotide deaminase proteins may be provided with 1 or more, such as with, 2, 3, 4, 5, 6, 7, 8, 9, 10, or more heterologous NLSs. In some embodiments, the proteins comprises about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g., zero or at least one or more NLS at the amino-terminus and zero or at one or more NLS at the carboxy terminus). When more than one NLS is present, each may be selected independently of the others, such that a single NLS may be present in more than one copy and/or in combination with one or more other NLSs present in one or more copies. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the N- or C-terminus. In preferred embodiments of the CRISPR-Cas proteins, an NLS attached to the C-terminal of the protein.

In certain embodiments, the CRISPR-Cas protein and the deaminase protein are delivered to the cell or expressed within the cell as separate proteins. In these embodiments, each of the CRISPR-Cas and deaminase protein can be provided with one or more NLSs as described herein. In certain embodiments, the CRISPR-Cas and deaminase proteins are delivered to the cell or expressed with the cell as a fusion protein. In these embodiments one or both of the CRISPR-Cas and deaminase protein is provided with one or more NLSs. Where the nucleotide deaminase is fused to an adaptor protein (such as MS2) as described above, the one or more NLS can be provided on the adaptor protein, provided that this does not interfere with aptamer binding. In particular embodiments, the one or more NLS sequences may also function as linker sequences between the nucleotide deaminase and the CRISPR-Cas protein.

In certain embodiments, guides of the disclosure comprise specific binding sites (e.g. aptamers) for adapter proteins, which may be linked to or fused to an nucleotide deaminase or catalytic domain thereof. When such a guide forms a CRISPR complex (e.g., CRISPR-Cas protein binding to guide and target) the adapter proteins bind and, the nucleotide deaminase or catalytic domain thereof associated with the adapter protein is positioned in a spatial orientation which is advantageous for the attributed function to be effective.

The skilled person will understand that modifications to the guide which allow for binding of the adapter + nucleotide deaminase, but not proper positioning of the adapter + nucleotide deaminase (e.g., due to steric hindrance within the three dimensional structure of the CRISPR complex) are modifications which are not intended. The one or more modified guide may be modified at the tetra loop, the stem loop 1, stem loop 2, or stem loop 3, as described herein, preferably at either the tetra loop or stem loop 2, and in some cases at both the tetra loop and stem loop 2.

In some embodiments, a component (e.g., the dead Cas protein, the nucleotide deaminase protein or catalytic domain thereof, or a combination thereof) in the systems may comprise one or more nuclear export signals (NES), one or more nuclear localization signals (NLS), or any combinations thereof. In some cases, the NES may be an HIV Rev NES. In certain cases, the NES may be MAPK NES. When the component is a protein, the NES or NLS may be at the C terminus of component. Alternatively, or additionally, the NES or NLS may be at the N terminus of component. In some examples, the Cas protein and optionally said nucleotide deaminase protein or catalytic domain thereof comprise one or more heterologous nuclear export signal(s) (NES(s)) or nuclear localization signal(s) (NLS(s)), preferably an HIV Rev NES or MAPK NES, preferably C-terminal.

### Templates

In some embodiments, the composition for engineering cells comprise a template, e.g., a recombination template. A template may be a component of another vector as described herein, contained in a separate vector, or provided as a separate polynucleotide. In some embodiments, a recombination template is designed to serve as a template in homologous recombination, such as within or near a target sequence nicked or cleaved by a nucleic acid-targeting effector protein as a part of a nucleic acid-targeting complex.

In an embodiment, the template nucleic acid alters the sequence of the target position. In an embodiment, the template nucleic acid results in the incorporation of a modified, or non-naturally occurring base into the target nucleic acid.

The template sequence may undergo a breakage mediated or catalyzed recombination with the target sequence. In an embodiment, the template nucleic acid may include sequence that corresponds to a site on the target sequence that is cleaved by a Cas protein mediated cleavage event. In an embodiment, the template nucleic acid may include sequence that corresponds to both, a first site on the target sequence that is cleaved in a first Cas protein mediated event, and a second site on the target sequence that is cleaved in a second Cas protein mediated event.

In certain embodiments, the template nucleic acid can include sequence which results in an alteration in the coding sequence of a translated sequence, e.g., one which results in the substitution of one amino acid for another in a protein product, e.g., transforming a mutant allele into a wild type allele, transforming a wild type allele into a mutant allele, and/or introducing a stop codon, insertion of an amino acid residue, deletion of an amino acid residue, or a nonsense mutation. In certain embodiments, the template nucleic acid can include sequence which results in an alteration in a non-coding sequence, e.g., an alteration in an exon or in a 5' or 3' non-translated or non-transcribed region. Such alterations include an alteration in a control element, e.g., a promoter, enhancer, and an alteration in a cis-acting or trans-acting control element.

A template nucleic acid having homology with a target position in a target gene may be used to alter the structure of a target sequence. The template sequence may be used to alter an unwanted structure, e.g., an unwanted or mutant nucleotide. The template nucleic acid may include sequence which, when integrated, results in: decreasing the activity of a positive control element; increasing the activity of a positive control element; decreasing the activity of a negative control element; increasing the activity of a negative control element; decreasing the expression of a gene; increasing the expression of a gene; increasing resistance to a disorder or disease; increasing resistance to viral entry; correcting a mutation or altering an unwanted amino acid residue conferring, increasing, abolishing or decreasing a biological property of a gene product, e.g., increasing the enzymatic activity of an enzyme, or increasing the ability of a gene product to interact with another molecule.

The template nucleic acid may include sequence which results in: a change in sequence of 1, 2, 3, 4, 5, 6, 7, 8, 9, 10, 1 1, 12 or more nucleotides of the target sequence.

A template polynucleotide may be of any suitable length, such as about or more than about 10, 15, 20, 25, 50, 75, 100, 150, 200, 500, 1000, or more nucleotides in length. In an embodiment, the template nucleic acid may be 20+/- 10, 30+/- 10, 40+/- 10, 50+/- 10, 60+/- 10, 70+/- 10, 80+/- 10, 90+/- 10, 100+/- 10, 1 10+/- 10, 120+/- 10, 130+/- 10, 140+/- 10, 150+/- 10, 160+/- 10, 170+/- 10, 1 80+/- 10, 190+/- 10, 200+/- 10, 210+/-10, of 220+/- 10 nucleotides in length. In an embodiment, the template nucleic acid may be 30+/-20, 40+/-20, 50+/-20, 60+/- 20, 70+/- 20, 80+/-20, 90+/-20, 100+/-20, 1 10+/-20, 120+/-20, 130+/-20, 140+/-20, 150+/-20, 160+/-20, 170+/-20, 180+/-20, 190+/-20, 200+/-20, 210+/-20, of 220+/-20 nucleotides in length. In an embodiment, the template nucleic acid is 10 to 1 ,000, 20 to 900, 30 to 800, 40 to 700, 50 to 600, 50 to 500, 50 to 400, 50 to300, 50 to 200, or 50 to 100 nucleotides in length.

In some embodiments, the template polynucleotide is complementary to a portion of a polynucleotide comprising the target sequence. When optimally aligned, a template polynucleotide might overlap with one or more nucleotides of a target sequences (e.g., about or more than about 1, 5, 10, 15, 20, 25, 30, 35, 40, 45, 50, 60, 70, 80, 90, 100 or more nucleotides). In some embodiments, when a template sequence and a polynucleotide comprising a target sequence are optimally aligned, the nearest nucleotide of the template polynucleotide is within about 1, 5, 10, 15, 20, 25, 50, 75, 100, 200, 300, 400, 500, 1000, 5000, 10000, or more nucleotides from the target sequence.

The exogenous polynucleotide template comprises a sequence to be integrated (e.g., a mutated gene). The sequence for integration may be a sequence endogenous or exogenous to the cell. Examples of a sequence to be integrated include polynucleotides encoding a protein or a non-coding RNA (e.g., a microRNA). Thus, the sequence for integration may be operably linked to an appropriate control sequence or sequences. Alternatively, the sequence to be integrated may provide a regulatory function.

An upstream or downstream sequence may comprise from about 20 bp to about 2500 bp, for example, about 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, or 2500 bp. In some methods, the exemplary upstream or downstream sequence have about 200 bp to about 2000 bp, about 600 bp to about 1000 bp, or more particularly about 700 bp to about 1000.

An upstream or downstream sequence may comprise from about 20 bp to about 2500 bp, for example, about 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, or 2500 bp. In some methods, the exemplary upstream or downstream sequence have about 200 bp to about 2000 bp, about 600 bp to about 1000 bp, or more particularly about 700 bp to about 1000.

In certain embodiments, one or both homology arms may be shortened to avoid including certain sequence repeat elements. For example, a 5' homology arm may be shortened to avoid a sequence repeat element. In other embodiments, a 3' homology arm may be shortened to avoid a sequence repeat element. In some embodiments, both the 5' and the 3' homology arms may be shortened to avoid including certain sequence repeat elements.

In some methods, the exogenous polynucleotide template may further comprise a marker. Such a marker may make it easy to screen for targeted integrations. Examples of suitable markers include restriction sites, fluorescent proteins, or selectable markers. The exogenous polynucleotide template of the disclosure can be constructed using recombinant techniques (see, for example, Sambrook et al., 2001 and Ausubel et al., 1996).

In certain embodiments, a template nucleic acid for correcting a mutation may be designed for use as a single-stranded oligonucleotide. When using a single-stranded oligonucleotide, 5' and 3' homology arms may range up to about 200 base pairs (bp) in length, e.g., at least 25, 50, 75, 100, 125, 150, 175, or 200 bp in length.

In certain embodiments, a template nucleic acid for correcting a mutation may be designed for use with a homology-independent targeted integration system. Suzuki et al. describe *in vivo* genome editing via CRISPR/Cas9 mediated homology-independent targeted integration (2016, Nature 540:144-149). Schmid-Burgk, et al. describe use of the CRISPR-Cas9 system to introduce a double-strand break (DSB) at a user-defined genomic location and insertion of a universal donor DNA (Nat Commun. 2016 Jul 28;7:12338). Gao, et al. describe "Plug-and-Play Protein Modification Using Homology-Independent Universal Genome Engineering" (Neuron. 2019 Aug 21;103(4):583-597).

### RNAi

In some embodiments, the genetic modifying agents may be interfering RNAs. In certain embodiments, diseases caused by a dominant mutation in a gene is targeted by silencing the mutated gene using RNAi. In some cases, the nucleotide sequence may comprise coding sequence for one or more interfering RNAs. In certain examples, the nucleotide sequence may be interfering RNA (RNAi). As used herein, the term "RNAi" refers to any type of interfering RNA, including but not limited to, siRNAi, shRNAi, endogenous microRNA and artificial microRNA. For instance, it includes sequences previously identified as siRNA, regardless of the mechanism of down-stream processing of the RNA (i.e., although siRNAs are believed to have a specific method of in vivo processing resulting in the cleavage of mRNA, such sequences can be incorporated into the vectors in the context of the flanking sequences described herein). The term "RNAi" can include both gene silencing RNAi molecules, and also RNAi effector molecules which activate the expression of a gene.

In certain embodiments, a modulating agent may comprise silencing one or more endogenous genes. As used herein, "gene silencing" or "gene silenced" in reference to an activity of an RNAi molecule, for example a siRNA or miRNA refers to a decrease in the mRNA level in a cell for a target gene by at least about 5%, about 10%, about 20%, about 30%, about 40%, about 50%, about 60%, about 70%, about 80%, about 90%, about 95%, about 99%, about 100% of the mRNA level found in the cell without the presence of the miRNA or RNA interference molecule. In one preferred embodiment, the mRNA levels are decreased by at least about 70%, about 80%, about 90%, about 95%, about 99%, about 100%.

As used herein, a "siRNA" refers to a nucleic acid that forms a double stranded RNA, which double stranded RNA has the ability to reduce or inhibit expression of a gene or target gene when the siRNA is present or expressed in the same cell as the target gene. The double stranded RNA siRNA can be formed by the complementary strands. In one embodiment, a siRNA refers to a nucleic acid that can form a double stranded siRNA. The sequence of the siRNA can correspond to the full-length target gene, or a subsequence thereof. Typically, the siRNA is at least about 15-50 nucleotides in length (e.g., each complementary sequence of the double stranded siRNA is about 15-50 nucleotides in length, and the double stranded siRNA is about 15-50 base pairs in length, preferably about 19-30 base nucleotides, preferably about 20-25 nucleotides in length, e.g., 20, 21, 22, 23, 24, 25, 26, 27, 28, 29, or 30 nucleotides in length).

As used herein "shRNA" or "small hairpin RNA" (also called stem loop) is a type of siRNA. In one embodiment, these shRNAs are composed of a short, e.g., about 19 to about 25 nucleotide, antisense strand, followed by a nucleotide loop of about 5 to about 9 nucleotides, and the analogous sense strand. Alternatively, the sense strand can precede the nucleotide loop structure and the antisense strand can follow.

The terms "microRNA" or "miRNA" are used interchangeably herein are endogenous RNAs, some of which are known to regulate the expression of protein-coding genes at the posttranscriptional level. Endogenous microRNAs are small RNAs naturally present in the genome that are capable of modulating the productive utilization of mRNA. The term artificial microRNA includes any type of RNA sequence, other than endogenous microRNA, which is capable of modulating the productive utilization of mRNA. MicroRNA sequences have been described in publications such as Lim, et al., Genes & Development, 17, p. 991 - 1008 (2003), Lim et al Science 299, 1540 (2003), Lee and Ambros Science, 294, 862

(2001), Lau et al., Science 294, 858-861 (2001), Lagos-Quintana et al, Current Biology, 12, 735-739 (2002), Lagos Quintana et al, Science 294, 853- 857 (2001), and Lagos-Quintana et al, RNA, 9, 175- 179 (2003). Multiple microRNAs can also be incorporated into a precursor molecule. Furthermore, miRNA-like stem-loops can be expressed in cells as a vehicle to deliver artificial miRNAs and short interfering RNAs (siRNAs) for the purpose of modulating the expression of endogenous genes through the miRNA and or RNAi pathways.

As used herein, "double stranded RNA" or "dsRNA" refers to RNA molecules that are comprised of two strands. Double-stranded molecules include those comprised of a single RNA molecule that doubles back on itself to form a two-stranded structure. For example, the stem loop structure of the progenitor molecules from which the single-stranded miRNA is derived, called the pre-miRNA (Bartel et al. 2004. Cell 1 16:281 -297), comprises a dsRNA molecule.

### Administration of Therapeutic Agents

For therapeutic uses, the compositions or agents described herein may be administered systemically, for example, formulated in a pharmaceutically-acceptable buffer such as physiological saline. Preferable routes of administration include, for example, subcutaneous, intravenous, interperitoneal, intramuscular, or intradermal injections that provide continuous, sustained levels of the drug in the patient. Treatment of human patients or other animals will be carried out using a therapeutically effective amount of a therapeutic identified herein in a physiologically-acceptable carrier. Suitable carriers and their formulation are described, for example, in Remington's Pharmaceutical Sciences by E. W. Martin. The amount of the therapeutic agent to be administered varies depending upon the manner of administration, the age and body weight of the patient, and with the clinical symptoms of the neoplasia. Generally, amounts will be in the range of those used for other agents used in the treatment of other diseases associated with neoplasia, although in certain instances lower amounts will be needed because of the increased specificity of the compound. For example, a therapeutic compound is administered at a dosage that is cytotoxic to a neoplastic cell.

Human dosage amounts can initially be determined by extrapolating from the amount of compound used in mice, as a skilled artisan recognizes it is routine in the art to modify the dosage for humans compared to animal models. In certain embodiments, it is envisioned that the dosage may vary from between about 1 µg compound/Kg body weight to about 5000 mg compound/Kg body weight; or from about 5 mg/Kg body weight to about 4000 mg/Kg body weight or from about 10 mg/Kg body weight to about 3000 mg/Kg body weight; or from about 50 mg/Kg body weight to about 2000 mg/Kg body weight; or from about 100 mg/Kg body weight to about 1000 mg/Kg body weight; or from about 150 mg/Kg body weight to about 500 mg/Kg body weight. In other cases, this dose may be about 1, 5, 10, 25, 50, 75, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1600, 1700, 1800, 1900, 2000, 2500, 3000, 3500, 4000, 4500, or 5000 mg/Kg body weight. In other aspects, it is envisaged that doses may be in the range of about 5 mg compound/Kg body to about 20 mg compound/Kg body. In other embodiments, the doses may be about 8, 10, 12, 14, 16 or 18 mg/Kg body weight. Of course, this dosage amount may be adjusted upward or downward, as is routinely done in such treatment protocols, depending on the results of the initial clinical trials and the needs of a particular patient.

In some cases, the compound or composition of the invention is administered at a dose that is lower than the human equivalent dosage (HED) of the no observed adverse effect level (NOAEL) over a period of three months, four months, six months, nine months, 1 year, 2 years, 3 years, 4 years or more. The NOAEL, as determined in animal studies, is useful in determining the maximum recommended starting dose for human clinical trials. For instance, the NOAELs can be extrapolated to determine human equivalent dosages. Typically, such extrapolations between species are conducted based on the doses that are normalized to body surface area (i.e., mg/m²). In specific embodiments, the NOAELs are determined in mice, hamsters, rats, ferrets, guinea pigs, rabbits, dogs, primates, primates (monkeys, marmosets, squirrel monkeys, baboons), micropigs or minipigs. For a discussion on the use of NOAELs and their extrapolation to determine human equivalent doses, see Guidance for Industry Estimating the Maximum Safe Starting Dose in Initial Clinical Trials for Therapeutics in Adult Healthy Volunteers, U.S. Department of Health and Human Services Food and Drug Administration Center for Drug Evaluation and Research (CDER), Pharmacology and Toxicology, July 2005.

The amount of an agent of the invention used in the prophylactic and/or therapeutic regimens which will be effective in the prevention, treatment, and/or management of cancer can be based on the currently prescribed dosage of the agent as well as assessed by methods disclosed herein and known in the art. The frequency and dosage will vary also according to factors specific for each patient depending on the specific compounds administered, the severity of the cancerous condition, the route of administration, as well as age, body, weight, response, and the past medical history of the patient. For example, the dosage of an agent of the invention which will be effective in the treatment, prevention, and/or management of cancer can be determined by administering the compound to an animal model such as, e.g., the animal models disclosed herein or known to those skilled in the art. In addition, in vitro assays may optionally be employed to help identify optimal dosage ranges.

In some aspects, the prophylactic and/or therapeutic regimens comprise titrating the dosages administered to the patient so as to achieve a specified measure of therapeutic efficacy. Such measures include a reduction in the cancer cell population in the patient.

In certain cases, the dosage of the compound of the invention in the prophylactic and/or therapeutic regimen is adjusted so as to achieve a reduction in the number or amount of cancer cells found in a test specimen extracted from a patient after undergoing the prophylactic and/or therapeutic regimen, as compared with a reference sample. Here, the reference sample is a specimen extracted from the patient undergoing therapy, wherein the specimen is extracted from the patient at an earlier time point. In one aspect, the reference sample is a specimen extracted from the same patient, prior to receiving the prophylactic and/or therapeutic regimen. For example, the number or amount of cancer cells in the test specimen is at least 2%, 5%, 10%, 15%, 20%, 30%, 40%, 50%, 60%, 70%, 80%, 90%, 95% or 99% lower than in the reference sample.

In some cases, the dosage of the compound of the invention in the prophylactic and/or therapeutic regimen is adjusted so as to achieve a number or amount of cancer cells that falls within a predetermined reference range. In these embodiments, the number or amount of cancer cells in a test specimen is compared with a predetermined reference range.

In other embodiments, the dosage of the compound of the invention in prophylactic and/or therapeutic regimen is adjusted so as to achieve a reduction in the number or amount of cancer cells found in a test specimen extracted from a patient after undergoing the prophylactic and/or therapeutic regimen, as compared with a reference sample, wherein the reference sample is a specimen is extracted from a healthy, noncancer-afflicted patient. For example, the number or amount of cancer cells in the test specimen is at least within 60%, 50%, 40%, 30%, 20%, 15%, 10%, 5%, or 2% of the number or amount of cancer cells in the reference sample.

In treating certain human patients having solid tumors, extracting multiple tissue specimens from a suspected tumor site may prove impracticable. In these cases, the dosage of the compounds of the invention in the prophylactic and/or therapeutic regimen for a human patient is extrapolated from doses in animal models that are effective to reduce the cancer population in those animal models. In the animal models, the prophylactic and/or therapeutic regimens are adjusted so as to achieve a reduction in the number or amount of cancer cells found in a test specimen extracted from an animal after undergoing the prophylactic and/or therapeutic regimen, as compared with a reference sample. The reference sample can be a specimen extracted from the same animal, prior to receiving the prophylactic and/or therapeutic regimen. In specific embodiments, the number or amount of cancer cells in the test specimen is at least 2%, 5%, 10%, 15%, 20%, 30%, 40%, 50% or 60% lower than in the reference sample. The doses effective in reducing the number or amount of cancer cells in the animals can be normalized to body surface area (e.g., mg/m²) to provide an equivalent human dose.

The prophylactic and/or therapeutic regimens disclosed herein comprise administration of compounds of the invention or pharmaceutical compositions thereof to the patient in a single dose or in multiple doses (e.g., 1, 2, 3, 4, 5, 6, 7, 8, 10, 15, 20, or more doses).

In one aspect, the prophylactic and/or therapeutic regimens comprise administration of the compounds of the invention or pharmaceutical compositions thereof in multiple doses. When administered in multiple doses, the compounds or pharmaceutical compositions are administered with a frequency and in an amount sufficient to prevent, treat, and/or manage the condition. For example, the frequency of administration ranges from once a day up to about once every eight weeks. In another example, the frequency of administration ranges from about once a week up to about once every six weeks. In another example, the frequency of administration ranges from about once every three weeks up to about once every four weeks.

Generally, the dosage of a compound of the invention administered to a subject to prevent, treat, and/or manage cancer is in the range of 0.01 to 500 mg/kg, e.g., in the range of 0.1 mg/kg to 100 mg/kg, of the subject's body weight. For example, the dosage administered to a subject is in the range of 0.1 mg/kg to 50 mg/kg, or 1 mg/kg to 50 mg/kg, of the subject's body weight, more preferably in the range of 0.1 mg/kg to 25 mg/kg, or 1 mg/kg to 25 mg/kg, of the patient's body weight. In another example, the dosage of a compound of the invention administered to a subject to prevent, treat, and/or manage cancer in a patient is 500 mg/kg or less, preferably 250 mg/kg or less, 100 mg/kg or less, 95 mg/kg or less, 90 mg/kg or less, 85 mg/kg or less, 80 mg/kg or less, 75 mg/kg or less, 70 mg/kg or less, 65 mg/kg or less, 60 mg/kg or less, 55 mg/kg or less, 50 mg/kg or less, 45 mg/kg or less, 40 mg/kg or less, 35 mg/kg or less, 30 mg/kg or less, 25 mg/kg or less, 20 mg/kg or less, 15 mg/kg or less, 10 mg/kg or less, 5 mg/kg or less, 2.5 mg/kg or less, 2 mg/kg or less, 1.5 mg/kg or less, or 1 mg/kg or less of a patient's body weight.

In another example, the dosage of a compound of the invention administered to a subject to prevent, treat, and/or manage cancer in a patient is a unit dose of 0.1 to 50 mg, 0.1 mg to 20 mg, 0.1 mg to 15 mg, 0.1 mg to 12 mg, 0.1 mg to 10 mg, 0.1 mg to 8 mg, 0.1 mg to 7 mg, 0.1 mg to 5 mg, 0.1 to 2.5 mg, 0.25 mg to 20 mg, 0.25 to 15 mg, 0.25 to 12 mg, 0.25 to 10 mg, 0.25 to 8 mg, 0.25 mg to 7 mg, 0.25 mg to 5 mg, 0.5 mg to 2.5 mg, 1 mg to 20 mg, 1 mg to 15 mg, 1 mg to 12 mg, 1 mg to 10 mg, 1 mg to 8 mg, 1 mg to 7 mg, 1 mg to 5 mg, or 1 mg to 2.5 mg.

In another example, the dosage of a compound of the invention administered to a subject to prevent, treat, and/or manage cancer in a patient is in the range of 0.01 to 10 g/m², and more typically, in the range of 0.1 g/m² to 7.5 g/m², of the subject's body weight. For example, the dosage administered to a subject is in the range of 0.5 g/m² to 5 g/m², or 1 g/m² to 5 g/m² of the subject's body's surface area.

In another example, the prophylactic and/or therapeutic regimen comprises administering to a patient one or more doses of an effective amount of a compound of the invention, wherein the dose of an effective amount achieves a plasma level of at least 0.1 µg/mL, at least 0.5 µg/mL, at least 1 µg/mL, at least 2 µg/mL, at least 5 µg/mL, at least 6 µg/mL, at least 10 µg/mL, at least 15 µg/mL, at least 20 µg/mL, at least 25 µg/mL, at least 50 µg/mL, at least 100 µg/mL, at least 125 µg/mL, at least 150 µg/mL, at least 175 µg/mL, at least 200 µg/mL, at least 225 µg/mL, at least 250 µg/mL, at least 275 µg/mL, at least 300 µg/mL, at least 325 µg/mL, at least 350 µg/mL, at least 375 µg/mL, or at least 400 µg/mL of the compound of the invention.

In another example, the prophylactic and/or therapeutic regimen comprises administering to a patient a plurality of doses of an effective amount of a compound of the invention, wherein the plurality of doses maintains a plasma level of at least 0.1 µg/mL, at least 0.5 µg/mL, at least 1 µg/mL, at least 2 µg/mL, at least 5 µg/mL, at least 6 µg/mL, at least 10 µg/mL, at least 15 µg/mL, at least 20 µg/mL, at least 25 µg/mL, at least 50 µg/mL, at least 100 µg/mL, at least 125 µg/mL, at least 150 µg/mL, at least 175 µg/mL, at least 200 µg/mL, at least 225 µg/mL, at least 250 µg/mL, at least 275 µg/mL, at least 300 µg/mL, at least 325 µg/mL, at least 350 µg/mL, at least 375 µg/mL, or at least 400 µg/mL of the compound of the invention for at least 1 day, 1 month, 2 months, 3 months, 4 months, 5 months, 6 months, 7 months, 8 months, 9 months, 10 months, 11 months, 12 months, 15 months, 18 months, 24 months or 36 months.

In other embodiments, the prophylactic and/or therapeutic regimen comprises administering to a patient a plurality of doses of an effective amount of a compound of the invention, wherein the plurality of doses maintains a plasma level of at least 0.1 µg/mL, at least 0.5 µg/mL, at least 1 µg/mL, at least 2 µg/mL, at least 5 µg/mL, at least 6 µg/mL, at least 10 µg/mL, at least 15 µg/mL, at least 20 µg/mL, at least 25 µg/mL, at least 50 µg/mL, at least 100 µg/mL, at least 125 µg/mL, at least 150 µg/mL, at least 175 µg/mL, at least 200 µg/mL, at least 225 µg/mL, at least 250 µg/mL, at least 275 µg/mL, at least 300 µg/mL, at least 325 µg/mL, at least 350 µg/mL, at least 375 µg/mL, or at least 400 µg/mL of the compound of the invention for at least 1 day, 1 month, 2 months, 3 months, 4 months, 5 months, 6 months, 7 months, 8 months, 9 months, 10 months, 11 months, 12 months, 15 months, 18 months, 24 months or 36 months.

### Release of pharmaceutical compositions

Pharmaceutical compositions according to the invention may be formulated to release the active compound substantially immediately upon administration or at any predetermined time or time period after administration. The latter types of compositions are generally known as controlled release formulations, which include (i) formulations that create a substantially constant concentration of the drug within the body over an extended period of time; (ii) formulations that after a predetermined lag time create a substantially constant concentration of the drug within the body over an extended period of time; (iii) formulations that sustain action during a predetermined time period by maintaining a relatively, constant, effective level in the body with concomitant minimization of undesirable side effects associated with fluctuations in the plasma level of the active substance (sawtooth kinetic pattern); (iv) formulations that localize action by, e.g., spatial placement of a controlled release composition adjacent to or in contact with the thymus; (v) formulations that allow for convenient dosing, such that doses are administered, for example, once every one or two weeks; and (vi) formulations that target a neoplasia by using carriers or chemical derivatives to deliver the therapeutic agent to a particular cell type (e.g., neoplastic cell). For some applications, controlled release formulations obviate the need for frequent dosing during the day to sustain the plasma level at a therapeutic level.

Any of a number of strategies can be pursued in order to obtain controlled release in which the rate of release outweighs the rate of metabolism of the compound in question. In one example, controlled release is obtained by appropriate selection of various formulation parameters and ingredients, including, e.g., various types of controlled release compositions and coatings. Thus, the therapeutic is formulated with appropriate excipients into a pharmaceutical composition that, upon administration, releases the therapeutic in a controlled manner. Examples include single or multiple unit tablet or capsule compositions, oil solutions, suspensions, emulsions, microcapsules, microspheres, molecular complexes, nanoparticles, patches, and liposomes.

### Parenteral Compositions

The pharmaceutical composition may be administered parenterally by injection, infusion or implantation (subcutaneous, intravenous, intramuscular, intraperitoneal, or the like) in dosage forms, formulations, or via suitable delivery devices or implants containing conventional, non-toxic pharmaceutically acceptable carriers and adjuvants. The formulation and preparation of such compositions are well known to those skilled in the art of pharmaceutical formulation. Formulations can be found in Remington: The Science and Practice of Pharmacy, supra.

Compositions for parenteral use may be provided in unit dosage forms (e.g., in single-dose ampoules), or in vials containing several doses and in which a suitable preservative may be added (see below). The composition may be in the form of a solution, a suspension, an emulsion, an infusion device, or a delivery device for implantation, or it may be presented as a dry powder to be reconstituted with water or another suitable vehicle before use. Apart from the active agent that reduces or ameliorates a neoplasia, the composition may include suitable parenterally acceptable carriers and/or excipients. The active therapeutic agent(s) may be incorporated into microspheres, microcapsules, nanoparticles, liposomes, or the like for controlled release. Furthermore, the composition may include suspending, solubilizing, stabilizing, pH-adjusting agents, tonicity adjusting agents, and/or dispersing, agents.

As indicated above, the pharmaceutical compositions according to the invention may be in the form suitable for sterile injection. To prepare such a composition, the suitable active antineoplastic therapeutic(s) are dissolved or suspended in a parenterally acceptable liquid vehicle. Among acceptable vehicles and solvents that may be employed are water, water adjusted to a suitable pH by addition of an appropriate amount of hydrochloric acid, sodium hydroxide or a suitable buffer, 1,3-butanediol, Ringer's solution, and isotonic sodium chloride solution and dextrose solution. The aqueous formulation may also contain one or more preservatives (e.g., methyl, ethyl or n-propyl p-hydroxybenzoate). In cases where one of the compounds is only sparingly or slightly soluble in water, a dissolution enhancing or solubilizing agent can be added, or the solvent may include 10-60% w/w of propylene glycol.

### Controlled Release Parenteral Compositions

Controlled release parenteral compositions may be in form of aqueous suspensions, microspheres, microcapsules, magnetic microspheres, oil solutions, oil suspensions, or emulsions. Alternatively, the active drug may be incorporated in biocompatible carriers, liposomes, nanoparticles, implants, or infusion devices.

Materials for use in the preparation of microspheres and/or microcapsules are, e.g., biodegradable/bioerodible polymers such as polygalactin, poly-(isobutyl cyanoacrylate), poly(2-hydroxyethyl-L-glutam- nine) and, poly(lactic acid). Biocompatible carriers that may be used when formulating a controlled release parenteral formulation are carbohydrates (e.g., dextrans), proteins (e.g., albumin), lipoproteins, or antibodies. Materials for use in implants can be non-biodegradable (e.g., polydimethyl siloxane) or biodegradable (e.g., poly(caprolactone), poly(lactic acid), poly(glycolic acid) or poly(ortho esters) or combinations thereof).

### Vector Delivery

The invention also provides a delivery system comprising one or more vectors or one or more polynucleotide molecules, the one or more vectors or polynucleotide molecules comprising one or more polynucleotide molecules encoding components of a non-naturally occurring or engineered composition which is a composition having the characteristics as discussed herein or defined in any of the herein described methods. Delivery vehicles, vectors, particles, nanoparticles, formulations and components thereof for expression of one or more elements of a nucleic acid-targeting system are as used in the foregoing documents, such as WO 2014/093622 (PCT/US2013/074667).

In general, and throughout this specification, the term "vector" refers to a nucleic acid molecule capable of transporting another nucleic acid to which it has been linked. Vectors include, but are not limited to, nucleic acid molecules that are single-stranded, double-stranded, or partially double-stranded; nucleic acid molecules that comprise one or more free ends, no free ends (e.g., circular); nucleic acid molecules that comprise DNA, RNA, or both; and other varieties of polynucleotides known in the art. One type of vector is a "plasmid," which refers to a circular double stranded DNA loop into which additional DNA segments can be inserted, such as by standard molecular cloning techniques. Another type of vector is a viral vector, wherein virally-derived DNA or RNA sequences are present in the vector for packaging into a virus (e.g., retroviruses, replication defective retroviruses, adenoviruses, replication defective adenoviruses, and adeno-associated viruses). Viral vectors also include polynucleotides carried by a virus for transfection into a host cell. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors." Vectors for and that result in expression in a eukaryotic cell can be referred to herein as "eukaryotic expression vectors." Common expression vectors of utility in recombinant DNA techniques are often in the form of plasmids.

### Ribonucleoprotein (RNP)

In particular embodiments, pre-complexed guide RNA and CRISPR effector protein, (optionally, adenosine deaminase fused to a CRISPR protein or an adaptor) are delivered as a ribonucleoprotein (RNP). RNPs have the advantage that they lead to rapid editing effects even more so than the RNA method because this process avoids the need for transcription. An important advantage is that both RNP delivery is transient, reducing off-target effects and toxicity issues. Efficient genome editing in different cell types has been observed by Kim et al. (2014, Genome Res. 24(6):1012-9), Paix et al. (2015, Genetics 204(1):47-54), Chu et al. (2016, BMC Biotechnol. 16:4), and Wang et al. (2013, Cell. 9;153(4):910-8).

In particular embodiments, the ribonucleoprotein is delivered by way of a polypeptide-based shuttle agent as described in WO2016161516. WO2016161516 describes efficient transduction of polypeptide cargos using synthetic peptides comprising an endosome leakage domain (ELD) operably linked to a cell penetrating domain (CPD), to a histidine-rich domain and a CPD. Similarly, these polypeptides can be used for the delivery of CRISPR-effector based RNPs in eukaryotic cells.

### Administration of proteins

Significant progress has been made in understanding pharmacokinetics (PK), pharmacodynamics (PD), as well as toxicity profiles of therapeutic proteins in animals and humans, which have been in commercial development for more than three decades (see, e.g., Vugmeyster et al., Pharmacokinetics and toxicology of therapeutic proteins: Advances and challenges, World J Biol Chem. 2012 Apr 26; 3(4): 73-92). In certain embodiments, therapeutic proteins are administered by parenteral routes, such as intravenous (IV), subcutaneous (SC) or intramuscular (IM) injection. Molecular size, hydrophilicity, and gastric degradation are the main factors that preclude gastrointestinal (GI) absorption of therapeutic proteins (see, e.g., Keizer, et al., Clinical pharmacokinetics of therapeutic monoclonal antibodies. Clin Pharmacokinet. 2010 Aug; 49(8):493-507). Pulmonary delivery with aerosol formulations or dry powder inhalers has been used for selected proteins, e.g., exubera (TM) (see, e.g., Scheuch and Siekmeier, Novel approaches to enhance pulmonary delivery of proteins and peptides. J Physiol Pharmacol. 2007 Nov; 58 Suppl 5(Pt 2):615-25). Intravitreal injections have been used for peptides and proteins that require only local activity (see, e.g., Suresh, et al., Ocular Delivery of Peptides and Proteins. In: Van Der Walle C., editor. Peptide and Protein Delivery. London: Academic Press; 2011. pp. 87-103). In certain embodiments, SC administration of therapeutic proteins is often a preferred route. In particular, the suitability of SC dosing for self-administration translates into significantly reduced treatment costs.

### Standard of Care

Aspects of the invention involve modifying the therapy within a standard of care based on the detection of any of the biomarkers as described herein. In one embodiment, therapy comprising an agent is administered within a standard of care where addition of the agent is synergistic within the steps of the standard of care. In one embodiment, the agent targets and/or shifts a tumor to be more vulnerable to a therapeutic agent targeting XPR1:KIDINS220-mediated phosphate export (e.g., an inhibitor). In one embodiment, the agent inhibits expression or activity of one or more genes involved in phosphate homeostasis. The term "standard of care" as used herein refers to the current treatment that is accepted by medical experts as a proper treatment for a certain type of disease and that is widely used by healthcare professionals. Standard of care is also called best practice, standard medical care, and standard therapy. Standards of care for cancer generally include surgery, lymph node removal, radiation, chemotherapy, targeted therapies, antibodies targeting the tumor, and immunotherapy. Immunotherapy can include checkpoint blockers (CBP), chimeric antigen receptors (CARs), and adoptive T-cell therapy. The standards of care for the most common cancers can be found on the website of National Cancer Institute (www.cancer.gov/cancertopics). A treatment clinical trial is a research study meant to help improve current treatments or obtain information on new treatments for patients with cancer. When clinical trials show that a new treatment is better than the standard treatment, the new treatment may be considered the new standard treatment.

In certain embodiments, the present invention provides for one or more therapeutic agents (e.g., inhibitors) that can be used in combination with the standard of care for the cancer. Targeting XPR1:KIDINS220-mediated phosphate export in combination within a standard of care may provide for enhanced or otherwise previously unknown activity in the treatment of disease.

In certain embodiments, the present invention provides for a combination therapy comprising a treatment described herein with a treatment that is part of the standard of care for a cancer (i.e., a therapeutic regime). In certain embodiments, the standard of care for treating ovarian cancer comprises surgery, chemotherapy, and targeted therapy (see, e.g., Lheureux et al., Epithelial ovarian cancer: Evolution of management in the era of precision medicine. CA Cancer J Clin. 2019 Jul;69(4):280-304). Ovarian cancer is a cancer that forms in or on an ovary. Symptoms may include bloating, pelvic pain, abdominal swelling, and loss of appetite, among others. Common areas to which the cancer may spread include the lining of the abdomen, lymph nodes, lungs, and liver. The most common type of ovarian cancer is ovarian carcinoma (>95% of all cases). There are five main subtypes of ovarian carcinoma, of which high-grade serous carcinoma is the most common. These tumors are believed to start in the cells covering the ovaries, though some may form at the Fallopian tubes. Less common types of ovarian cancer include germ cell tumors and sex cord stromal tumors. A diagnosis of ovarian cancer is confirmed through a biopsy of tissue, usually removed during surgery.

If caught and treated in an early stage, ovarian cancer is often curable. Treatment usually includes some combination of surgery, radiation therapy, and chemotherapy. Outcomes depend on the extent of the disease, the subtype of cancer present, and other medical conditions. The overall five-year survival rate in the United States is 45%.

If ovarian cancer recurs, it is considered partially platinum-sensitive or platinum-resistant, based on the time since the last recurrence treated with platins: partially platinum-sensitive cancers recurred 6-12 months after last treatment, and platinum-resistant cancers have an interval of less than 6 months.

For platinum-sensitive tumors, platins are utilized for second-line chemotherapy, often in combination with other cytotoxic agents. Regimens include carboplatin combined with pegylated liposomal doxorubicin, gemcitabine, or paclitaxel. If the tumor is determined to be platinum-resistant, vincristine, dactinomycin, and cyclophosphamide (VAC) or some combination of paclitaxel, gemcitabine, and oxaliplatin can be used as a second-line therapy.

Systemic therapy can include single to combination chemotherapy approaches alone or in combination with targeted therapy. In certain embodiments, surgery includes surgery for accurate surgical staging, primary debulking surgery, interval debulking surgery, and secondary debulking surgery. In certain embodiments, chemotherapy includes carboplatin, cisplatin and paclitaxel. In certain embodiments, targeted therapy includes Bevacizumab, which is a humanized monoclonal antibody against vascular endothelial growth factor (VEGF), and poly (ADP-ribose) polymerase (PARP) inhibitors (e.g., Olaparib, Niraparib, and Rucaparib). Other therapies that may be used in combination with the present invention include agents targeting the folate receptor (e.g., mirvetuximab soravtansine (IMGN853), which is an ADC consisting of an anti-FRα antibody linked to the tubulin-disrupting maytansinoid DM4 drug, a potent antimitotic agent). In certain embodiments, checkpoint blockade therapy is used in a combination therapy. As used herein, checkpoint blockade therapy (CPB) refers to antibodies that block the activity of checkpoint receptors, including CTLA-4, PD-1, Tim-3, Lag-3, and TIGIT, either alone or in combination. The checkpoint blockade therapy may comprise anti-TIM3, anti-CTLA4, anti-PD-L1, anti-PD1, anti-TIGIT, anti-LAG3, or combinations thereof. Anti-PD1 antibodies are disclosed in U.S. Pat. No. 8,735,553. Antibodies to LAG-3 are disclosed in U.S. Pat. No. 9,132,281. Anti-CTLA4 antibodies are disclosed in U.S. Pat. No. 9,327,014; U.S. Pat. No. 9,320,811; and U.S. Pat. No. 9,062,111. Specific check point inhibitors include, but are not limited to, anti-CTLA4 antibodies (e.g., Ipilimumab and Tremelimumab), anti-PD-1 antibodies (e.g., Nivolumab, Pembrolizumab), and anti-PD-L1 antibodies (e.g., Atezolizumab). In certain embodiments, chemotherapy in combination with immunotherapy is used in the treatment of ovarian cancer. In certain embodiments, the combination therapy comprises paclitaxel plus pembrolizumab, preferably in patients with platinum-resistant ovarian cancer. In certain embodiments, the combination therapy comprises immunotherapy combined with PARP inhibitors.

In one example, therapeutic agents are administered in a combination therapy, i.e., combined with other agents, e.g., therapeutic agents, that are useful for treating pathological conditions or disorders, such as various forms of cancer. The term "in combination" in this context means that the agents are given substantially contemporaneously, either simultaneously or sequentially. If given sequentially, at the onset of administration of the second agent, the first of the two agents is in some cases still detectable at effective concentrations at the site of treatment.

The administration of an agent or a combination of agents for the treatment of a neoplasia may be by any suitable means that results in a concentration of the therapeutic that, combined with other components, is effective in ameliorating, reducing, or stabilizing a neoplasia. The agent may be contained in any appropriate amount in any suitable carrier substance, and is generally present in an amount of 1-95% by weight of the total weight of the composition. The composition may be provided in a dosage form that is suitable for parenteral (e.g., subcutaneously, intravenously, intramuscularly, or intraperitoneally) administration route. The pharmaceutical compositions may be formulated according to conventional pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York).

Accordingly, in some examples, the prophylactic and/or therapeutic regimen comprises administration of an agent of the invention in combination with one or more additional anticancer therapeutics. In one example, the dosages of the one or more additional anticancer therapeutics used in the combination therapy is lower than those which have been or are currently being used to prevent, treat, and/or manage cancer. The recommended dosages of the one or more additional anticancer therapeutics currently used for the prevention, treatment, and/or management of cancer can be obtained from any reference in the art including, but not limited to, Hardman et al., eds., Goodman & Gilman's The Pharmacological Basis of Therapeutics, 10th ed., McGraw-Hill, New York, 2001; Physician's Desk Reference (60th ed., 2006).

The agent of the invention and the one or more additional anticancer therapeutics can be administered separately, simultaneously, or sequentially. In various aspects, the agent of the invention and the additional anticancer therapeutic are administered less than 5 minutes apart, less than 30 minutes apart, less than 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In another example, two or more anticancer therapeutics are administered within the same patient visit.

In certain aspects, the agent of the invention and the additional anticancer therapeutic are cyclically administered. Cycling therapy involves the administration of one anticancer therapeutic for a period of time, followed by the administration of a second anticancer therapeutic for a period of time and repeating this sequential administration, i.e., the cycle, in order to reduce the development of resistance to one or both of the anticancer therapeutics, to avoid or reduce the side effects of one or both of the anticancer therapeutics, and/or to improve the efficacy of the therapies. In one example, cycling therapy involves the administration of a first anticancer therapeutic for a period of time, followed by the administration of a second anticancer therapeutic for a period of time, optionally, followed by the administration of a third anticancer therapeutic for a period of time and so forth, and repeating this sequential administration, i.e., the cycle in order to reduce the development of resistance to one of the anticancer therapeutics, to avoid or reduce the side effects of one of the anticancer therapeutics, and/or to improve the efficacy of the anticancer therapeutics.

In another example, the anticancer therapeutics are administered concurrently to a subj ect in separate compositions. The combination anticancer therapeutics of the invention may be administered to a subject by the same or different routes of administration.

When an agent of the invention and the additional anticancer therapeutic are administered to a subject concurrently, the term "concurrently" is not limited to the administration of the anticancer therapeutics at exactly the same time, but rather, it is meant that they are administered to a subject in a sequence and within a time interval such that they can act together (e.g., synergistically to provide an increased benefit than if they were administered otherwise). For example, the anticancer therapeutics may be administered at the same time or sequentially in any order at different points in time; however, if not administered at the same time, they should be administered sufficiently close in time so as to provide the desired therapeutic effect, preferably in a synergistic fashion. The combination anticancer therapeutics of the invention can be administered separately, in any appropriate form and by any suitable route. When the components of the combination anticancer therapeutics are not administered in the same pharmaceutical composition, it is understood that they can be administered in any order to a subject in need thereof. For example, a agent of the invention can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of the additional anticancer therapeutic, to a subject in need thereof. In various aspects, the anticancer therapeutics are administered 1 minute apart, 10 minutes apart, 30 minutes apart, less than 1 hour apart, 1 hour apart, 1 hour to 2 hours apart, 2 hours to 3 hours apart, 3 hours to 4 hours apart, 4 hours to 5 hours apart, 5 hours to 6 hours apart, 6 hours to 7 hours apart, 7 hours to 8 hours apart, 8 hours to 9 hours apart, 9 hours to 10 hours apart, 10 hours to 11 hours apart, 11 hours to 12 hours apart, no more than 24 hours apart or no more than 48 hours apart. In one example, the anticancer therapeutics are administered within the same office visit. In another example, the combination anticancer therapeutics of the invention are administered at 1 minute to 24 hours apart.

### DIAGNOSTIC METHODS

The invention provides biomarkers (e.g., malignant cell specific markers) for the identification, diagnosis, prognosis and manipulation of cell properties, for use in a variety of diagnostic and/or therapeutic indications. In certain embodiments, the marker may be SLC34A2, SLC20A1, FGF23 and/or PAX8. In certain embodiments, the marker is a gene that covaries with SLC34A2 (e.g., PAX8) and thus can be detected in combination or alone. In certain embodiments, detecting a tumor marker may indicate that a subject suffering from a cancer may respond to inhibition of XPR1:KIDINS220-mediated phosphate export. In certain embodiments, detecting a tumor marker may indicate prognosis for a subject suffering from cancer. In certain embodiments, detecting a tumor marker may indicate a treatment is effective (i.e., monitoring the efficacy of the treatment). In certain embodiments, tumor cells that express SLC34A2 higher than in normal tissue are vulnerable to inhibition of XPR1:KIDINS220-mediated phosphate export (see, therapeutic methods). In certain embodiments, inhibition of XPR1:KIDINS220-mediated phosphate export results in a decrease in SLC34A2 and/or SLC20A1 expression, and/or an increase in FGF23 expression.

In certain embodiments, morphological changes can be used to determine whether a treatment is effective. In certain embodiments, treatment of a subject with an inhibitor of XPR1:KIDINS220-mediated phosphate export results in an increase in vacuole-like structures in tumor cells. In certain embodiments, the morphological changes are detected by microscopy.

In certain embodiments, an RBD protein as described in the therapeutic methods can be used for detection of XPR1 expressing cells. Giovannini, et al., 2013 show monitoring cell-surface expression of XPR1 with a soluble ligand derived from X-MLV Env RBD (XRBD) See also, US 9,791,435 B2; and US 2015/0099653 A1.

Biomarkers in the context of the present invention encompasses, without limitation nucleic acids, proteins, reaction products, and metabolites, together with their polymorphisms, mutations, variants, modifications, subunits, fragments, and other analytes or sample-derived measures. In certain embodiments, biomarkers include the signature genes or signature gene products, and/or cells as described herein.

Biomarkers are useful in methods of diagnosing, prognosing and/or staging an immune response in a subject by detecting a first level of expression, activity and/or function of one or more biomarker and comparing the detected level to a control of level wherein a difference in the detected level and the control level indicates that the presence of an immune response in the subject.

The terms "diagnosis" and "monitoring" are commonplace and well-understood in medical practice. By means of further explanation and without limitation the term "diagnosis" generally refers to the process or act of recognising, deciding on or concluding on a disease or condition in a subject on the basis of symptoms and signs and/or from results of various diagnostic procedures (such as, for example, from knowing the presence, absence and/or quantity of one or more biomarkers characteristic of the diagnosed disease or condition).

The terms "prognosing" or "prognosis" generally refer to an anticipation on the progression of a disease or condition and the prospect (e.g., the probability, duration, and/or extent) of recovery. A good prognosis of the diseases or conditions taught herein may generally encompass anticipation of a satisfactory partial or complete recovery from the diseases or conditions, preferably within an acceptable time period. A good prognosis of such may more commonly encompass anticipation of not further worsening or aggravating of such, preferably within a given time period. A poor prognosis of the diseases or conditions as taught herein may generally encompass anticipation of a substandard recovery and/or unsatisfactorily slow recovery, or to substantially no recovery or even further worsening of such.

The biomarkers of the present invention are useful in methods of identifying patient populations at risk or suffering from cancer or for identifying patients that will respond to specific treatments based on a detected level of expression, activity and/or function of one or more biomarkers. These biomarkers are also useful in monitoring subjects undergoing treatments and therapies for suitable or aberrant response(s) to determine efficaciousness of the treatment or therapy and for selecting or modifying therapies and treatments that would be efficacious in treating, delaying the progression of or otherwise ameliorating a symptom. The biomarkers provided herein are useful for selecting a group of patients at a specific state of a disease with accuracy that facilitates selection of treatments.

The term "monitoring" generally refers to the follow-up of a disease or a condition in a subject for any changes which may occur over time.

The terms also encompass prediction of a disease. The terms "predicting" or "prediction" generally refer to an advance declaration, indication or foretelling of a disease or condition in a subject not (yet) having said disease or condition. For example, a prediction of a disease or condition in a subject may indicate a probability, chance or risk that the subject will develop said disease or condition, for example within a certain time period or by a certain age. Said probability, chance or risk may be indicated *inter alia* as an absolute value, range or statistics, or may be indicated relative to a suitable control subject or subject population (such as, e.g., relative to a general, normal or healthy subject or subject population). Hence, the probability, chance or risk that a subject will develop a disease or condition may be advantageously indicated as increased or decreased, or as fold-increased or fold-decreased relative to a suitable control subject or subject population. As used herein, the term "prediction" of the conditions or diseases as taught herein in a subject may also particularly mean that the subject has a 'positive' prediction of such, i.e., that the subject is at risk of having such (e.g., the risk is significantly increased vis-à-vis a control subject or subject population). The term "prediction of no" diseases or conditions as taught herein as described herein in a subject may particularly mean that the subject has a 'negative' prediction of such, i.e., that the subject's risk of having such is not significantly increased vis-à-vis a control subject or subject population.

Suitably, an altered quantity or phenotype of the immune cells in the subject compared to a control subject having normal immune status or not having a disease comprising an immune component indicates that the subject has an impaired immune status or has a disease comprising an immune component or would benefit from an immune therapy.

Hence, the methods may rely on comparing the quantity of immune cell populations, biomarkers, or gene or gene product signatures measured in samples from patients with reference values, wherein said reference values represent known predictions, diagnoses and/or prognoses of diseases or conditions as taught herein.

For example, distinct reference values may represent the prediction of a risk (e.g., an abnormally elevated risk) of having a given disease or condition as taught herein vs. the prediction of no or normal risk of having said disease or condition. In another example, distinct reference values may represent predictions of differing degrees of risk of having such disease or condition.

In a further example, distinct reference values can represent the diagnosis of a given disease or condition as taught herein vs. the diagnosis of no such disease or condition (such as, e.g., the diagnosis of healthy, or recovered from said disease or condition, etc.). In another example, distinct reference values may represent the diagnosis of such disease or condition of varying severity.

In yet another example, distinct reference values may represent a good prognosis for a given disease or condition as taught herein vs. a poor prognosis for said disease or condition. In a further example, distinct reference values may represent varyingly favourable or unfavourable prognoses for such disease or condition.

Such comparison may generally include any means to determine the presence or absence of at least one difference and optionally of the size of such difference between values being compared. A comparison may include a visual inspection, an arithmetical or statistical comparison of measurements. Such statistical comparisons include, but are not limited to, applying a rule.

Reference values may be established according to known procedures previously employed for other cell populations, biomarkers and gene or gene product signatures. For example, a reference value may be established in an individual or a population of individuals characterised by a particular diagnosis, prediction and/or prognosis of said disease or condition (i.e., for whom said diagnosis, prediction and/or prognosis of the disease or condition holds true). Such population may comprise without limitation 2 or more, 10 or more, 100 or more, or even several hundred or more individuals.

A "deviation" of a first value from a second value may generally encompass any direction (e.g., increase: first value > second value; or decrease: first value < second value) and any extent of alteration.

For example, a deviation may encompass a decrease in a first value by, without limitation, at least about 10% (about 0.9-fold or less), or by at least about 20% (about 0.8-fold or less), or by at least about 30% (about 0.7-fold or less), or by at least about 40% (about 0.6-fold or less), or by at least about 50% (about 0.5-fold or less), or by at least about 60% (about 0.4-fold or less), or by at least about 70% (about 0.3-fold or less), or by at least about 80% (about 0.2-fold or less), or by at least about 90% (about 0.1-fold or less), relative to a second value with which a comparison is being made.

For example, a deviation may encompass an increase of a first value by, without limitation, at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 150% (about 2.5-fold or more), or by at least about 200% (about 3-fold or more), or by at least about 500% (about 6-fold or more), or by at least about 700% (about 8-fold or more), or like, relative to a second value with which a comparison is being made.

Preferably, a deviation may refer to a statistically significant observed alteration. For example, a deviation may refer to an observed alteration which falls outside of error margins of reference values in a given population (as expressed, for example, by standard deviation or standard error, or by a predetermined multiple thereof, e.g., ±1xSD or ±2xSD or ±3xSD, or ±1xSE or ±2xSE or ±3xSE). Deviation may also refer to a value falling outside of a reference range defined by values in a given population (for example, outside of a range which comprises ≥40%, ≥ 50%, ≥60%, ≥70%, ≥75% or ≥80% or ≥85% or ≥90% or ≥95% or even ≥100% of values in said population).

In a further embodiment, a deviation may be concluded if an observed alteration is beyond a given threshold or cut-off. Such threshold or cut-off may be selected as generally known in the art to provide for a chosen sensitivity and/or specificity of the prediction methods, e.g., sensitivity and/or specificity of at least 50%, or at least 60%, or at least 70%, or at least 80%, or at least 85%, or at least 90%, or at least 95%.

For example, receiver-operating characteristic (ROC) curve analysis can be used to select an optimal cut-off value of the quantity of a given immune cell population, biomarker or gene or gene product signatures, for clinical use of the present diagnostic tests, based on acceptable sensitivity and specificity, or related performance measures which are well-known *per se,* such as positive predictive value (PPV), negative predictive value (NPV), positive likelihood ratio (LR+), negative likelihood ratio (LR-), Youden index, or similar.

In one embodiment, the signature genes, biomarkers, and/or cells may be detected or isolated by immunofluorescence, immunohistochemistry (IHC), microscopy, fluorescence activated cell sorting (FACS), mass spectrometry (MS), mass cytometry (CyTOF), RNA-seq, single cell RNA-seq (described further herein), quantitative RT-PCR, single cell qPCR, Fluorescence In Situ Hybridization (FISH), RNA-FISH, MERFISH (multiplex (in situ) RNA FISH) and/or by in situ hybridization. Other methods including absorbance assays and colorimetric assays are known in the art and may be used herein. detection may comprise primers and/or probes or fluorescently bar-coded oligonucleotide probes for hybridization to RNA (see e.g., Geiss GK, et al., Direct multiplexed measurement of gene expression with color-coded probe pairs. Nat Biotechnol. 2008 Mar;26(3):317-25).

### Detection of SLC34A2

In certain embodiments, detection of increased expression of SLC34A2 and/or covarying genes indicates that a tumor is sensitive to inhibition of XPR1:KIDINS220-mediated phosphate export. SLC34A2 expression can be determined by detection of the protein or RNA transcripts using any method described further herein. Antibodies capable of detecting SLC34A2 have been developed (see, e.g., MX35: Yin BW, et al., Monoclonal antibody MX35 detects the membrane transporter NaPi2b (SLC34A2) in human carcinomas. Cancer Immun. 2008;8:3; Levan K, et al., Immunohistochemical evaluation of epithelial ovarian carcinomas identifies three different expression patterns of the MX35 antigen, NaPi2b. BMC Cancer. 2017;17(1):303; and RebMab200: Lopes dos Santos, et al., Rebmab200, a Humanized Monoclonal Antibody Targeting the Sodium Phosphate Transporter NaPi2b Displays Strong Immune Mediated Cytotoxicity against Cancer: A Novel Reagent for Targeted Antibody Therapy of Cancer. PLoS One. 2013; 8(7): e70332) and are applicable to the present invention. Detection of SLC34A2 with an anti-NaPi2b antibody has been described for determining whether a cancer is responsive to a NaPi2b-targeted antibody drug conjugate (see, US 2019/0160181 A1). Any future antibodies developed are also applicable to the present invention. In certain embodiments, antibodies as described in the therapeutic methods may be used.

In certain embodiments, detecting comprises one or more of immunohistochemistry (IHC), in situ RNA-seq (Ke, R. et al. In situ sequencing for RNA analysis in preserved tissue and cells. Nat. Methods 10, 857-860 (2013)), quantitative PCR, RNA-seq, CITE-seq (Stoeckius, M. et al. Simultaneous epitope and transcriptome measurement in single cells. Nat. Methods 14, 865-868 (2017)), western blot, Fluorescence In Situ Hybridization (FISH), MERFISH (Chen, K. H., Boettiger, A. N., Moffitt, J. R., Wang, S. & Zhuang, X. Spatially resolved, highly multiplexed RNA profiling in single cells. Science 348, (2015)), RNA-FISH, mass spectrometry, or FACS.

### Copy Number Variation

In certain embodiments, copy number variations (CNV) are detected in a tumor (e.g., XPR1) (see, e.g., Carter SL, et al., Absolute quantification of somatic DNA alterations in human cancer. Nat Biotechnol. 2012 May; 30(5):413-21; Tirosh, I. et al. Dissecting the multicellular ecosystem of metastatic melanoma by single-cell RNA-seq. Science 352, 189-196 (2016); Sathirapongsasuti, J. F. et al. Exome sequencing-based copy-number variation and loss of heterozygosity detection: ExomeCNV. Bioinformatics 27, 2648-2654 (2011); Krumm, N. et al. Copy number variation detection and genotyping from exome sequence data. Genome Res. 22, 1525-1532 (2012); de Araújo Lima, L. & Wang, K. PennCNV in whole-genome sequencing data. BMC Bioinformatics 18, 383 (2017); Fan, J. et al. Linking transcriptional and genetic tumor heterogeneity through allele analysis of single-cell RNA-seq data. Genome Res. 28, 1217-1227 (2018); Campbell, K. R. et al. Clonealign: statistical integration of independent single-cell RNA and DNA sequencing data from human cancers. Genome Biol. 20, 54 (2019); Chen, M., Gunel, M. & Zhao, H. SomatiCA: Identifying, characterizing and quantifying somatic copy number aberrations from cancer genome sequencing data. PLoS ONE 8, e78143

(2013); Serin Harmanci, A., et al., CaSpER identifies and visualizes CNV events by integrative analysis of single-cell or bulk RNA-sequencing data. Nat Commun 11, 89 (2020); and Oh, et al., Reliable Analysis of Clinical Tumor-Only Whole-Exome Sequencing Data. JCO Clin Cancer Inform. 2020; 4). In certain embodiments, amplifications are detected in XPR1 to identify tumors that are sensitive to inhibition of XPR1:KIDINS220-mediated phosphate export. In certain embodiments, FISH is used to detect CNVs. In certain embodiments, CNVs are detected by whole-exome sequencing (WES) or targeted panel sequencing. In certain embodiments, CNVs are detected by inference from a target sequencing panel. In certain embodiments, CNVs are determined using RNA-seq.

### Microscopy

In certain embodiments, the morphological changes are detected by microscopy. Microscopy is the technical field of using microscopes to view objects and areas of objects that cannot be seen with the naked eye (objects that are not within the resolution range of the normal eye) (see, e.g., Mualla et al., editors. In: Medical Imaging Systems: An Introductory Guide [Internet]. Cham (CH): Springer; 2018. Chapter 5. 2018 Aug 3. DOI: 10.1007/978-3-319-96520-8_5). Any method of microscopy may be used in the present invention (e.g., optical, electron, and scanning probe microscopy, or X-ray microscopy). In preferred embodiments, phase contrast, fluorescence or confocal microscopy is used.

### MS methods

Biomarker detection may also be evaluated using mass spectrometry methods. A variety of configurations of mass spectrometers can be used to detect biomarker values. Several types of mass spectrometers are available or can be produced with various configurations. In general, a mass spectrometer has the following major components: a sample inlet, an ion source, a mass analyzer, a detector, a vacuum system, and instrument-control system, and a data system. Difference in the sample inlet, ion source, and mass analyzer generally define the type of instrument and its capabilities. For example, an inlet can be a capillary-column liquid chromatography source or can be a direct probe or stage such as used in matrix-assisted laser desorption. Common ion sources are, for example, electrospray, including nanospray and microspray or matrix-assisted laser desorption. Common mass analyzers include a quadrupole mass filter, ion trap mass analyzer and time-of-flight mass analyzer. Additional mass spectrometry methods are well known in the art (see Burlingame et al., Anal. Chem. 70:647 R-716R (1998); Kinter and Sherman, New York (2000)).

Protein biomarkers and biomarker values can be detected and measured by any of the following: electrospray ionization mass spectrometry (ESI-MS), ESI-MS/MS, ESI-MS/(MS)n, matrix-assisted laser desorption ionization time-of-flight mass spectrometry (MALDI-TOF-MS), surface-enhanced laser desorption/ionization time-of-flight mass spectrometry (SELDI-TOF-MS), desorption/ionization on silicon (DIOS), secondary ion mass spectrometry (SIMS), quadrupole time-of-flight (Q-TOF), tandem time-of-flight (TOF/TOF) technology, called ultraflex III TOF/TOF, atmospheric pressure chemical ionization mass spectrometry (APCI-MS), APCI-MS/MS, APCI-(MS).sup.N, atmospheric pressure photoionization mass spectrometry (APPI-MS), APPI-MS/MS, and APPI-(MS).sup.N, quadrupole mass spectrometry, Fourier transform mass spectrometry (FTMS), quantitative mass spectrometry, and ion trap mass spectrometry.

Sample preparation strategies are used to label and enrich samples before mass spectroscopic characterization of protein biomarkers and determination biomarker values. Labeling methods include but are not limited to isobaric tag for relative and absolute quantitation (iTRAQ) and stable isotope labeling with amino acids in cell culture (SILAC). Capture reagents used to selectively enrich samples for candidate biomarker proteins prior to mass spectroscopic analysis include but are not limited to aptamers, antibodies, nucleic acid probes, chimeras, small molecules, an F(ab')₂ fragment, a single chain antibody fragment, an Fv fragment, a single chain Fv fragment, a nucleic acid, a lectin, a ligand-binding receptor, affybodies, nanobodies, ankyrins, domain antibodies, alternative antibody scaffolds (e.g. diabodies etc) imprinted polymers, avimers, peptidomimetics, peptoids, peptide nucleic acids, threose nucleic acid, a hormone receptor, a cytokine receptor, and synthetic receptors, and modifications and fragments of these.

### Immunoassays

Immunoassay methods are based on the reaction of an antibody to its corresponding target or analyte and can detect the analyte in a sample depending on the specific assay format. To improve specificity and sensitivity of an assay method based on immunoreactivity, monoclonal antibodies are often used because of their specific epitope recognition. Polyclonal antibodies have also been successfully used in various immunoassays because of their increased affinity for the target as compared to monoclonal antibodies Immunoassays have been designed for use with a wide range of biological sample matrices Immunoassay formats have been designed to provide qualitative, semi-quantitative, and quantitative results.

Quantitative results may be generated through the use of a standard curve created with known concentrations of the specific analyte to be detected. The response or signal from an unknown sample is plotted onto the standard curve, and a quantity or value corresponding to the target in the unknown sample is established.

Numerous immunoassay formats have been designed. ELISA or EIA can be quantitative for the detection of an analyte/biomarker. This method relies on attachment of a label to either the analyte or the antibody and the label component includes, either directly or indirectly, an enzyme. ELISA tests may be formatted for direct, indirect, competitive, or sandwich detection of the analyte. Other methods rely on labels such as, for example, radioisotopes (I¹²⁵) or fluorescence. Additional techniques include, for example, agglutination, nephelometry, turbidimetry, Western blot, immunoprecipitation, immunocytochemistry, immunohistochemistry, flow cytometry, Luminex assay, and others (see ImmunoAssay: A Practical Guide, edited by Brian Law, published by Taylor & Francis, Ltd., 2005 edition).

Exemplary assay formats include enzyme-linked immunosorbent assay (ELISA), radioimmunoassay, fluorescent, chemiluminescence, and fluorescence resonance energy transfer (FRET) or time resolved-FRET (TR-FRET) immunoassays. Examples of procedures for detecting biomarkers include biomarker immunoprecipitation followed by quantitative methods that allow size and peptide level discrimination, such as gel electrophoresis, capillary electrophoresis, planar electrochromatography, and the like.

Methods of detecting and/or quantifying a detectable label or signal generating material depend on the nature of the label. The products of reactions catalyzed by appropriate enzymes (where the detectable label is an enzyme; see above) can be, without limitation, fluorescent, luminescent, or radioactive or they may absorb visible or ultraviolet light. Examples of detectors suitable for detecting such detectable labels include, without limitation, x-ray film, radioactivity counters, scintillation counters, spectrophotometers, colorimeters, fluorometers, luminometers, and densitometers.

Any of the methods for detection can be performed in any format that allows for any suitable preparation, processing, and analysis of the reactions. This can be, for example, in multi-well assay plates (e.g., 96 wells or 384 wells) or using any suitable array or microarray. Stock solutions for various agents can be made manually or robotically, and all subsequent pipetting, diluting, mixing, distribution, washing, incubating, sample readout, data collection and analysis can be done robotically using commercially available analysis software, robotics, and detection instrumentation capable of detecting a detectable label.

### Hybridization assays

Such applications are hybridization assays in which a nucleic acid that displays "probe" nucleic acids for each of the genes to be assayed/profiled in the profile to be generated is employed. In these assays, a sample of target nucleic acids is first prepared from the initial nucleic acid sample being assayed, where preparation may include labeling of the target nucleic acids with a label, e.g., a member of a signal producing system. Following target nucleic acid sample preparation, the sample is contacted with the array under hybridization conditions, whereby complexes are formed between target nucleic acids that are complementary to probe sequences attached to the array surface. The presence of hybridized complexes is then detected, either qualitatively or quantitatively. Specific hybridization technology which may be practiced to generate the expression profiles employed in the subject methods includes the technology described in U.S. Pat. Nos. 5,143,854; 5,288,644; 5,324,633; 5,432,049; 5,470,710; 5,492,806; 5,503,980; 5,510,270; 5,525,464; 5,547,839; 5,580,732; 5,661,028; 5,800,992; as well as WO 95/21265; WO 96/31622; WO 97/10365; WO 97/27317; EP 373 203; and EP 785 280. In these methods, an array of "probe" nucleic acids that includes a probe for each of the biomarkers whose expression is being assayed is contacted with target nucleic acids as described above. Contact is carried out under hybridization conditions, e.g., stringent hybridization conditions as described above, and unbound nucleic acid is then removed. The resultant pattern of hybridized nucleic acids provides information regarding expression for each of the biomarkers that have been probed, where the expression information is in terms of whether or not the gene is expressed and, typically, at what level, where the expression data, i.e., expression profile, may be both qualitative and quantitative.

Optimal hybridization conditions will depend on the length (e.g., oligomer vs. polynucleotide greater than 200 bases) and type (e.g., RNA, DNA, PNA) of labeled probe and immobilized polynucleotide or oligonucleotide. General parameters for specific (i.e., stringent) hybridization conditions for nucleic acids are described in Sambrook et al., supra, and in Ausubel et al., "Current Protocols in Molecular Biology", Greene Publishing and Wiley-interscience, NY (1987), When the cDNA microarrays are used, typical hybridization conditions are hybridization in 5xSSC plus 0.2% SDS at 65C for 4 hours followed by washes at 25°C in low stringency wash buffer (1xSSC plus 0.2% SDS) followed by 10 minutes at 25°C in high stringency wash buffer (0.1SSC plus 0.2% SDS) (see Shena et al., Proc. Natl. Acad. Sci. USA, Vol. 93, p. 10614 (1996)). Useful hybridization conditions are also provided in, e.g., Tijessen, Hybridization With Nucleic Acid Probes", Elsevier Science Publishers B.V. (1993) and Kricka, "Nonisotopic DNA Probe Techniques", Academic Press, San Diego, Calif. (1992).

### Sequencing and Nucleic Acid Profiling

In certain embodiments, the invention involves targeted nucleic acid profiling (e.g., sequencing, quantitative reverse transcription polymerase chain reaction, and the like) (see e.g., Geiss GK, et al., Direct multiplexed measurement of gene expression with color-coded probe pairs. Nat Biotechnol. 2008 Mar;26(3):317-25). In certain embodiments, a target nucleic acid molecule (e.g., RNA molecule), may be sequenced by any method known in the art, for example, methods of high-throughput sequencing, also known as next generation sequencing or deep sequencing. A nucleic acid target molecule labeled with a barcode (for example, an origin-specific barcode) can be sequenced with the barcode to produce a single read and/or contig containing the sequence, or portions thereof, of both the target molecule and the barcode. Exemplary next generation sequencing technologies include, for example, Illumina sequencing, Ion Torrent sequencing, 454 sequencing, SOLiD sequencing, and nanopore sequencing amongst others.

In certain embodiments, the invention involves single cell RNA sequencing to detect or quantitate cells that are vulnerable to XPR1:KIDINS220-mediated phosphate export (see, e.g., Kalisky, T., Blainey, P. & Quake, S. R. Genomic Analysis at the Single-Cell Level. Annual review of genetics 45, 431-445, (2011); Kalisky, T. & Quake, S. R. Single-cell genomics. Nature Methods 8, 311-314 (2011); Islam, S. et al. Characterization of the single-cell transcriptional landscape by highly multiplex RNA-seq. Genome Research, (2011); Tang, F. et al. RNA-Seq analysis to capture the transcriptome landscape of a single cell. Nature Protocols 5, 516-535, (2010); Tang, F. et al. mRNA-Seq whole-transcriptome analysis of a single cell. Nature Methods 6, 377-382, (2009); Ramskold, D. et al. Full-length mRNA-Seq from single-cell levels of RNA and individual circulating tumor cells. Nature Biotechnology 30, 777-782, (2012); and Hashimshony, T., Wagner, F., Sher, N. & Yanai, I. CEL-Seq: Single-Cell RNA-Seq by Multiplexed Linear Amplification. Cell Reports, Cell Reports, Volume 2, Issue 3, p666-673, 2012).

In certain embodiments, the invention involves plate based single cell RNA sequencing (see, e.g., Picelli, S. et al., 2014, "Full-length RNA-seq from single cells using Smart-seq2" Nature protocols 9, 171-181, doi:10.1038/nprot.2014.006).

In certain embodiments, the invention involves high-throughput single-cell RNA-seq. In this regard reference is made to Macosko et al., 2015, "Highly Parallel Genome-wide Expression Profiling of Individual Cells Using Nanoliter Droplets" Cell 161, 1202-1214; International patent application number PCT/US2015/049178, published as WO2016/040476 on March 17, 2016; Klein et al., 2015, "Droplet Barcoding for Single-Cell Transcriptomics Applied to Embryonic Stem Cells" Cell 161, 1187-1201; International patent application number PCT/US2016/027734, published as WO2016168584A1 on October 20, 2016; Zheng, et al., 2016, "Haplotyping germline and cancer genomes with high-throughput linked-read sequencing" Nature Biotechnology 34, 303-311; Zheng, et al., 2017, "Massively parallel digital transcriptional profiling of single cells" Nat. Commun. 8, 14049 doi: 10.1038/ncomms14049; International patent publication number WO2014210353A2; Zilionis, et al., 2017, "Single-cell barcoding and sequencing using droplet microfluidics" Nat Protoc. Jan;12(1):44-73; Cao et al., 2017, "Comprehensive single cell transcriptional profiling of a multicellular organism by combinatorial indexing" bioRxiv preprint first posted online Feb. 2, 2017, doi: dx.doi.org/10.1101/104844; Rosenberg et al., 2017, "Scaling single cell transcriptomics through split pool barcoding" bioRxiv preprint first posted online Feb. 2, 2017, doi: dx.doi.org/10.1101/105163; Rosenberg et al., "Single-cell profiling of the developing mouse brain and spinal cord with split-pool barcoding" Science 15 Mar 2018; Vitak, et al., "Sequencing thousands of single-cell genomes with combinatorial indexing" Nature Methods, 14(3):302-308, 2017; Cao, et al., Comprehensive single-cell transcriptional profiling of a multicellular organism. Science, 357(6352):661-667, 2017; Gierahn et al., "Seq-Well: portable, low-cost RNA sequencing of single cells at high throughput" Nature Methods 14, 395-398 (2017); and Hughes, et al., "Highly Efficient, Massively-Parallel Single-Cell RNA-Seq Reveals Cellular States and Molecular Features of Human Skin Pathology" bioRxiv 689273; doi: doi.org/10.1101/689273.

In certain embodiments, the invention involves single nucleus RNA sequencing. In this regard reference is made to Swiech et al., 2014, "In vivo interrogation of gene function in the mammalian brain using CRISPR-Cas9" Nature Biotechnology Vol. 33, pp. 102-106; Habib et al., 2016, "Div-Seq: Single-nucleus RNA-Seq reveals dynamics of rare adult newborn neurons" Science, Vol. 353, Issue 6302, pp. 925-928; Habib et al., 2017, "Massively parallel single-nucleus RNA-seq with DroNc-seq" Nat Methods. 2017 Oct;14(10):955-958; International patent application number PCT/US2016/059239, published as WO2017164936 on September 28, 2017; International patent application number PCT/US2018/060860, published as WO/2019/094984 on May 16, 2019; International patent application number PCT/US2019/055894, published as WO/2020/077236 on April 16, 2020; and Drokhlyansky, et al., "The enteric nervous system of the human and mouse colon at a single-cell resolution," bioRxiv 746743; doi: doi.org/10.1101/746743.

In certain embodiments, the invention involves the Assay for Transposase Accessible Chromatin using sequencing (ATAC-seq) as described. (See, e.g., Buenrostro, et al., Transposition of native chromatin for fast and sensitive epigenomic profiling of open chromatin, DNA-binding proteins and nucleosome position. Nature methods 2013; 10 (12): 1213-1218; Buenrostro et al., Single-cell chromatin accessibility reveals principles of regulatory variation. Nature 523, 486-490 (2015); Cusanovich, D. A., Daza, R., Adey, A., Pliner, H., Christiansen, L., Gunderson, K. L., Steemers, F. J., Trapnell, C. & Shendure, J. Multiplex single-cell profiling of chromatin accessibility by combinatorial cellular indexing. Science. 2015 May 22;348(6237):910-4. doi: 10.1126/science.aab1601. Epub 2015 May 7; US20160208323A1; US20160060691A1; and WO2017156336A1).

### SCREENING METHODS

In certain embodiments, therapeutic agents that are capable of inhibiting XPR1:KIDINS220-mediated phosphate export are screened. Screening may be performed *in vitro* or *in vivo.* For example, agents that modulate phosphate export in a tumor microenvironment may be screened *in vivo.* In certain embodiments, cancer cell lines can be assayed for phosphate efflux as described herein.

A further aspect of the invention relates to a method for identifying an agent capable of inhibiting XPR1:KIDINS220-mediated phosphate export, comprising: a) applying a candidate agent to a cancer cell or cell population; b) detecting modulation of phosphate efflux in the cell or cell population by the candidate agent, thereby identifying the agent. The term "modulate" broadly denotes a qualitative and/or quantitative alteration, change or variation in that which is being modulated. Where modulation can be assessed quantitatively - for example, where modulation comprises or consists of a change in a quantifiable variable such as a quantifiable property of a cell or where a quantifiable variable provides a suitable surrogate for the modulation - modulation specifically encompasses both increase (e.g., activation) or decrease (e.g., inhibition) in the measured variable. The term encompasses any extent of such modulation, e.g., any extent of such increase or decrease, and may more particularly refer to statistically significant increase or decrease in the measured variable. By means of example, modulation may encompass an increase in the value of the measured variable by at least about 10%, e.g., by at least about 20%, preferably by at least about 30%, e.g., by at least about 40%, more preferably by at least about 50%, e.g., by at least about 75%, even more preferably by at least about 100%, e.g., by at least about 150%, 200%, 250%, 300%, 400% or by at least about 500%, compared to a reference situation without said modulation; or modulation may encompass a decrease or reduction in the value of the measured variable by at least about 10%, e.g., by at least about 20%, by at least about 30%, e.g., by at least about 40%, by at least about 50%, e.g., by at least about 60%, by at least about 70%, e.g., by at least about 80%, by at least about 90%, e.g., by at least about 95%, such as by at least about 96%, 97%, 98%, 99% or even by 100%, compared to a reference situation without said modulation. Preferably, modulation may be specific or selective, hence, one or more desired phenotypic aspects of an immune cell or immune cell population may be modulated without substantially altering other (unintended, undesired) phenotypic aspect(s).

The term "agent" broadly encompasses any condition, substance or agent capable of modulating one or more phenotypic aspects of a cell or cell population as disclosed herein. Such conditions, substances or agents may be of physical, chemical, biochemical and/or biological nature. The term "candidate agent" refers to any condition, substance or agent that is being examined for the ability to modulate one or more phenotypic aspects of a cell or cell population as disclosed herein in a method comprising applying the candidate agent to the cell or cell population (e.g., exposing the cell or cell population to the candidate agent or contacting the cell or cell population with the candidate agent) and observing whether the desired modulation takes place.

Agents may include any potential class of biologically active conditions, substances or agents, such as for instance antibodies, proteins, peptides, nucleic acids, oligonucleotides, small molecules, or combinations thereof, as described herein.

The screening methods can be utilized for evaluating environmental stress and/or state, for screening of chemical libraries, and to screen or identify structural, syntenic, genomic, and/or organism and species variations. For example, a culture of cells, can be exposed to an environmental stress, such as but not limited to heat shock, osmolarity, hypoxia, cold, oxidative stress, radiation, starvation, a chemical (for example a therapeutic agent or potential therapeutic agent) and the like. After the stress is applied, a representative sample can be subjected to analysis, for example at various time points, and compared to a control, such as a sample from an organism or cell, for example a cell from an organism, or a standard value. By exposing cells, or fractions thereof, tissues, or even whole animals, to different members of the chemical libraries, and performing the methods described herein, different members of a chemical library can be screened for their effect on phenotypes thereof simultaneously in a relatively short amount of time, for example using a high throughput method.

In some embodiments, screening of test agents involves testing a combinatorial library containing a large number of potential modulator agents. A combinatorial chemical library may be a collection of diverse chemical agents generated by either chemical synthesis or biological synthesis, by combining a number of chemical "building blocks" such as reagents. For example, a linear combinatorial chemical library, such as a polypeptide library, is formed by combining a set of chemical building blocks (amino acids) in every possible way for a given agent length (for example the number of amino acids in a polypeptide agent). Millions of chemical agents can be synthesized through such combinatorial mixing of chemical building blocks.

In certain embodiments, cancers sensitive to inhibition of XPR1:KIDINS220-mediated phosphate export are identified by screening tumor cells from a cancer. The method may comprise applying an inhibitor of XPR1:KIDINS220-mediated phosphate export to a cancer cell or cell population (e.g., RBD); and detecting the phosphate concentration in the cell or cell population, wherein the cancer is sensitive if the phosphate concentration is increased as compared to a control cell or population not treated with the inhibitor. In certain embodiments, the inhibitor of XPR1:KIDINS220-mediated phosphate export is one or more therapeutic agents according to any embodiment herein. In certain embodiments, the cancer cell or population is obtained or derived from a subject in need thereof. For example, the method can include personalized therapeutics, such that tumor cells from a subject are grown and assayed for sensitivity to inhibition of XPR1:KIDINS220-mediated phosphate export. In certain embodiments, cell lines derived from specific cancers are screened.

### KITS OR PHARMACEUTICAL SYSTEMS

The present compositions may be assembled into kits or pharmaceutical systems for use in ameliorating a neoplasia. Kits or pharmaceutical systems according to this aspect of the invention comprise a carrier means, such as a box, carton, tube or the like, having in close confinement therein one or more container means, such as vials, tubes, ampoules, or bottles. The kits or pharmaceutical systems of the invention may also comprise associated instructions for using the agents of the invention. The present invention also may comprise a kit with a detection reagent that binds to one or more biomarkers or can be used to detect one or more biomarkers.

Further embodiments are illustrated in the following Examples which are given for illustrative purposes only and are not intended to limit the scope of the invention.

### EXAMPLES

### Example 1 - Phosphate dysregulation via the novel XPR1:KIDINS220 protein complex is a therapeutic vulnerability in ovarian cancer

Applicants hypothesized that phosphate dysregulation had therapeutic potential for cancer by examining the cancer Dependency Map for targets with a viability defect in ovarian cancer ^{18,19}. Across all 733 cancer cell lines screened with CRISPR/Cas9, the phosphate exporter XPR1 has one of the most selective and predictive profiles across all genes **(****Figure 1a**), and especially for the smaller number of genetic dependencies observed in ovarian and uterine cancers. XPR1 inactivation has no effect on most cancer cell lines while ovarian and uterine cancer cell lines display a high degree of dependency on XPR1 (15 out of 63 cell lines with a CERES score < -0.6). This selective dependency profile is comparable to emerging (e.g. WRN ²⁰, EGLN1 ²¹) and well-established cancer therapeutic targets (e.g., EGFR or IKZF1). Therapeutic inhibition of XPR1 is also more feasible¹² than other highly selective yet challenging targets (e.g., transcription factors such as PAX8, HGM1, and WT1).

Applicants next pursued the molecular basis of the selective dependency on XPR1. Applicants built multivariate predictive models from the features most correlated with XPR1 dependency among the many cellular and molecular features of each cancer cell line ²². Using variable importance analysis of the most accurate model (r = 0.391) **(****Figure 1a**, Y-axis), the most predominant predictive feature is expression of the phosphate importer gene SLC34A2 **(****Figure 1b**, 0.38 normalized Gini importance). The correlation between high SLC34A2 expression and XPR1 dependency is mostly driven by a strong relationship in ovarian and uterine tissues (R² = 0.15, n = 731 in all versus R² = 0.33, n = 63 in ovarian/uterine, **Figure 2a****).** Interestingly, although increased SLC34A2 expression in ovarian cancer is of unknown consequence, its enhanced expression is well established ²³⁻²⁵.

The observation that high expression of SLC34A2 is associated with dependency on XPR1 led us to hypothesize that accumulation of intracellular phosphate upon XPR1 inactivation is selectively toxic to ovarian and uterine cancer cells **(****Figure 1c****).** Applicants first validated these observations by assessing cellular viability after genetic inactivation of XPR1 in a panel of ovarian and uterine cancer cell lines with low or high SLC334A2 expression. XPR1 inactivation by CRISPR/Cas9 decreased cellular viability in SLC34A2-high cell lines to a level similar to inactivation of pan-essential controls. In contrast, Applicants found no decrease of viability in SLC34A2-low cell lines **(****Figure 1d**). Similarly, this viability defect is seen when XPR1 is suppressed using shRNA reagents **(****Figure 2b****-d).**

Applicants next sought to systematically determine the extent to which SLC34A2 - or other genes - is necessary to confer dependency on XPR1 by conducting a genome-scale genetic loss-of-function modifier screen **(****Figure 3a****-b** ²⁶). Of the ~17,000 genes tested, the only genetic knockout which rescued the viability defects of XPR1 inactivation was SLC34A2 **(****Figure 1e****,** **Figure 3c****).** Applicants further tested the necessity and sufficiency of SLC34A2 by stably inactivating or overexpressing SLC34A2 in several cell lines. Altered SLC34A2 expression alone caused no growth or viability defects (see the dependency profile in **Figure 1b****),** but its expression is both necessary and sufficient to confer XPR1 dependency **(****Figure 1f**).

Dependencies sensitive to the metabolic environment can often be different between tissue culture and more physiologically relevant settings. Applicants next investigated if Applicants could find evidence of phosphate dysregulation in primary ovarian or uterine tumors and if Applicants could identify tumor samples where XPR1 might be predicted to be a dependency. Applicants first evaluated the relationship between XPR1 and SLC34A2, Applicants first sought evidence in primary samples from tumors in the The Cancer Genome Atlas (TCGA)^{27,28} and normal tissue from the Genotype-Tissue Expression project (GTEx) ^{29,30}. Applicants found that ovarian and uterine cancers are among the few tissues which display enhanced SLC34A2 expression **(****Figure 5a****).** Interestingly, although the fallopian tube does not have a known role in phosphate homeostasis, these normal samples have slightly higher expression of SLC34A2 relative to normal ovarian or uterine tissue **(****Figure 4a****).** Compared to normal fallopian tube epithelium, which may be a cell of origin for these tumors (Köbel et al. 2008; Piek et al. 2001; Jarboe et al. 2008; Hu et al. 2020), there was a 16.3- and 2.66-fold increase in ovarian and uterine cancers, respectively **(****Figure 4a****).** Most tumor samples had equivalent SLC34A2 expression as cancer cell lines highly dependent on XPR1.

Applicants next sought evidence of what is driving the high levels of SLC34A2 expression in these tumor samples. SLC34A2 has previously been reported to be a component of the paired box 8 (PAX8) transcriptional program ³⁵. PAX8 is a lineage-defining transcription factor which is amplified and upregulated in the course of ovarian carcinogenesis (Mittag et al. 2007; Cheung et al. 2011). Applicants found that all the ovarian cell lines expressing high levels of SLC34A2 also express high levels of PAX8 **(****Figure 5b****-c).** This may indicate that PAX8 expression drives the over-expression of SLC34A2 in ovarian cancer, an appealing hypothesis that requires further study.

According to the therapeutic hypothesis **(****Figure 1c****),** increased expression of SLC34A2 in tumor samples would create an increased demand for phosphate efflux, and a reliance on XPR1. In line with this hypothesis, Applicants found strong evidence for copy number amplifications of XPR1 in ovarian and uterine cancer **(****Figure 4b****)** ²⁷. The XPR1 locus undergoes significant and recurrent copy gains or amplifications in ovarian cancer **(****Figure 4c****;** q = 0.0015 by GISTIC ³⁸), that are often focal and only include XPR1. Uterine samples also had frequent XPR1 copy gains (44% of samples), although they were not significantly recurrent **(****Figure 5d****,** q=0.568; ref. ³⁹). Accordingly, XPR1 mRNA expression levels correlate with XPR1 copy number alterations, but other mechanisms are additionally driving XPR1 mRNA expression **(****Figures 4c****).** While the causative event behind increased expression of both XPR1 and SLC34A2 is not clear from these analyses, Applicants propose that reliance upon increased XPR1 expression is a consequence of increased SLC34A2. Together, these data indicate that ovarian tumors have altered phosphate homeostasis relative to normal tissue, and that high expression of SLC34A2 may serve as a therapeutic biomarker to predict response to XPR1 inhibition.

Applicants next sought evidence that the XPR1 dependency is not an artifact of supra-physiological phosphate levels from in vitro culture. Applicants first noted that while phosphate concentrations vary drastically between the growth medium of different cell lines (ranging from 15 to nearly 80 mg/dL), there was no correlation with the strength of the XPR1 dependency (R² = 0.00, n = 658, **Figure 6a****).** Furthermore, when Applicants lowered the phosphate concentration in a highly XPR1 dependent cell line by ~10-fold (from 72.8 to 7.8 mg/dL), the cells remained highly dependent on XPR1 **(****Figure 6b****-c),** indicating that physiological concentrations of extracellular phosphate are sufficient to inhibit cancer cell survival.

Applicants next directly evaluated whether XPR1 inactivation would affect the initiation and maintenance of cancer cell line xenografts. Applicants first developed in a CRISPR/Cas9-based tumor formation competition assay, in which a small library of sgRNA was delivered via lentivirus to cancer cell lines in vitro and rapidly inoculated subcutaneously **(****Figure 7a****),.** XPR1 inactivation sgRNAs were depleted caused a competitive disadvantage in this assay, and its depletion was noted in both tissue culture and all xenograft tumors in the two SLC34A2 high-expressing cell lines tested **(****Figure 4d****-e,** Supplemental table 1 and **Figure 7b****e).** In contrast, other metabolic genes, most notably the ferroptosis regulator GPX4, showed differences between their in vitro and in vivo viability effects **(****Figure 4d****-e),** indicating this assay can detect dependencies which are sensitive to the metabolic environment. Next, Applicants evaluated the effect of XPR1 suppression on established ovarian peritoneal carcinomatosis, a clinically relevant model. Applicants injected a SLC34A2 high-expressing ovarian cancer cell line expressing luciferase and a doxycycline-inducible shRNA targeting XPR1 or a seed-matched control shRNA **(****Figure 2****)** into the peritoneal cavity (see **Figure 8a** and methods for study design and timeline). After three weeks of tumor growth and acclimation to physiologically relevant levels of phosphate, tumor burden was consistent across animals **(****Figure 8b****-d),** and so Applicants treated the animals with control or doxycycline diets **(****Figure 4f****-g,** inset, n = 4 per group). XPR1 suppression significantly delayed tumor progression for two weeks following doxycycline treatment **(****Figure 4f****),** whereas induction of the control shRNA had no significant effect on tumor growth **(****Figure 4g****).** Taken together, the results of the subcutaneous sgRNA competition assays and the intraperitoneal xenografts indicate that the XPR1 dependency is retained in vivo with physiological levels of inorganic phosphate.

Applicants next pursued a mechanistic understanding of why SLC34A2-overexpressing cell lines lose viability after XPR1 knockout. Consistent with the therapeutic hypothesis, Applicants observed a 2-4 fold increase in intracellular phosphate after knockdown of XPR1 which occurred in the same timeframe as viability defects **(****Figure 9a****,** **Figure 10a****).** As the mechanisms to manage intracellular phosphate are not well-understood in human biology¹⁰, Applicants pursued gene expression profiling. Applicants used MixSeq⁴⁰, a multiplexed single-cell RNA sequencing assay to compare the transcriptional response across cell lines with varying degrees of dependency on XPR1.Applicants collected data from 2,501 single cells, representing 8 different ovarian and uterine cancer cell lines, 4 days after XPR1 inactivation using CRISPR/Cas9. This time-point was chosen to identify primary phosphate homeostatic mechanisms rather than secondary effects. At this time-point there were no significant differences in the number of cells between control and XPR1 inactivation conditions, indicating viability effects had not yet occurred.

Applicants observed a strong and highly correlated transcriptional response among the most XPR1-dependent cell lines. While there are few canonical gene sets represented in this signature **(****Figure 10f****-i)** this transcriptional program - at least in part - attempts to restore phosphate homeostasis which Applicants conclude based on the differential expression of several genes. First, Applicants observed the up-regulation of FGF23 **(****Figure 9f****),** a critical phosphate homeostatic hormone typically expressed by osteogenic bone cells in response to elevated serum phosphate ⁴¹. This is consistent with the increased intracellular phosphate Applicants observe **(****Figure 9a****).** Fallopian tube, ovarian, and uterine tissues have not been implicated in phosphate homeostasis, and so these cell lines' ability - though partial **(****Figure 10j****)** - to regulate FGF23 is surprising.⁹

Applicants also observed the downregulation of phosphate import. Two phosphate importer genes, SLC20A1 and SLC34A2, were significantly decreased after XPR1 knockout **(****Figure 9f****).** SLC34A2 protein levels are also dramatically reduced after knockout of XPR1 **(Figure 9g),** and XPR1 knockout causes a ~60% decrease in phosphate uptake, which is most likely due to the partial suppression of SLC34A2 **(Figure 9h).** These results are somewhat paradoxical given that full knockout of SLC34A2 can fully rescue the XPR1 viability defect (compare **Figures 1e****-f**) and indicate the dysregulation of SLC34A2. Incomplete suppression of SLC34A2 may be due to an alternative regulatory mechanism (such as that of PAX8 ³⁵), but this hypothesis requires further study. Overall, these data highlight the presence of a phosphate-sensing mechanism which attempts to counteract increased intracellular phosphate.

These results prompted Applicants to seek direct evidence that phosphate efflux is required for cell survival. Correlated dependency relationships often identify genes that are part of the same pathway or processes inform the mechanisms of cell killing^{42,43}, and highly correlated dependencies are often part of the same complex. Applicants were surprised that the dependency profile of an unrelated gene - Kinase D Interacting Substrate 220⁴⁴⁻⁴⁷ (KIDINS220, **Figure 11a** and **Figure 12a****-b)** - was strongly correlated with the dependency on XPR1. XPR1 and KIDINS220 expression is highly correlated across diverse tissues **(****Figure 12c****),** which led us to hypothesize that these genes likely co-operate to achieve phosphate efflux. Indeed, loss of KIDINS220 or XPR1 causes similar increases in intracellular phosphate **(****Figure 12d****).** While the exact mechanisms of phosphate efflux is unknown, previous work suggests that XPR1 and other orthologs use a transmembrane protein domain - called the EXS domain - to move between the golgi apparatus and the plasma membrane in order to achieve phosphate eflux move between the plasma membrane and the golgi apparatus to effect phosphate efflux, and this movement requires the C-terminal EXS domain^{48,49}. Interestingly, KIDINS220 also moves between these compartments⁵⁰. This prompted us to evaluate whether the localization and phosphate efflux of XPR1 requires KIDINS220.

By using publicly available mass spectrometry databases **(****Figure 12e****),** co-immunoprecipitation, and immunofluorescence, Applicants confirmed that XPR1 and KIDINS220 form a protein complex required for phosphate efflux. First, Applicants were able to identify the complex using conventional immunoprecipitation approaches. Applicants found that the his interaction is not mediated by the N-terminal SPX domain^{17,51}, but appears to requires a portion of the C-terminal EXS domain of XPR1, which is critical for proper localization of the protein ⁴⁸ **(****Figure 11b****,** **Figure 13a****-c).** Second, Applicants noted that inactivation of KIDINS220 causes mislocalization of XPR1 **(****Figure 11c****).** Finally, Applicants directly measured phosphate efflux in ovarian cancer cell lines, and found that inactivation of XPR1 or KIDINS220 decreased phosphate efflux to similar degrees **(****Figure 11d****).** The loss of KIDINS220 protein after XPR1 knockout **(****Figure 2b****)** and the observation that one cannot compensate for loss of the other **(****Figure 11a****)** indicates that these proteins are required for the function of the same protein complex and not separate efflux complexes. Thus, the co-dependency of two previously unconnected transmembrane proteins converges on a fundamental inability to export inorganic phosphate.

Applicants confirmed that XPR1 and KIDINS220 form a protein complex required for phosphate efflux with several experiments. First, in publicly available mass spectrometry datasets, XPR1 and KIDINS220 interact together, and with other proteins within the secretory pathway **(****Figure 13a****).** Second, Applicants confirmed the XPR1:KIDINS220 protein complex with co-immunoprecipitation, and found that the EXS domain of XPR1 is critical for this interaction **(****Figure 11b****).** Interestingly, the N-terminal SPX domain of XPR1 - which has been implicated in phosphate efflux and regulation - was neither necessary nor sufficient to bind KIDINS220. In line with this physical interaction, KIDINS220 protein levels decrease dramatically upon suppression of XPR1 **(****Figure 2b****).** Third, by using immunofluorescence, Applicants noted that KIDINS220 inactivation causes XPR1 to mislocalize from puncate secretory vesicles to a more diffuse cytoplasmic pattern. Finally, Applicants directly measured phosphate efflux and found that XPR1 and KIDINS220 inactivation impaired phosphate efflux to the same degree **(****Figure 11d****).** Importantly, the loss of KIDINS220 protein after XPR1 knockout **(****Figure 2b****)** and the observation that one cannot compensate for loss of the other **(****Figure 11a****)** indicates that these proteins are required for the function of the same protein complex and not separate efflux complexes. Thus, the co-dependency of two previously unconnected transmembrane proteins converges on a fundamental inability to export inorganic phosphate.

To further confirm that the phosphate efflux capacity of XPR1 is required for cancer cell viability, Applicants employed hypomorphic mutations of XPR1 which have been reported in a rare brain calcification disorder ^{15,52}. While knockout of endogenous XPR1 can be rescued by ectopic expression of full-length, wildtype XPR1, the L218S mutation did not support cellular viability despite proper localization **(****Figure 11e** and **Figure 13****).** This mutation has some residual phosphate efflux capacity¹⁵, perhaps indicating these cancer cells are sensitive to even partial loss of function. These data clearly indicate that functional phosphate efflux is required for cellular viability in ovarian cancer with SLC34A2 overexpression.

Vacuole-like structures have been described as phosphate-storage mechanisms in plants. During the course of these studies Applicants noticed that the XPR1-dependent cells formed large, "vacuole-like" structures in the cytoplasm, acytoplasma striking and highly penetrant change in the morphology of cells with high SLC34A2 expression after XPR1 or KIDINS220 inactivation **(****Figure 11f** and **Figure 14a****).** These cells formed large, "vacuole-like" structures in the cytoplasm. The structures were observed in all XPR1-dependent ovarian cancer cell lines tested, invariably preceded loss of cell viability (Supplemental movies), and frequently appeared in multinucleated cells. In some cases, the entire cytoplasm was filled with these structures which Applicants show are not derived from the Endoplasmic Reticulum, Golgi Apparatus, Mitochondria, Nucleus, or Early Endosomes **(****Figure 14b****).** However, these structures were stained with the acidic dye Lysotracker **(****Figure 11g****)** and appear to co-localize with the lysosomal marker LAMP1 **(****Figure 14b****),** suggesting they may be related to the lysosomal-system. Ultrastructural analysis by transmission electron microscopy revealed that these structures are bound by a double-membrane and are often fenestrated **(****Figure 11h****).** They do not have the electron-dense appearance typical of lysosomes, but Applicants did observe the fusion of lysosomes with these "vacuole-like" structures. Applicants hypothesize that these are novel structures but are related to phosphate-storage structures which have been reported in human biology ⁵³⁻⁵⁵ or other organisms. Perhaps inactivation of XPR1 or KIDINS220 causes the excessive cytoplasmic accumulation and fusion of vesicles used in the process of phosphate efflux. ^{49,56,57} Whether these structures are supporting cell survival or the 'cytoplasmic crowding' observed in these cancer cell lines is related to the observed cell cycle arrest **(****Figure 9c****)** and loss of cell viability is yet to be determined.

Treating cells with high concentrations of phosphate has been shown to be toxic before ^{58,59}, but this study exploits a unique synthetic lethal interaction between SLC34A2 overexpression and XPR1 inhibition. XPR1 inactivation, regardless of high expression of SLC34A2, is not toxic in tissues with known roles for organismal phosphate homeostasis (e.g., lung cancer ^{60,61}, **Figure 2a****).** This is possibly due to feedback mechanisms which suppress phosphate import upon increased intracellular phosphate (such as observed in **Figure 9** and reported before¹⁶). In contrast, over-expression of SLC34A2 - perhaps through PAX8 or another pathway - breaks this feedback mechanism in ovarian and uterine cancers, leading to accumulation of intracellular phosphate and cell death. The identity of this feedback mechanism, and whether its inhibition would enhance the XPR1 dependency, requires further study.

Therapeutic strategies to exploit phosphate dysregulation in ovarian cancer should focus on inhibiting the phosphate efflux capacity of XPR1:KIDINS220, perhaps through the extracellular binding of protein ligands¹². Applicants expect that a large patient population with SLC34A2 overexpression would respond to such a therapy, although intra-tumor heterogeneity would need to be evaluated^{23,25}. The side-effects of inhibiting XPR1:KIDINS220 are likely minimal and manageable. Although phosphate toxicity is unlikely to be toxic to individual cells - as most cell lines are not dependent on XPR1 or KIDINS220 - organismal perturbations in phosphate homeostasis should be avoided^{13,41}. Nevertheless, transient or partial inhibition of XPR1 are likely to be tolerated^{15,52,62} and there is precedent for the clinical management of hypophosphatemia ^{41,63}. Applicants hope that such a strategy will one day benefit cancer patients.

### Example 2 - Therapeutic Targeting of XPR1:KIDINS220

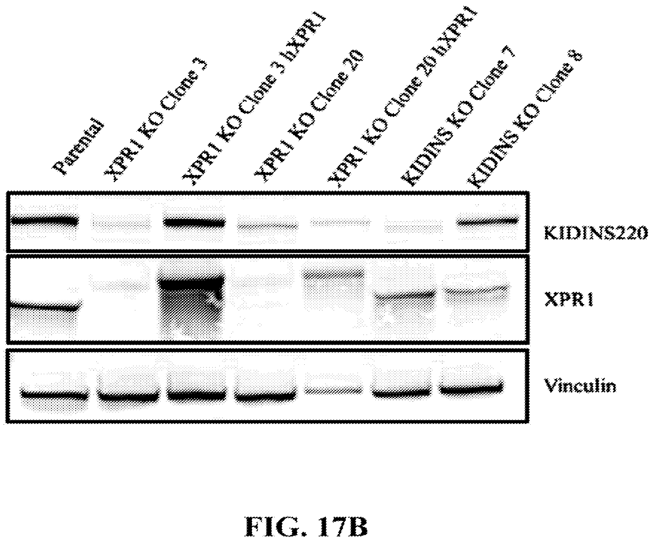

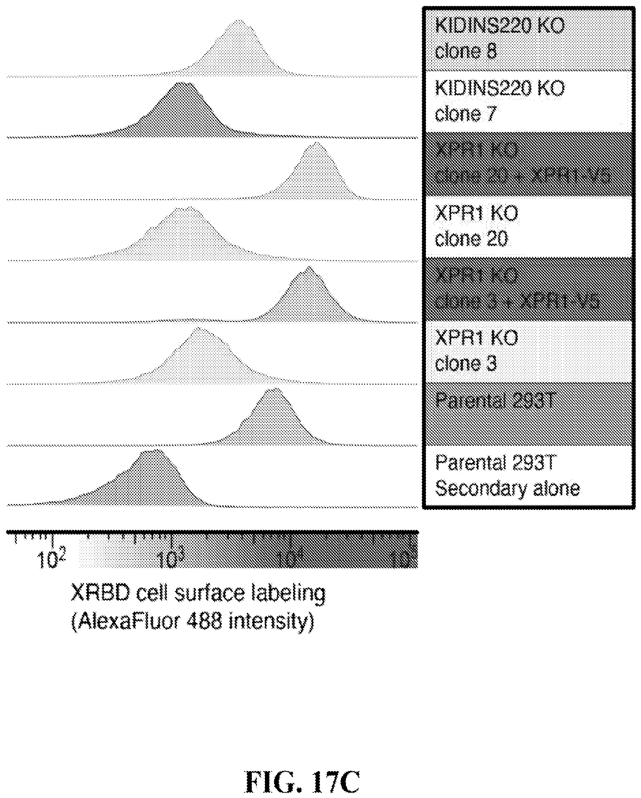

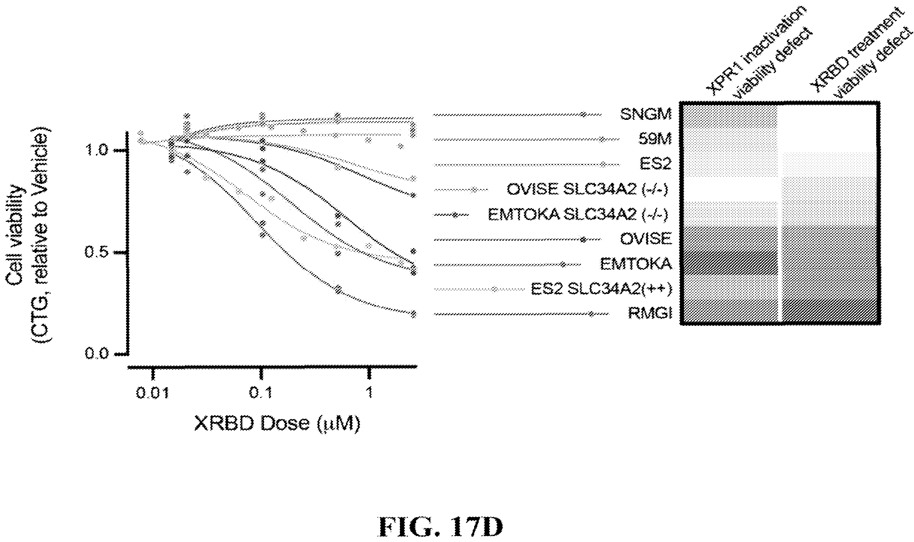

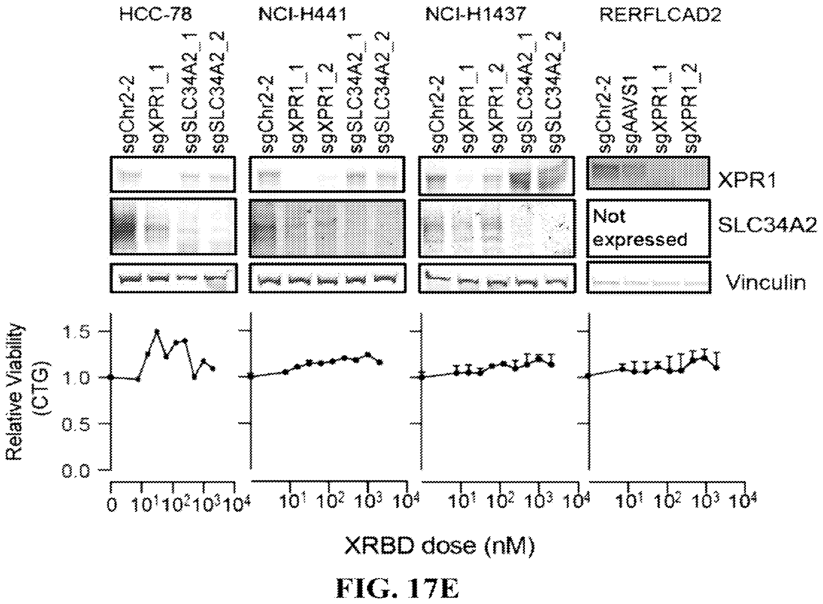

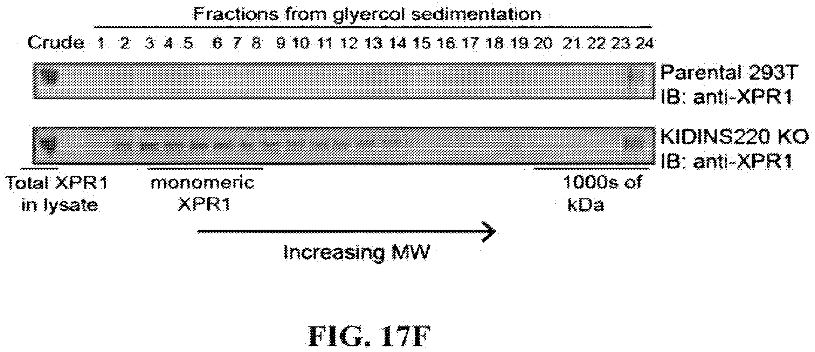

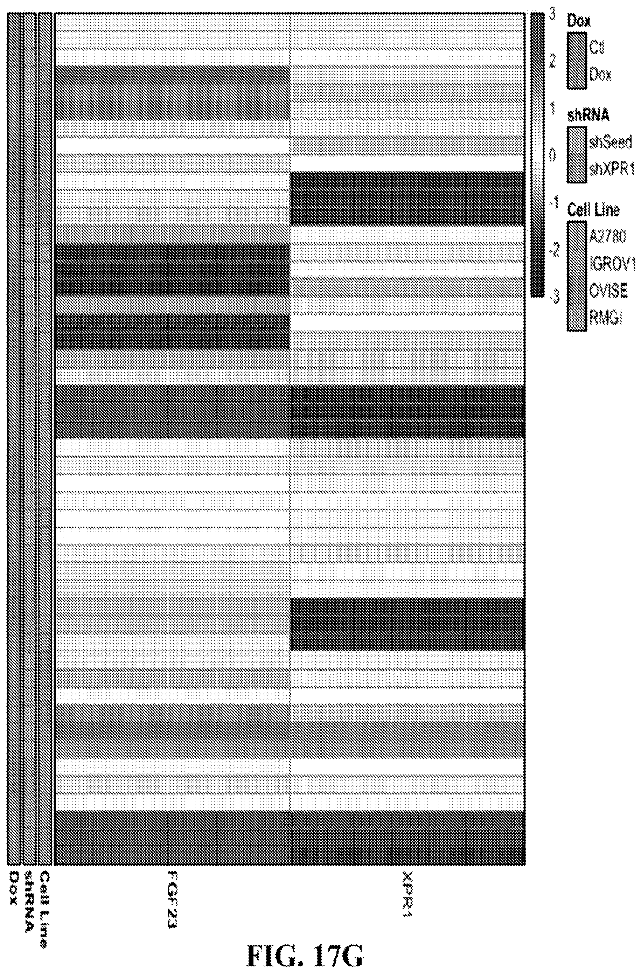

Applicants show that XRBD is a drug-like inhibitor of XPR1 by inhibiting XPR1-dependent phosphate efflux in ovarian cancer cell lines **(****Figure 17A****).** Using stable knockout of XPR1 or KIDINS220 in 293T cells Applicants show that XPR1 and KIDINS220 are in a protein complex, and KIDINS220 protein levels can be a surrogate marker for XPR1 inactivation and a potential biomarker of therapeutic response **(****Figure 17B** and corresponding to **Figure 2B****).** Using XRBD flow cytometry analysis of the 293T cells analyzed in **Figure 17B** Applicants show that KIDINS220 inactivation leads to a drastic decrease in XPR1 cell-surface localization **(****Figure 17C** and corresponding to **Figure 11C****).** Applicants further show that XRBD is a drug-like inhibitor of XPR1 by treating ovarian cancer cell lines with XRBD, such that the treatment causes viability defects in the ovarian cancer cell lines **(****Figure 17D****).** The XRBD sensitivity (decrease in cellular viability after five-day treatment with the top dose of XRBD relative to vehicle control) correlates to each cell lines' XPR1 inactivation sensitivity (assessed by CRISPR viability assays). Applicants analyzed XRBD treatment in a small panel of lung cancer cell lines **(****Figure 17E** and corresponding to **Figure 5A****).** Although expressing the predictive biomarker at high levels (SLC34A2), lung cancer cell lines can suppress the SLC34A2 phosphate importer to protect against cell viability defects upon XPR1 inhibition. Using glycerol gradient sedimentation analysis of XPR1-containing native protein complexes with or without KIDINS220 inactivation Applicants show that KIDINS220 inactivation leads to a drastic reduction in the molecular weight of XPR1 protein complexes, suggesting altered protein complex composition **(****Figure 17F****).** Using XPR1 RT-PCR Applicants show that inactivation of XPR1 leads to a change in phosphate-related genes (e.g., upregulation of FGF23), indicating a phosphate dysregulation state **(****Figure 17G****).**

The sequence of the XRBD protein used in **Figure 17** is:

MLVMEGSAFSKPLKDKINPWGPLIVMGILVRAGASVQRDSPHQIFNVTWRVTNLMTGQTANA
TSLLGTMTDTFPKLYFDLCDLVGDYWDDPEPDIGDGCRTPGGRRRTRLYDFYVCPGHTVPIG
CGGPGEGYCGKWGCETTGQAYWKPSSSWDLISLKRGNTPKDQGPCYDSSVSSGVQGATPGGR
CNPLVLEFTDAGRKASWDAPKVWGLRLYRSTGADPVTRFSLTRQVLNVGPRVPIGSVDVPRD
CGCKPCICTVPEVSSVFIFPPKPKDVLTITLTPKVTCVVVDISKDDPEVQFSWFVDDVEVHT
AQTQPREEQFNSTFRSVSELPIMHQDWLNGKEFKCRVNSAAFPAPIEKTISKTKGRPKAPQV
YTIPPPKEQMAKDKVSLTCMITDFFPEDITVEWQWNGQPAENYKNTQPIMDTDGSYFVYSKL
NVQKSNWEAGNTFTCSVLHEGLHNHHTEKSLSHSPGK (SEQ ID NO: 2).

### References

1. Faubert, B., Solmonson, A. & DeBerardinis, R. J. Metabolic reprogramming and cancer progression. Science 368, (2020).
2. Lord, C. J. & Ashworth, A. PARP inhibitors: Synthetic lethality in the clinic. Science 355, 1152-1158 (2017).
3. Fong, P. C. et al. Poly(ADP)-ribose polymerase inhibition: frequent durable responses in BRCA carrier ovarian cancer correlating with platinum-free interval. J. Clin. Oncol. 28, 2512-2519 (2010).
4. Ledermann, J. et al. Olaparib maintenance therapy in platinum-sensitive relapsed ovarian cancer. N. Engl. J. Med. 366, 1382-1392 (2012).
5. Lheureux, S., Braunstein, M. & Oza, A. M. Epithelial ovarian cancer: Evolution of management in the era of precision medicine. CA Cancer J. Clin. 69, 280-304 (2019).
6. Vaughan, S. et al. Rethinking ovarian cancer: recommendations for improving outcomes. Nat. Rev. Cancer 11, 719-725 (2011).
7. Virkki, L. V., Biber, J., Murer, H. & Forster, I. C. Phosphate transporters: a tale of two solute carrier families. Am. J. Physiol. Renal Physiol. 293, F643-54 (2007).
8. Blaine, J., Chonchol, M. & Levi, M. Renal control of calcium, phosphate, and magnesium homeostasis. Clin. J. Am. Soc. Nephrol. 10, 1257-1272 (2015).
9. Sabbagh, Y. et al. Intestinal npt2b plays a major role in phosphate absorption and homeostasis. J. Am. Soc. Nephrol. 20, 2348-2358 (2009).
10. Azevedo, C. & Saiardi, A. Eukaryotic Phosphate Homeostasis: The Inositol Pyrophosphate Perspective. Trends Biochem. Sci. 42, 219-231 (2017).
11. Desfougères, Y., Saiardi, A. & Azevedo, C. Inorganic polyphosphate in mammals: where's Wally? Biochem. Soc. Trans. (2020) doi:10.1042BST20190328.
12. Giovannini, D., Touhami, J., Charnet, P., Sitbon, M. & Battini, J.-L. Inorganic phosphate export by the retrovirus receptor XPR1 in metazoans. Cell Rep. 3, 1866-1873 (2013).
13. Ansermet, C. et al. Renal Fanconi Syndrome and Hypophosphatemic Rickets in the Absence of Xenotropic and Polytropic Retroviral Receptor in the Nephron. J. Am. Soc. Nephrol. 28, 1073-1078 (2017).
14. Xu, X. et al. Murine Placental-Fetal Phosphate Dyshomeostasis Caused by an Xpr1 Deficiency Accelerates Placental Calcification and Restricts Fetal Growth in Late Gestation. J. Bone Miner. Res. 35, 116-129 (2020).
15. Legati, A. et al. Mutations in XPR1 cause primary familial brain calcification associated with altered phosphate export. Nat. Genet. 47, 579-581 (2015).
16. Li, X. et al. Control of XPR1-dependent cellular phosphate efflux by InsP8 is an exemplar for functionally-exclusive inositol pyrophosphate signaling. Proc. Natl. Acad. Sci. U. S. A. (2020) doi:10.1073/pnas.1908830117.
17. Wilson, M. S., Jessen, H. J. & Saiardi, A. The inositol hexakisphosphate kinases IP6K1 and -2 regulate human cellular phosphate homeostasis, including XPR1-mediated phosphate export. J. Biol. Chem. 294, 11597-11608 (2019).
18. Tsherniak, A. et al. Defining a Cancer Dependency Map. Cell 170, 564-576.e16 (2017).
19. Meyers, R. M. et al. Computational correction of copy number effect improves specificity of CRISPR-Cas9 essentiality screens in cancer cells. Nat. Genet. 49, 1779-1784 (2017).
20. Chan, E. M. et al. WRN helicase is a synthetic lethal target in microsatellite unstable cancers. Nature 568, 551-556 (2019).
21. Price, C. et al. Genome-Wide Interrogation of Human Cancers Identifies EGLN1 Dependency in Clear Cell Ovarian Cancers. Cancer Res. 79, 2564-2579 (2019).
22. Ghandi, M. et al. Next-generation characterization of the Cancer Cell Line Encyclopedia. Nature 569, 503-508 (2019).
23. Yin, B. W. T. et al. Monoclonal antibody MX35 detects the membrane transporter NaPi2b (SLC34A2) in human carcinomas. Cancer Immun. 8, 3 (2008).
24. Gryshkova, V. et al. The study of phosphate transporter NAPI2B expression in different histological types of epithelial ovarian cancer. Exp. Oncol. 31, 37-42 (2009).
25. Levan, K. et al. Immunohistochemical evaluation of epithelial ovarian carcinomas identifies three different expression patterns of the MX35 antigen, NaPi2b. BMC Cancer 17, 303 (2017).
26. DeWeirdt, P. C. et al. Genetic screens in isogenic mammalian cell lines without single cell cloning. Nat. Commun. 11, 752 (2020).
27. Cancer Genome Atlas Research Network. Integrated genomic analyses of ovarian carcinoma. Nature 474, 609-615 (2011).
28. Cancer Genome Atlas Research Network et al. Integrated genomic characterization of endometrial carcinoma. Nature 497, 67-73 (2013).
29. Goldman, M. et al. The UCSC Xena platform for public and private cancer genomics data visualization and interpretation. bioRxiv 326470 (2019) doi:10.1101/326470.
30. Vivian, J. et al. Toil enables reproducible, open source, big biomedical data analyses. Nat. Biotechnol. 35, 314-316 (2017).
31. Wang, Y. et al. Tubal origin of ovarian endometriosis and clear cell and endometrioid carcinoma. Am. J. Cancer Res. 5, 869-879 (2015).
32. Elias, K. M. et al. Primordial germ cells as a potential shared cell of origin for mucinous cystic neoplasms of the pancreas and mucinous ovarian tumors. J. Pathol. 246, 459-469 (2018).
33. Lawrenson, K. et al. Integrated Molecular Profiling Studies to Characterize the Cellular Origins of High-Grade Serous Ovarian Cancer. bioRxiv 330597 (2018) doi:10.1101/330597.
34. Köbel, M. et al. Ovarian carcinoma subtypes are different diseases: implications for biomarker studies. PLoS Med. 5, e232 (2008).
35. Shi, K. et al. PAX8 regulon in human ovarian cancer links lineage dependency with epigenetic vulnerability to HDAC inhibitors. Elife 8, (2019).
36. Mittag, J., Winterhager, E., Bauer, K. & Grümmer, R. Congenital hypothyroid female pax8-deficient mice are infertile despite thyroid hormone replacement therapy. Endocrinology 148, 719-725 (2007).
37. Cheung, H. W. et al. Systematic investigation of genetic vulnerabilities across cancer cell lines reveals lineage-specific dependencies in ovarian cancer. Proc. Natl. Acad. Sci. U. S. A. 108, 12372-12377 (2011).
38. Mermel, C. H. et al. GISTIC2.0 facilitates sensitive and confident localization of the targets of focal somatic copy-number alteration in human cancers. Genome Biol. 12, R41 (2011).
39. TCGA Copy Number Portal. http://portals.broadinstitute.org/tcga/home.
40. McFarland, J. M. et al. Multiplexed single-cell profiling of post-perturbation transcriptional responses to define cancer vulnerabilities and therapeutic mechanism of action. bioRxiv 868752 (2019) doi:10.1101/868752.
41. Minisola, S. et al. Tumour-induced osteomalacia. Nat Rev Dis Primers 3, 17044 (2017).
42. Wainberg, M. et al. A genome-wide almanac of co-essential modules assigns function to uncharacterized genes. bioRxiv 827071 (2019) doi:10.1101/827071.
43. Kim, E. et al. A network of human functional gene interactions from knockout fitness screens in cancer cells. Life Sci Alliance 2, (2019).
44. Cabrera-Poch, N., Sánchez-Ruiloba, L., Rodriguez-Martinez, M. & Iglesias, T. Lipid raft disruption triggers protein kinase C and Src-dependent protein kinase D activation and Kidins220 phosphorylation in neuronal cells. J. Biol. Chem. 279, 28592-28602 (2004).
45. Iglesias, T. et al. Identification and cloning of Kidins220, a novel neuronal substrate of protein kinase D. J. Biol. Chem. 275, 40048-40056 (2000).
46. Arévalo, J. C., Yano, H., Teng, K. K. & Chao, M. V. A unique pathway for sustained neurotrophin signaling through an ankyrin-rich membrane-spanning protein. EMBO J. 23, 2358-2368 (2004).
47. Kong, H., Boulter, J., Weber, J. L., Lai, C. & Chao, M. V. An evolutionarily conserved transmembrane protein that is a novel downstream target of neurotrophin and ephrin receptors. J. Neurosci. 21, 176-185 (2001).
48. Wege, S. et al. The EXS Domain of PHO1 Participates in the Response of Shoots to Phosphate Deficiency via a Root-to-Shoot Signal. Plant Physiol. 170, 385-400 (2016).
49. Arpat, A. B. et al. Functional expression of PHO1 to the Golgi and trans-Golgi network and its role in export of inorganic phosphate: PHO1 localization to the Golgi/trans-Golgi network. Plant J. 5, no-no (2012).
50. Sánchez-Ruiloba, L. et al. Protein kinase D intracellular localization and activity control kinase D-interacting substrate of 220-kDa traffic through a postsynaptic density-95/discs large/zonula occludens-1-binding motif. J. Biol. Chem. 281, 18888-18900 (2006).
51. Wild, R. et al. Control of eukaryotic phosphate homeostasis by inositol polyphosphate sensor domains. Science 352, 986-990 (2016).
52. López-Sánchez, U. et al. Characterization of XPR1/SLC53A1 variants located outside of the SPX domain in patients with primary familial brain calcification. Sci. Rep. 9, 6776 (2019).
53. Ruiz, F. A., Lea, C. R., Oldfield, E. & Docampo, R. Human platelet dense granules contain polyphosphate and are similar to acidocalcisomes of bacteria and unicellular eukaryotes. J. Biol. Chem. 279, 44250-44257 (2004).
54. Marks, M. S., Heijnen, H. F. G. & Raposo, G. Lysosome-related organelles: unusual compartments become mainstream. Curr. Opin. Cell Biol. 25, 495-505 (2013).
55. Omelon, S. et al. Control of vertebrate skeletal mineralization by polyphosphates. PLoS One 4, e5634 (2009).
56. Snyder, N. A. et al. H+ and Pi Byproducts of Glycosylation Affect Ca2+ Homeostasis and Are Retrieved from the Golgi Complex by Homologs of TMEM165 and XPR1. G3 7, 3913-3924 (2017).
57. Wege, S. & Poirier, Y. Expression of the mammalian Xenotropic Polytropic Virus Receptor 1 (XPR1) in tobacco leaves leads to phosphate export. FEBS Lett. 588, 482-489 (2014).
58. He, P., Mann-Collura, O., Fling, J., Edara, N. & Razzaque, M. S. Elevated phosphate mediates extensive cellular toxicity: from abnormal proliferation to excessive cell death. bioRxiv 2020.01.02.892638 (2020) doi: 10.1101/2020.01.02.892638.
59. Di Marco, G. S. et al. Increased inorganic phosphate induces human endothelial cell apoptosis in vitro. Am. J. Physiol. Renal Physiol. 294, F1381-7 (2008).
60. Feild, J. A., Zhang, L., Brun, K. A., Brooks, D. P. & Edwards, R. M. Cloning and functional characterization of a sodium-dependent phosphate transporter expressed in human lung and small intestine. Biochem. Biophys. Res. Commun. 258, 578-582 (1999).
61. Hernando, N. et al. Intestinal Depletion of NaPi-IIb/Slc34a2 in Mice: Renal and Hormonal Adaptation. J. Bone Miner. Res. 30, 1925-1937 (2015).
62. Anheim, M. et al. XPR1 mutations are a rare cause of primary familial brain calcification. J. Neurol. 263, 1559-1564 (2016).
63. Wöhrle, S. et al. Pharmacological inhibition of fibroblast growth factor (FGF) receptor signaling ameliorates FGF23-mediated hypophosphatemic rickets. J. Bone Miner. Res. 28, 899-911 (2013).
64. Li, W. et al. Quality control, modeling, and visualization of CRISPR screens with MAGeCK-VISPR. Genome Biol. 16, 281 (2015).
65. Cerami, E. et al. The cBio cancer genomics portal: an open platform for exploring multidimensional cancer genomics data. Cancer Discov. 2, 401-404 (2012).
66. Gao, J. et al. Integrative analysis of complex cancer genomics and clinical profiles using the cBioPortal. Sci. Signal. 6, 11 (2013).
67. Conrad, M. et al. Regulation of lipid peroxidation and ferroptosis in diverse species. Genes Dev. 32, 602-619 (2018).

## Claims

1. One or more therapeutic agents for use in a method of treating uterine or ovarian cancer in a subject in need thereof wherein at least one therapeutic agent in the one or more therapeutic agents is capable of inhibiting XPR1:KIDINS220-mediated phosphate export, the method comprising administering to the subject the one or more therapeutic agents,
wherein the one or more therapeutic agents comprises a Receptor Binding Domain (RBD) fusion protein comprising the amino acid sequence: 
MLVMEGSAFSKPLKDKINPWGPLIVMGILVRAGASVQRDSPHQIFNVTWRVTNL
MTGQTANATSLLGTMTDTFPKLYFDLCDLVGDYWDDPEPDIGDGCRTPGGRRRTRLYDFYVC
PGHTVPIGCGGPGEGYCGKWGCETTGQAYWKPSSSWDLISLKRGNTPKDQGPCYDSSVSSGV
QGATPGGRCNPLVLEFTDAGRKASWDAPKVWGLRLYRSTGADPVTRFSLTRQVLNVGPRVPI
GSVDVPRDCGCKPCICTVPEVSSVFIFPPKPKDVLTITLTPKVTCVVVDISKDDPEVQFSWF
VDDVEVHTAQTQPREEQFNSTFRSVSELPIMHQDWLNGKEFKCRVNSAAFPAPIEKTISKTK
GRPKAPQVYTIPPPKEQMAKDKVSLTCMITDFFPEDITVEWQWNGQPAENYKNTQPIMDTDG
SYFVYSKLNVQKSNWEAGNTFTCSVLHEGLHNHHTEKSLSHSPGK (SEQ ID NO: 2)
wherein the uterine or ovarian cancer is **characterized by** higher expression of SLC34A2 in tumor tissue as compared to expression in normal tissue.

2. The one or more therapeutic agents for use according to claim 1, wherein the cancer is ovarian cancer.

3. The one or more therapeutic agents for use according to claim 1 or 2, wherein the one or more therapeutic agents disrupt XPR1/KIDINS220 interaction.

4. The one or more therapeutic agents for use according to any of claims 1 to 3, wherein one or more therapeutic agents capable of inhibiting XPR1:KIDINS220-mediated phosphate export are co-administered within a standard of care treatment regimen, wherein the standard of care treatment regimen comprises
surgery and chemotherapy, alternatively
administration of an immunotherapy, checkpoint blockade therapy or a PARP inhibitor.

5. The one or more therapeutic agents for use according to claims 1 to 4, further comprising monitoring the efficacy of the treatment comprising
detecting, after administration of the one or more therapeutic agents, in a tumor sample obtained from the subject the expression of one or more genes selected from the group consisting of SLC34A2, SLC20A1 and FGF23, wherein the treatment is effective if the expression level of SLC34A2 and/or SLC20A1 in the tumor sample are decreased, and/or the expression level of FGF23 in the tumor sample is increased relative to expression in tumor samples obtained before administration of the one or more therapeutic agents, alternatively detecting morphological changes associated with phosphate dysregulation in tumor cells obtained from the subject relative to morphology of tumor samples obtained before administration of the one or more therapeutic agents, wherein the treatment is effective if an increase in vacuole-like structures in the tumor cells is detected.

6. An *in vitro* method of determining whether a uterine or ovarian cancer is sensitive to phosphate dysregulation comprising:
detecting the expression of SLC34A2 in a tumor sample obtained from a subject,
wherein if SLC34A2 expression is higher in the tumor sample as compared to expression in normal tissue the tumor is sensitive, optionally
wherein the method further comprises detecting PAX8, alternatively detecting the amplification in XPR1 copy number in a tumor sample obtained from the subject, wherein if XPR1 copy number amplification is detected in the tumor sample the tumor
is sensitive, optionally
wherein the method further comprises detecting the copy number by inference from a target sequencing panel at the XPR1 locus on chromosome 1,
wherein detecting comprises one or more of immunohistochemistry (IHC), in situ RNA-seq, quantitative PCR, RNA-seq, CITE-seq, western blot, Fluorescence In Situ Hybridization (FISH), RNA-FISH, mass spectrometry, or FACS.

7. The method according to claim 6, wherein the cancer is ovarian cancer.

8. An *in vitro* method for identifying therapeutic agent capable of inhibiting mediated phosphate export, comprising:
applying a candidate agent to a cancer cell or cell population; and
detecting inhibition of phosphate efflux in the cell or cell population by the candidate agent, thereby identifying the agent.

9. An *in vitro* method for identifying a cancer sensitive to inhibition of XPR1:KIDINS220-mediated phosphate export, comprising:
applying an inhibitor of XPR1:KIDINS220-mediated phosphate export to a cancer cell
or cell population; and
detecting the phosphate concentration in the cell or cell population, wherein the cancer
is sensitive if the phosphate concentration is increased as compared to a control cell or population not treated with the inhibitor, optionally
wherein the inhibitor of XPR1:KIDINS220-mediated phosphate export is one or more therapeutic agents according to claim 3.

10. The method of claim 9, wherein the cancer cell or population is obtained or derived from a subject in need thereof.

## Patentansprüche

1. Ein oder mehrere therapeutische Mittel zur Verwendung bei einem Verfahren zum Behandeln von Gebärmutter- oder Eierstockkrebs bei einem Subjekt, das dessen bedarf, wobei mindestens ein therapeutisches Mittel in dem einen oder den mehreren therapeutischen Mitteln in der Lage ist, XPR1:KIDINS220-vermittelten Phosphatexport zu hemmen, wobei das Verfahren das Verabreichen, dem Subjekt, des einen oder der mehreren therapeutischen Mittel umfasst,
wobei das eine oder die mehreren therapeutischen Mittel ein Rezeptorbindedomäne-(RBD-) Fusionsprotein umfassen, das die Aminosäuresequenz umfasst: 
MLVMEGSAFSKPLKDKINPWGPLIVMGILVRAGASVQRDSPHQIFNVTWRVTNL
MTGQTANATSLLGTMTDTFPKLYFDLCDLVGDYWDDPEPDIGDGCRTPGGRRRTRLYDFYVC
PGHTVPIGCGGPGEGYCGKWGCETTGQAYWKPSSSWDLISLKRGNTPKDQGPCYDSSVSSGV
QGATPGGRCNPLVLEFTDAGRKASWDAPKVWGLRLYRSTGADPVTRFSLTRQVLNVGPRVPI
GSVDVPRDCGCKPCICTVPEVSSVFIFPPKPKDVLTITLTPKVTCVVVDISKDDPEVQFSWF
VDDVEVHTAQTQPREEQFNSTFRSVSELPIMHQDWLNGKEFKCRVNSAAFPAPIEKTISKTK
GRPKAPQVYTIPPPKEQMAKDKVSLTCMITDFFPEDITVEWQWNGQPAENYKNTQPIMDTDG
SYFVYSKLNVQKSNWEAGNTFTCSVLHEGLHNHHTEKSLSHSPGK (SEQ ID NO: 2)
wobei der Gebärmutter- oder Eierstockkrebs durch höhere Expression von SLC34A2 in Tumorgewebe im Vergleich mit der Expression in normalem Gewebe gekennzeichnet ist.

2. Ein oder mehrere therapeutische Mittel zur Verwendung nach Anspruch 1, wobei der Krebs Eierstockkrebs ist.

3. Ein oder mehrere therapeutische Mittel zur Verwendung nach Anspruch 1 oder 2, wobei das eine oder die mehreren therapeutischen Mittel die XPR1/KIDINS220-Wechselwirkung unterbrechen.

4. Ein oder mehrere therapeutische Mittel zur Verwendung nach einem der Ansprüche 1 bis 3, wobei ein oder mehrere therapeutische Mittel, die in der Lage sind, XPR1:KIDINS220-vermittelten Phosphatexport zu hemmen, gleichzeitig innerhalb eines Sorgfaltsmaßstab-Behandlungsregime verabreicht werden, wobei das Sorgfaltsmaßstab-Behandlungsregime Folgendes umfasst alternativ Chirurgie und Chemotherapie Verabreichen einer Immuntherapie, Checkpoint-Blockadetherapie oder eines PARP-Inhibitors.

5. Ein oder mehrere therapeutische Mittel zur Verwendung nach den Ansprüchen 1 bis 4, ferner Überprüfen der Wirksamkeit der Behandlung umfassend, umfassend Entdecken, nach Verabreichen des einen oder der mehreren therapeutischen Mittel, in einer Tumorprobe, die von dem Subjekt erhalten worden ist, der Expression eines oder mehrerer Gene ausgewählt aus der Gruppe bestehend aus SLC34A2, SLC20A1 und FGF23, wobei die Behandlung wirksam ist, wenn das Expressionsniveau von SLC34A2 und/oder SLC20A1 in der Tumorprobe reduziert ist und/oder das Expressionsniveau von FGF23 in der Tumorprobe mit Bezug auf die Expression in Tumorproben erhöht ist, die vor Verabreichen des einen oder der mehreren therapeutischen Mittel erhalten worden sind, alternativ Entdecken morphologischer Änderungen, die mit Phosphatdysregulierung in Tumorzellen, die von dem Subjekt erhalten worden sind, verbunden sind, im Vergleich mit der Morphologie von Tumorproben, die vor Verabreichen des einen oder der mehreren therapeutischen Mittel erhalten worden sind, wobei die Behandlung wirksam ist, wenn eine Erhöhung in der vakuolähnlichen Struktur in den Tumorzellen entdeck wird.

6. In Vitro-Verfahren zum Bestimmen, ob ein Gebärmutter- oder Eierstockkrebs gegen Phosphatdysregulierung empfindlich ist, umfassend
Entdecken der Expression von SLC34A2 in einer Tumorproben, die von einem Subjekt erhalten worden ist, wobei, wenn die SLC34A2-Expression höher in der Tumorproben im Vergleich mit der Expression in normalem Gewebe ist, der Tumor empfindlich ist,
wobei wahlweise das Verfahren ferner Entdecken von PAX8, alternativ Entdecken der Amplifikation der XPR1-Kopieanzahl in einer Tumorprobe umfasst, die von dem Subjekt erhalten worden ist, wobei, wenn die XPR1-Kopieanzahlamplifikation in der Tumorproben entdeckt wird, der Tumor
empfindlich ist,
wobei wahlweise das Verfahren ferner Entdecken der Kopieanzahl durch Rückschluss von einer Zielsequenzierplatte an der XPR1-Stelle am Chromosom 1 umfasst,
wobei das Entdecken eines oder mehrere von Immunhistochemie (IHC), In-Situ-RNA-Seq, quantitativer PCR, RNA-Seq, CITE-Seq, Western-Blot, Fluoreszenz-in-Situ-Hybridisierung (FISH), RNA-FISH, Massenspektrometrie oder FACS umfasst.

7. Verfahren nach Anspruch 6, wobei der Krebs Gebärmutterkrebs ist.

8. In-Vitro-Verfahren zum Identifizieren eines therapeutischen Mittels, das in der Lage ist, XPR1:
KIDINS220-vermittelten Phosphatexport zu hemmen, umfassend:
Aufbringen eines Kandidatenmittels auf eine Krebszelle oder Zellpopulation; und
Entdecken des Hemmens von Phosphatausfluss in der Zelle oder Zellpopulation durch das Kandidatenmittel, dadurch Identifizieren des Mittels.

9. In-Vitro-Verfahren zum Identifizieren eines Krebses, der gegen das Hemmen von XPR1:KIDINS220-vermittelten Phosphatexport empfindlich ist, umfassend:
Aufbringen eines Inhibitors von XPR1:KIDINS220-vermitteltem Phosphatexport auf eine Krebszelle oder Zellpopulation; und
Entdecken der Phosphatkonzentration in der Zelle oder Zellpopulation, wobei der Krebs empfindlich ist, wenn die Phosphatkonzentration im Vergleich mit einer Kontrollzelle steigt oder die Population nicht mit dem Inhibitor behandelt wird,
wobei wahlweise der Inhibitor von XPR1:KIDINS220-vermitteltem Phosphatexport ein oder mehrere therapeutische Mittel nach Anspruch 3 ist.

10. Verfahren nach Anspruch 9, wobei die Krebszelle oder - population von einem Subjekt erhalten oder abgeleitet ist, das dessen bedarf.

## Revendications

1. Un ou plusieurs agents thérapeutiques destinés à être utilisés dans un procédé de traitement du cancer de l'utérus ou de l'ovaire chez un sujet qui en a besoin, où au moins un agent thérapeutique dans les un ou plusieurs agents thérapeutiques est capable d'inhiber l'exportation de phosphate médiée par XPR1:KIDINS220, le procédé comprenant l'administration au sujet des un ou plusieurs agents thérapeutiques,
où les un ou plusieurs agents thérapeutiques comprennent une protéine de fusion à domaine de liaison au récepteur (RBD) comprenant la séquence d'acides aminés: 
MLVMEGSAFSKPLKDKINPWGPLIVMGILVRAGASVQRDSPHQIFNVTWRVTNL
MTGQTANATSLLGTMTDTFPKLYFDLCDLVGDYWDDPEPDIGDGCRTPGGRRRTRLYDFYVC
PGHTVPIGCGGPGEGYCGKWGCETTGQAYWKPSSSWDLISLKRGNTPKDQGPCYDSSVSSGV
QGATPGGRCNPLVLEFTDAGRKASWDAPKVWGLRLYRSTGADPVTRFSLTRQVLNVGPRVPI
GSVDVPRDCGCKPCICTVPEVSSVFIFPPKPKDVLTITLTPKVTCVVVDISKDDPEVQFSWF
VDDVEVHTAQTQPREEQFNSTFRSVSELPIMHQDWLNGKEFKCRVNSAAFPAPIEKTISKTK
GRPKAPQVYTIPPPKEQMAKDKVSLTCMITDFFPEDITVEWQWNGQPAENYKNTQPIMDTDG
SYFVYSKLNVQKSNWEAGNTFTCSVLHEGLHNHHTEKSLSHSPGK (SEQ ID NO: 2)
où le cancer de l'utérus ou de l'ovaire est **caractérisé par** une expression plus forte de SLC34A2 dans le tissu tumoral par comparaison avec l'expression dans un tissu normal.

2. Les un ou plusieurs agents thérapeutiques destinés à être utilisés selon la revendication 1, où le cancer est un cancer de l'ovaire.

3. Les un ou plusieurs agents thérapeutiques destinés à être utilisés selon la revendication 1 ou 2, où les un ou plusieurs agents thérapeutiques perturbent l'interaction XPR1/KIDINS220.

4. Les un ou plusieurs agents thérapeutiques destinés à être utilisés selon l'une quelconque des revendications 1 à 3, où un ou plusieurs agents thérapeutiques capables d'inhiber l'exportation de phosphate médiée par XPR1:KIDINS220 sont co-administrés dans le cadre d'un schéma de traitement standard, où le schéma de traitement standard comprend
une chirurgie et une chimiothérapie, alternativement
l'administration d'une immunothérapie, d'une thérapie d'inhibition de point de contrôle ou d'un inhibiteur de PARP.

5. Les un ou plusieurs agents thérapeutiques destinés à être utilisés selon les revendications 1 à 4, comprenant en outre le suivi de l'efficacité du traitement comprenant
la détection, après administration des un ou plusieurs agents thérapeutiques, dans un échantillon tumoral prélevé chez le sujet, de l'expression d'un ou plusieurs gènes choisis dans le groupe consistant en SLC34A2, SLC20A1 et FGF23, où le traitement est efficace si le niveau d'expression de SLC34A2 et/ou SLC20A1 dans l'échantillon tumoral est réduit, et/ou si le niveau d'expression de FGF23 dans l'échantillon tumoral est augmenté par rapport à l'expression dans des échantillons tumoraux prélevés avant l'administration des un ou plusieurs agents thérapeutiques, alternativement la détection de modifications morphologiques associées à une dérégulation du phosphate dans des cellules tumorales prélevées chez le sujet par rapport à la morphologie des échantillons tumoraux prélevés avant l'administration des un ou plusieurs agents thérapeutiques, où le traitement est efficace si une augmentation des structures analogues à des vacuoles dans les cellules tumorales est détectée.

6. Procédé *in vitro* de détermination si un cancer de l'utérus ou de l'ovaire est sensible à une dérégulation du phosphate, comprenant:
la détection de l'expression de SLC34A2 dans un échantillon tumoral prélevé chez un sujet,
où si l'expression de SLC34A2 est plus élevée dans l'échantillon tumoral comparée à l'expression dans un tissu normal, la tumeur est sensible, éventuellement
où le procédé comprend en outre la détection de PAX8, alternativement la détection de l'amplification du nombre de copies de XPR1 dans un échantillon tumoral prélevé chez le sujet, où si une amplification du nombre de copies de XPR1 est détectée dans l'échantillon tumoral, la tumeur est sensible, éventuellement
où le procédé comprend en outre la détection du nombre de copies par inférence à partir d'un panel de séquençage cible au locus XPR1 sur le chromosome 1,
où la détection comprend un ou plusieurs parmi l'immunohistochimie (IHC), le séquençage d'ARN in situ, la PCR quantitative, le séquençage d'ARN, le le séquençage CITE, le western blot, l'hybridation in situ en fluorescence (FISH), la FISH sur ARN, la spectrométrie de masse, ou FACS.

7. Procédé selon la revendication 6, où le cancer est un cancer de l'ovaire.

8. Procédé *in vitro* d'identification d'un agent thérapeutique capable d'inhiber l'exportation de phosphate médiée par XPR1:KIDINS220, comprenant:
l'application d'un agent candidat à une cellule ou une population cellulaire cancéreuse; et
la détection de l'inhibition de l'efflux de phosphate dans la cellule ou la population cellulaire par l'agent candidat, identifiant ainsi l'agent.

9. Procédé *in vitro* d'identification d'un cancer sensible à l'inhibition de l'exportation de phosphate médiée par XPR1:KIDINS220, comprenant:
l'application d'un inhibiteur de l'exportation de phosphate médiée par XPR1:KIDINS220 à une cellule ou une population cellulaire cancéreuse; et
la détection de la concentration de phosphate dans la cellule ou la population cellulaire, où le cancer est sensible si la concentration de phosphate est augmentée par comparaison avec une cellule ou population témoin non traitée avec l'inhibiteur, éventuellement
où l'inhibiteur de l'exportation de phosphate médiée par XPR1:KIDINS220 est un ou plusieurs agents thérapeutiques selon la revendication 3.

10. Procédé selon la revendication 9, où la cellule ou population cancéreuse est obtenue ou dérivée d'un sujet qui en a besoin.
